(19) Europäisches Patentamt
European Patent Office
Office européen des brevets

(11) EP 4 810 545 A1

# (12) EUROPEAN PATENT APPLICATION
published in accordance with Art. 153(4) EPC

(43) Date of publication:
**23.09.2026 Bulletin 2026/39**

(21) Application number: **24891435.0**

(22) Date of filing: **13.11.2024**

(51) International Patent Classification (IPC):
***C08J 3/24*** *(2006.01)* ***A61F 13/53*** *(2006.01)*
***B01J 20/26*** *(2006.01)* ***B01J 20/30*** *(2006.01)*
***C08J 3/12*** *(2006.01)*

(52) Cooperative Patent Classification (CPC):
**A61F 13/53; B01J 20/26; B01J 20/30; C08J 3/12; C08J 3/24**

(86) International application number:
**PCT/JP2024/040355**

(87) International publication number:
**WO 2025/105399 (22.05.2025 Gazette 2025/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.11.2023 JP 2023193270**

(71) Applicant: **Nippon Shokubai Co., Ltd.**
**Osaka-shi, Osaka 541-0043 (JP)**

(72) Inventors:
- **TADA, Kenji**
  **Himeji-shi, Hyogo 671-1282 (JP)**
- **UEDA, Manabu**
  **Himeji-shi, Hyogo 671-1282 (JP)**
- **NISHIOKA, Masaaki**
  **Himeji-shi, Hyogo 671-1282 (JP)**
- **KASHIMOTO, Masaki**
  **Himeji-shi, Hyogo 671-1282 (JP)**

(74) Representative: **Ricker, Mathias**
**Wallinger Ricker Schlotter Tostmann**
**Patent- und Rechtsanwälte Partnerschaft mbB**
**Zweibrückenstraße 5-7**
**80331 München (DE)**

## (54) WATER ABSORBENT COMPOSITION AND METHOD FOR PRODUCING SAME

(57) An object of the present invention is to provide a method for producing a water-absorbing agent composition that maintains the surface tension of a liquid to be absorbed even when a water-absorbent resin having a high specific surface area is surface-crosslinked, has high water absorption properties under pressure (for example, absorption capacity under pressure (AAP) and gap water ratio under pressure), and does not inhibit improvement in absorption speed. The production method according to the present invention is a method for producing a water-absorbing agent composition containing a water-absorbent resin as a main component, the method including a surface crosslinking step for the water-absorbent resin, wherein (1) the water-absorbent resin before surface crosslinking has a specific surface area of 25 $m^2$/kg or more; (2) specific peroxide and organic surface crosslinking agent are used; (3) a water vapor density in a heat treatment step is appropriately controlled.



Processed by Luminess, 75001 PARIS (FR)

## Description

Technical Field

The present invention relates to a water-absorbing agent composition containing a water-absorbent resin as a main component, and a method for producing the same.

Background Art

Water-absorbing agent compositions in which water-absorbent resins are used for the purpose of absorbing body fluids are widely used for hygienic materials such as disposable diapers, sanitary napkins, and so-called incontinence pats. As the water-absorbent resin, a crosslinked body of a partially neutralized polyacrylic acid and the like are known. In recent years, with the reduction in thickness of hygienic materials, there has been an increasing demand for the water-absorbing agent composition to have not only excellent water absorption properties such as absorption capacity without pressure and absorption capacity under pressure but also a high water absorption speed. To increase the water absorption speed, it is necessary to increase the contact area between a liquid to be absorbed and the water-absorbing agent composition, and a water-absorbent resin having an increased specific surface area is disclosed (Patent Documents 1 to 7). A water-absorbent resin having a high specific surface area for blood absorption is also disclosed (Patent Document 8).

It is known that increasing the specific surface area improves the absorption speed of the water-absorbent resin, but on the other hand, the gel strength decreases, and the water absorption properties under pressure (for example, absorption capacity under pressure (AAP)) decrease, and thus, the absorption speed and the water absorption properties under pressure are in a trade-off relationship.

On the other hand, to increase the absorption capacity under pressure (AAP), it is also known that a surface crosslinking agent capable of reacting with a functional group of a water-absorbent resin is caused to react in the vicinity of the surface of the water-absorbent resin to perform surface crosslinking for increasing the crosslinking density in the vicinity of the surface as compared with the inside of the water-absorbent resin, and various surface crosslinking techniques have also been proposed (for example, Patent Document 9).

Citation List

Patent Document

Patent Document 1: WO 97/03114
Patent Document 2: JP 10-057805 A
Patent Document 3: EP 0872491
Patent Document 4: EP 0937739
Patent Document 5: WO 99/03577
Patent Document 6: WO 2013/018571
Patent Document 7: WO 2016/111223
Patent Document 8: WO 02/085959
Patent Document 9: JP 5128098 B
Patent Document 10: WO 2013/072268

Summary of Invention

As described above, various surface crosslinking techniques have been proposed to enhance the water absorption properties under pressure (for example, absorption capacity under pressure (AAP)).

On the other hand, the inventors of the present invention have found that, in the course of studying the absorption behavior of a water-absorbing agent composition and a water-absorbent resin having an increased specific surface area, the absorption speed of a surface-crosslinked water-absorbent resin does not become sufficiently fast as designed even though the specific surface area is increased.

To increase the absorption speed, which has been inhibited from being improved as described above, to a desired level, a treatment for further increasing the specific surface area is performed, but the treatment for excessively increasing the specific surface area causes a decrease in physical properties such as an absorption capacity under pressure, which is in a trade-off relationship, and as a result, there has been a possibility that the water-absorbent resin

cannot obtain desired absorption characteristics, and the performance of the hygienic material also deteriorates.

On the other hand, a method of avoiding an excessive increase in specific surface area and hydrophilizing the water-absorbent resin is also conceivable. As such a method, a method of disposing a hydrophilic surfactant in a water-absorbent resin is known (for example, Patent Document 10). However, the addition of the surfactant lowers the surface tension of the liquid to be absorbed, and there is a problem that the return amount of liquid is increased in use in hygienic materials such as disposable diapers and incontinence pads.

An object of the present invention is to provide a method for producing a water-absorbing agent composition that maintains the surface tension of a liquid to be absorbed even when a water-absorbent resin having a high specific surface area is surface-crosslinked, has high water absorption properties under pressure (for example, absorption capacity under pressure (AAP) and gap water ratio under pressure), and does not inhibit improvement in absorption speed. Another object of the present invention is to provide a water-absorbing agent composition that maintains the surface tension of a liquid to be absorbed and has high water absorption properties under pressure (for example, absorption capacity under pressure (AAP) and gap water ratio under pressure) despite having a high specific surface area.

The present invention that has been able to solve the above-described problems has the following configuration.

That is, one aspect of the present invention is:

[1] a method for producing a water-absorbing agent composition including a surface-crosslinked water-absorbent resin as a main component, the method including a surface crosslinking step for a water-absorbent resin,
the method satisfying all of (1) to (3) described below:

(1) the water-absorbent resin before surface crosslinking has a specific surface area of 25 $m^2$/kg or more;
(2) the surface crosslinking step is performed in the presence of an organic surface crosslinking agent capable of reacting with a peroxide and a carboxyl group;
(3) the surface crosslinking step includes a heat treatment step of performing a heat treatment in an atmosphere having a water vapor density of more than 0.005 g/L simultaneously with or after adding the organic surface crosslinking agent to the water-absorbent resin;

[2] in the method for producing a water-absorbing agent composition according to [1], the water vapor density is preferably 0.6 g/L or less;
[3] in the production method according to [1] or [2], the water-absorbent resin before surface crosslinking preferably has an irregularly crushed shape;
[4] in the method for producing a water-absorbing agent composition according to any one of [1] to [3], the water-absorbent resin before surface crosslinking preferably has a D50 (mass-average particle diameter) 250 μm or more and less than 550 μm, and a mass proportion of particles contained in the water-absorbent resin before surface crosslinking and having a particle diameter of less than 150 μm is preferably is 3 mass% or less;
[5] in the method for producing a water-absorbing agent composition according to any one of [1] to [4], the method preferably further including a polymerization step for an aqueous unsaturated monomer solution, and the water-absorbent resin is preferably obtained by foaming polymerization of the aqueous unsaturated monomer solution;
[6] in the method for producing a water-absorbing agent composition according to any one of [1] to [5], the organic surface crosslinking agent is preferably added to the water-absorbent resin in a solution state, and a concentration of the organic surface crosslinking agent in the surface crosslinking agent solution is preferably 0.1 mass% or more and 60 mass% or less;
[7] in the method for producing a water-absorbing agent composition according to any one of [1] to [6], the organic surface crosslinking agent is preferably added to the water-absorbent resin from two or more addition nozzles installed in a mixing apparatus;
[8] in the method for producing a water-absorbing agent composition according to any one of [1] to [7], the surface crosslinking step is preferably performed under (a slight reduced pressure of) -10 kPa or more and 0 kPa or less as a differential pressure relative to atmospheric pressure;
[9] in the method for producing a water-absorbing agent composition according to any one of [1] to [8], the peroxide is preferably a persulfate.

Another aspect of the present invention is:

[10] a water-absorbing agent composition including a surface-crosslinked water-absorbent resin as a main component, the water-absorbing agent composition satisfying all of (1) to (2) described below:

(1) the water-absorbing agent composition has a specific surface area of 25 $m^2$/kg or more;
(2) the water-absorbing agent composition has a Washburn method contact angle $\theta 2$ of 72° or less as measured

with a 20 mass% aqueous sodium chloride solution.

[11] In the water-absorbing agent composition according to [10], the surface-crosslinked water-absorbent resin preferably has an irregularly crushed shape and has a D50 (mass-average particle diameter) of 250 μm or more and less than 550 μm, and a mass proportion of particles contained in the water-absorbent resin and having a particle diameter of less than 150 μm is preferably 3 mass% or less;
[12] in the water-absorbing agent composition according to [10] or [11], the water-absorbing agent composition preferably has a water content of more than 0 mass% and 5 mass% or less;
[13] in the water-absorbing agent composition according to any one of [10] to [12], the water-absorbing agent composition preferably has an absorption capacity under pressure (AAP) of 20.0 g/g or more at a load of 4.83 kPa;
[14] in the water-absorbing agent composition according to any one of [10] to [13], the water-absorbing agent composition preferably has a gap water ratio under pressure of 9.0 g/g or more;
[15] in the water-absorbing agent composition according to any one of [10] to [14], the water-absorbing agent composition preferably has a surface tension of 56 mN/m or more;
[16] in the water-absorbing agent composition according to any one of [10] to [15], the water-absorbing agent composition preferably has a Vortex of 50 seconds or less;
[17] in the water-absorbing agent composition according to any one of [10] to [16], the water-absorbing agent composition preferably further contains a peroxide decomposition product, and the peroxide decomposition product preferably has a higher concentration on a surface of the water-absorbing agent composition than a concentration inside the water-absorbing agent composition;
[18] in the water-absorbing agent composition according to any one of [10] to [17], when particles are sieved using a JIS standard sieve having a mesh size of 300 μm, and thereby particles having a particle diameter of 300 μm or more are defined as a particle group a and particles having a particle diameter of less than 300 μm are defined as a particle group b, and an impact test using a paint shaker is performed on the water-absorbing agent composition, and thereby an amount of a peroxide decomposition product present in the particle group a is defined as C1, and an amount of a peroxide decomposition product present in the particle group b is defined as C2, C2 - C1 is preferably more than 0 mass% and 1 mass% or less;
[19] in the water-absorbing agent composition according to any one of [10] to [18], the water-absorbing agent composition preferably has an SFC (saline flow conductivity) of $1 \times 10^{-7}$ $cm^3 \cdot sec/g$ or more.

Another aspect of the present invention is:
an absorbent article including the water-absorbing agent composition described in any one of [10] to [19].

Description of Embodiments

One aspect of the present invention is:

a method for producing a water-absorbing agent composition including a surface-crosslinked water-absorbent resin as a main component, the method comprising a surface crosslinking step for a water-absorbent resin,
the method satisfying all of (1) to (3) described below:

(1) the water-absorbent resin before surface crosslinking has a specific surface area of 25 $m^2/kg$ or more;
(2) the surface crosslinking step is performed in the presence of an organic surface crosslinking agent capable of reacting with a peroxide and a carboxyl group;
(3) the surface crosslinking step includes a heat treatment step of performing a heat treatment in an atmosphere having a water vapor density of more than 0.005 g/L simultaneously with or after addition of the organic surface crosslinking agent to the water-absorbent resin.

Another aspect of the present invention is:
a water-absorbing agent composition including a surface-crosslinked water-absorbent resin as a main component, the water-absorbing agent composition satisfying all of (1) to (2) described below:

(1) the water-absorbing agent composition has a specific surface area of 25 $m^2/kg$ or more;
(2) the water-absorbing agent composition has a Washburn method contact angle θ2 of 72° or less as measured with a 20 mass% aqueous sodium chloride solution.

The inventors of the present invention have considered that the reason of not having sufficient absorption speed as designed when surface crosslinking is performed on the water-absorbing agent composition or the water-absorbent

resin having an increased specific surface area as described above, is that in the known surface crosslinking treatment on a water-absorbent resin, the surface of the water-absorbent resin is slightly hydrophobized, and the affinity (liquid compatibility) between the liquid to be absorbed and the water-absorbent resin deteriorates. That is, the inventors have considered that the liquid to be absorbed hardly penetrates into the cavity formed on the surface of the water-absorbent resin having an increased specific surface area, and the contact area between the liquid to be absorbed and the surface of the water-absorbent resin is reduced, and thus, the surface of the water-absorbent resin cannot be effectively utilized as an absorption surface in contact with the liquid to be absorbed, and the improvement in absorption speed is inhibited. That is, it is considered that a desired absorption speed has not been obtained although the specific surface area has been increased at the somewhat expense of the absorption capacity under pressure (AAP) or the like having a trade-off relationship with the absorption speed and the gap water retention force.

The inventors of the present invention have found a method for precisely controlling the affinity of the surface of the water-absorbent resin with the liquid to be absorbed by paying attention to the composition of the treatment agent for performing the surface crosslinking treatment on the water-absorbing agent composition and the water-absorbent resin, and the dew point and water vapor density in the heat treatment machine for performing the surface crosslinking reaction, and have completed the present invention.

By adopting the method having the above configuration, it is possible to inhibit hydrophobization of the surface even when surface crosslinking is performed, and to provide a method for improving the affinity between the liquid to be absorbed and the surface of the water-absorbent resin, in which the liquid to be absorbed also penetrates into the cavity of the water-absorbent resin even in the water-absorbent resin or the water-absorbing agent composition having an increased specific surface area. It is considered that the liquid easily permeates into the particle cavity, and as a result, the water absorption speed improves. In addition, it is considered that the affinity between the liquid to be absorbed and the surface of the water-absorbent resin is improved, and thus, the amount of liquid that can be retained in the gaps between particle layers and the particle cavity, that is, the gap water retention amount is also increased, and a water-absorbent resin having a good absorption capacity under pressure (AAP) and gap water ratio under pressure can be obtained. In addition, there is no need to excessively increase the specific surface area to increase the absorption speed or the like, and there can be provided a water-absorbing agent composition in which the absorption capacity under pressure (AAP) and the gap water ratio under pressure showing a trade-off relationship with the increase in specific surface area are balanced.

The inventors of the present invention also have paid attention to a contact angle as an index indicating the affinity between the surface of the water-absorbent resin and the liquid to be absorbed. A high-speed camera has been used for measuring a contact angle of a water-absorbent resin to eliminate an influence of a property that the water-absorbent resin absorbs a desired liquid to be absorbed (for example, human urine, or physiological saline or artificial urine representative thereof) in a known method. The value of the contact angle has been determined by closely spreading a water-absorbent resin on a bed, dropping the liquid to be absorbed on the bed, capturing an image at the moment of contact, and analyzing the image. However, at the moment when the droplet falls and comes into contact with the water-absorbent resin bed, the influence of deformation of the droplet due to gravity cannot be eliminated, and as a result, stable contact angle data cannot be obtained. On the other hand, by adopting the improved Washburn method (permeation speed method), the contact angle can be measured more accurately than a known method.

In addition, the inventors of the present invention have found that by adopting the improved Washburn method, it is possible to precisely grasp and control the affinity of the surface of the water-absorbent resin with the liquid to be absorbed, which varies depending on the magnitude of the specific surface area and the conditions of the surface crosslinking treatment. As a result, by obtaining the water-absorbing agent composition having the configuration described above, it has become possible to obtain a water-absorbing agent composition exhibiting a desired absorption capacity under pressure (AAP), a desired gap water ratio under pressure, a desired liquid permeation rate under pressure (SFC or GBP), and the like while maintaining a high water absorption speed.

Hereinafter, the method for producing a water-absorbing agent composition of the present invention will be described in detail, but the scope of the present invention is not limited to these descriptions. Modifications and implementations other than the following examples can be appropriately made without impairing the spirit of the present invention. Specifically, the present invention is not limited to embodiments described below, and may be changed in various ways within the scope of the claims. Thus, an embodiment achieved by appropriately combining technical means disclosed in different embodiments is also included in the technical scope of the present invention.

[1] Definition of Terms

[1-1] Water-Absorbent Resin, Water-Absorbing Agent Composition

The term "water-absorbent resin" in the present description refers to a polymer gelling agent that is water-swellable and water-insoluble, and the water-absorbent resin is generally in a powder form. "Water-swellable" means that CRC (absorption capacity without pressure) defined by NWSP 241.0. R2 (19) is 5 g/g or more, and "water-insoluble"

means that Ext (soluble content) defined by NWSP 270.0. R2 (19) is 50 mass% or less.

The "water-absorbent resin" is preferably a hydrophilic crosslinked polymer obtained through crosslinking polymerization of an unsaturated monomer having a carboxyl group, but the whole amount, that is, 100 mass% of the "water-absorbent resin" does not need to be a crosslinked polymer. An additive or the like may be contained within the range that satisfies the performance of CRC, Ext, and the like.

The "water-absorbent resin" may refer to "a polymer in which only the inside is crosslinked, that is, a polymer in which the crosslinking density of the inside and the crosslinking density of the surface are substantially the same" or "a polymer in which the inside and the surface are crosslinked, that is, a polymer in which the crosslinking density of the surface is relatively high relative to the crosslinking density of the inside".

In the present description, the "polymer in which only the inside is crosslinked" and the "polymer in which the inside and the surface are crosslinked" are both described as "water-absorbent resin" without distinction in principle. However, when it is necessary to clearly distinguish the presence or absence of surface crosslinking, the "polymer in which only the inside is crosslinked" is described as "water-absorbent resin before surface crosslinking" because it is a polymer obtained before surface crosslinking is performed, and the "polymer in which the inside and the surface are crosslinked" is described as "water-absorbent resin after surface crosslinking" or "surface-crosslinked water-absorbent resin" because it is a polymer obtained after surface crosslinking is performed. The phrase "before surface crosslinking" means "before addition of a surface crosslinking agent" or "before start of a crosslinking reaction through heat treatment even after addition of a surface crosslinking agent".

The "water-absorbent resin" may refer to only a resin component, but it may contain a component other than a resin such as an additive.

The "water-absorbing agent composition" in the present description means a mixture of the "water-absorbent resin" and a component other than the water-absorbent resin such as an additive optionally contained.

The "water-absorbing agent composition" contains a water-absorbent resin as a main component. The "main component" means that the mass proportion of the water-absorbent resin to the whole water-absorbing agent composition is preferably 50 mass% or more, and 60 mass% or more, 70 mass% or more, 80 mass% or more, and 90 mass% or more, and 100 mass% or less, in this order. The "water-absorbing agent composition" preferably contains, as additional components, water, and minor components such as a polyvalent metal salt, a chelating agent, a surfactant within a range in which surface tension can be maintained, and a surface-modified polymer.

[1-2] Polyacrylic Acid (Salt)-Based Water-Absorbent Resin

The term "polyacrylic acid (salt)-based water-absorbent resin" in the present description means a water-absorbent resin that contains, as a raw material, an acrylic acid and/or a salt thereof (hereinafter, denoted as "acrylic acid (salt)"). That is, the "polyacrylic acid (salt)-based water-absorbent resin" is a polymer having a structural unit derived from an acrylic acid (salt), and is a polymer having a graft component as an optional component. Specifically, the polyacrylic acid (salt)-based water-absorbent resin is a polymer containing an acrylic acid (salt) in an amount of preferably 50 mol% or more, more preferably 70 mol% or more, still more preferably 90 mol% or more, and preferably 100 mol% or less, more preferably substantially 100 mol% relative to the portion excluding an internal crosslinking agent in the monomers involved in the polymerization reaction.

[1-3] "EDANA" and "NWSP"

"EDANA" is an abbreviation for the European Disposables and Nonwovens Association. "NWSP" is an abbreviation for Non-Woven Standard Procedure, and it indicates an international standard method of measuring a water-absorbing agent composition or a water-absorbent resin provided by EDANA. In the present invention, unless otherwise specified, the physical properties of the water-absorbing agent composition or the water-absorbent resin are measured in accordance with the original NWSP (revised in 2019). In the present invention, the measurements follow the measurement methods in the following Examples unless otherwise stated.

[1-4] CRC (NWSP 241.0.R2 (19))

"CRC" is an abbreviation for Centrifuge Retention Capacity, and it means an absorption capacity of the water-absorbing agent composition or the water-absorbent resin without pressure.

[1-5] Ext (NWSP 270.0.R2 (19))

"Ext" is an abbreviation for Extractables, and it means water-soluble content, that is, the amount of the water-soluble component in the water-absorbing agent composition or the water-absorbent resin. Specifically, it refers to the

amount (unit: mass%) of the dissolved polymer after 1.0 g of the water-absorbing agent composition or the water-absorbent resin is added to 200 ml of a 0.9 mass% aqueous sodium chloride solution and stirred at 250 rpm for 1 hour or 16 hours. The amount of dissolved polymer is determined using pH titration. The stirring time is recorded when the results are reported.

[1-6] AAP (NWSP 242.0.R2 (19))

"AAP" is an abbreviation for Absorption Against Pressure, and it means an absorption capacity of the water-absorbing agent composition or the water-absorbent resin under pressure.

[1-7] SFC

"SFC" is an abbreviation for Saline Flow Conductivity, and means liquid permeability (unit: $\times 10^{-7}$ $cm^3 \cdot sec/g$) of an aqueous sodium chloride solution through the water-absorbing agent composition or the water-absorbent resin under pressure (load: 2.07 kPa (0.3 psi)).

[1-8] Particle Size Distribution

The "particle size distribution" means a particle size distribution of the water-absorbing agent composition or the water-absorbent resin measured through sieve classification. Specifically, the particle size distribution is calculated by classifying 100.0 g of the water-absorbing agent composition or the water-absorbent resin with a sieve shaker for 10 minutes using a set of sieves having a diameter of 200 mm, sieve openings of 710 μm, 600 μm, 300 μm, 150 μm, and 45 μm, and a receptacle, and measuring the mass of the water-absorbing agent composition or the water-absorbent resin remaining on each of the sieve meshes and the receptacle (unit: mass%).

[1-9] Specific Surface Area

The "specific surface area" means a surface area (unit: $m^2/kg$) per unit mass of the water-absorbing agent composition or the water-absorbent resin.

[1-10] Gap Water Ratio Under Pressure

The "gap water ratio under pressure" means the mass of gap water per 1 g of the water-absorbing agent composition or the water-absorbent resin under a load of 0.3 psi (2.07 kPa). The "gap water" refers to the liquid retained between gel particles (gap) when 1 g of the water-absorbing agent composition or the water-absorbent resin is swollen with a 0.69 mass% aqueous sodium chloride solution under a load of 0.3 psi (2.07 kPa) to form a layer of gel particles. That is, when the water-absorbing agent composition or the water-absorbent resin is swollen with a 0.69 mass% aqueous sodium chloride solution, the gap water ratio under pressure is the mass (unit: g/g) of the aqueous sodium chloride solution held in the gap between the water-absorbing agent composition or the water-absorbent resin per 1 g of the water-absorbing agent composition or the water-absorbent resin under a load of 2.07 kPa (0.3 psi).

By measuring the gap water ratio under pressure, the liquid retention force between swollen gel particles can be grasped, and it can be used as an index for predicting the return amount when the absorbing agent is actually used for hygienic materials such as disposable diapers.

[1-11] Others

In the present description, "... acid (salt)" means a "... acid and/or a salt thereof", and "(meth)acryl" means "acryl and/or methacryl".

[2] Method for Producing Water-Absorbing Agent Composition

The water-absorbing agent composition according to the present invention is a water-absorbent resin composition containing a water-absorbent resin (preferably a polyacrylic acid (salt)-based water-absorbent resin) and optionally an additive. Hereinafter, the method for producing a water-absorbing agent composition will be described in detail.

The method for producing a water-absorbing agent composition includes a surface crosslinking step for a water-absorbent resin. The method for producing a water-absorbing agent composition may include a step of preparing an aqueous monomer solution, a polymerization step, a gel-grinding step, a drying step, a pulverizing step, a classification step, or a step of adding an additive. The production method according to the present invention may further include a

cooling step, a rewetting step, a fine powder granulation step, a transportation step, a storage step, a packing step, a warehousing step, and the like, as necessary, in addition to each step described above.

Hereinafter, each step will be described.

[2-1] Step of Preparing Aqueous Monomer Solution

This step is a step of preparing an aqueous monomer solution containing a monomer to be a raw material of the water-absorbent resin, preferably an unsaturated monomer, more preferably an unsaturated monomer having a carboxyl group, and still more preferably a monomer containing acrylic acid (salt) as a main component. The aqueous monomer solution preferably contains one or more polymerizable internal crosslinking agents. The "main component" means that the content of acrylic acid (salt) is 50 mol% or more (upper limit: 100 mol% or less), preferably 70 mol% or more, more preferably 90 mol% or more, and preferably 100 mol% or less relative to the portion excluding the internal crosslinking agent in the monomer to be subjected to the polymerization reaction. A slurry liquid of the monomer may be used within a range that does not impact the water absorption performance of the water-absorbing agent composition to be obtained as a final product. However, in the present description, an aqueous monomer solution will be described for convenience.

Acrylic Acid (Salt)

In the present invention, it is preferred to use a known acrylic acid (salt) as the monomer (hereinafter, it may be referred to as "polymerizable monomer") from the viewpoint of the physical properties and productivity of the water-absorbing agent composition or the water-absorbent resin. The known acrylic acid may contain a trace amount of a component such as a polymerization inhibitor or an impurity. As the polymerization inhibitor, methoxyphenols are preferably used, and p-methoxyphenols are more preferably used. The concentration of the polymerization inhibitor in the acrylic acid is preferably 10 ppm or more, more preferably 20 ppm or more, and preferably 200 ppm or less, more preferably 160 ppm or less, still more preferably 100 ppm or less, on a mass basis from the viewpoint of the polymerizability of the acrylic acid, the color tone of the water-absorbing agent composition or the water-absorbent resin, and the like. A preferred range of the concentration of the polymerization inhibitor in the acrylic acid can be a range defined by any combinations selected from the upper and lower limit values.

Examples of the impurity include organic compounds such as acetic acid, propionic acid, and furfural, and the compounds described in US 2008/0161512 A. Examples of the acrylic acid salt include salts obtained by neutralizing the above-described acrylic acids with the following basic compounds. The acrylic acid salt may be a commercially available acrylic acid salt or a salt obtained by neutralizing an acrylic acid.

Basic Compound

The "basic compound" in the present invention means a compound exhibiting basicity. Specifically, sodium hydroxide or the like corresponds to the above. The commercially available sodium hydroxide contains heavy metals such as zinc, lead, and iron in the order of ppm (on a mass basis), and thus can be strictly expressed as a composition. In the present invention, such a composition is also treated as being included in the category of basic compounds.

The specific examples of the basic compound include a carbonate or a hydrogen carbonate of an alkali metal, a hydroxide of an alkali metal, ammonia, and an organic amine. Among these, a strongly basic compound is selected from the viewpoint of the water absorption performance of the water-absorbing agent composition or the water-absorbent resin. Therefore, hydroxides of alkali metals such as sodium, potassium, and lithium are preferred, and sodium hydroxide is more preferred. Note that the basic compound is preferably an aqueous solution from the viewpoint of ease of handling.

Neutralization

When a salt obtained by neutralizing an acrylic acid is used as the acrylic acid salt, the neutralization may be performed at any time before polymerization, during polymerization, or after polymerization, or performed at a plurality of times or sites. From the viewpoint of production efficiency of the water-absorbing agent composition or the water-absorbent resin, neutralization is preferably performed continuously.

When an acrylic acid (salt) is used in the present invention, the neutralization percentage of the acrylic acid (salt) is preferably 10 mol% or more, more preferably 40 mol% or more, still more preferably 50 mol% or more, particularly preferably 60 mol% or more, and is preferably 90 mol% or less, more preferably 85 mol% or less, still more preferably 80 mol% or less, particularly preferably 75 mol% or less, relative to the acid groups of the monomer. A preferred range of the neutralization percentage of the acrylic acid (salt) can be a range defined by any combinations selected from the upper and lower limit values. When the neutralization percentage is within the above-described ranges, it is further easy to suppress deterioration in water absorption performance of the water-absorbing agent composition or the water-absorbent resin.

The above-described ranges for the neutralization percentage may be applied to any of the above-described neutralization timings, that is, before polymerization, during polymerization, and after polymerization. The same applies to the acid groups of the water-absorbing agent composition as a final product in addition to the acid groups of the water-absorbent resin.

Other Monomers

In the present invention, a monomer (hereinafter, referred to as "other monomer") other than the above-described acrylic acid (salt) can be used in combination with the acrylic acid (salt) as necessary. Specific examples of the additional monomer include anionic unsaturated monomers and salts thereof, such as maleic acid, maleic acid anhydride, itaconic acid, cinnamic acid, vinyl sulfonic acid, allyl toluene sulfonic acid, vinyl toluene sulfonic acid, styrene sulfonic acid, 2-(meth)acrylamide-2-methylpropane sulfonic acid, 2-(meth)acryloyl ethane sulfonic acid, 2-(meth)acryloyl propane sulfonic acid, and 2-hydroxyethyl (meth)acryloyl phosphate; mercaptan group-containing unsaturated monomers; phenolic hydroxyl group-containing unsaturated monomers; amide group-containing unsaturated monomers such as (meth)acrylamide, N-ethyl(meth)acrylamide, and N,N-dimethyl(meth)acrylamide; and amino group-containing unsaturated monomers such as N,N-dimethylaminoethyl (meth)acrylate, N,N-dimethylaminopropyl (meth)acrylate, and N,N-dimethylaminopropyl (meth)acrylamide. The additional monomers also include water-soluble or hydrophobic unsaturated monomers. When the additional monomer is used, the amount of the additional monomer used is preferably 30 mol% or less (lower limit 0 mol%), more preferably 10 mol% or less, still more preferably 5 mol% or less relative to the monomer excluding the internal crosslinking agent.

Internal Crosslinking Agent

In a preferred production method of the present invention, an internal crosslinking agent is used. Specific examples of the internal crosslinking agent include N,N'-methylenebis(meth)acrylamides, (poly)ethylene glycol di(meth) acrylates, (poly)propylene glycol di(meth)acrylates, trimethylolpropane di(meth)acrylates, trimethylolpropane tri(meth) acrylates, glycerin tri(meth)acrylates, glycerin acrylate methacrylates, ethylene oxide-modified trimethylolpropane tri(meth)acrylates, ethylene oxide-modified glycerin tri(meth)acrylates, pentaerythritol tetra(meth)acrylates, dipentaerythritol hexa(meth)acrylates, triallyl cyanurate, triallyl isocyanurate, triallyl phosphate, triallylamine, poly(meth)allyloxyalkanes, (poly)ethylene glycol diglycidyl ethers, glycerol diglycidyl ether, ethylene glycol, polyethylene glycols, propylene glycols, glycerin, pentaerythritol, ethylenediamine, polyethyleneimines, and glycidyl (meth)acrylates. One of these internal crosslinking agents may be used alone, or two or more thereof may be used in combination. Among these internal crosslinking agents, one or more internal crosslinking agents are selected in consideration of reactivity and the like. From the viewpoint of water absorption performance or the like of the water-absorbing agent composition or the water-absorbent resin, an internal crosslinking agent having two or more polymerizable unsaturated groups is preferably selected, an internal crosslinking agent that is thermally decomposable at the drying temperature described later is more preferably selected, and an internal crosslinking agent having a (poly)alkylene glycol structure and having two or more polymerizable unsaturated groups is still more preferably selected.

Specific examples of the polymerizable unsaturated group include an allyl group and a (meth)acrylate group. Among these, a (meth)acrylate group is preferred. Specific examples of the (poly)alkylene glycol structure include polyethylene glycol. Specific examples of the internal crosslinking agent having a (poly)alkylene glycol structure include polyethylene glycol, (poly)ethylene glycol di(meth)acrylate, and (poly)propylene glycol di(meth)acrylate. The number of alkylene glycol units (hereinafter, it may be denoted to as "n") is preferably 1 or more, more preferably 6 or more, and preferably 100 or less, more preferably 50 or less, still more preferably 20 or less, particularly preferably 10 or less. A preferred range of the number of alkylene glycol units can be a range defined by any combinations selected from the upper and lower limit values. The number of alkylene glycol units in the production method according to the present invention is preferably 1 or more and 100 or less, more preferably 6 or more and 50 or less, still more preferably 6 or more and 20 or less, particularly preferably 6 or more and 10 or less. The number of alkylene glycol units is an average of numbers having a distribution.

The amount of the internal crosslinking agent used is preferably 0.0001 mol% or more, more preferably 0.001 mol% or more, still more preferably 0.01 mol% or more, and preferably 10 mol% or less, more preferably 5 mol% or less, still more preferably 1 mol% or less, relative to the monomer excluding the internal crosslinking agent. A preferred range of the amount of the internal crosslinking agent used can be a range defined by any combination selected from the upper and lower limit values described above. The amount of the internal crosslinking agent used is, for example, preferably 0.0001 mol% or more and 10 mol% or less, more preferably 0.001 mol% or more and 5 mol% or less, still more preferably 0.01 mol% or more and 1 mol% or less, relative to the monomer excluding the internal crosslinking agent. When the amount used is within these ranges, an increase in the water-soluble content of the water-absorbing agent composition or the water-absorbent resin and a decrease in the absorption capacity are suppressed, and the water-absorbing agent

composition or the water-absorbent resin having desired water absorption performance (for example, the absorption capacity under pressure, the gap water ratio under pressure, and the liquid permeation rate under pressure (SFC or GBP) are high) is more likely to be obtained.

The internal crosslinking agent is preferably added in advance at the time of producing the aqueous monomer solution, and in this case, the crosslinking reaction is performed simultaneously with the polymerization reaction. On the other hand, the polymerization reaction may be initiated without adding the internal crosslinking agent, and the internal crosslinking agent may then be added during or after the polymerization reaction to cause a crosslinking reaction. These techniques may also be used in combination. In addition, self-crosslinking can be performed without using an internal crosslinking agent.

Substances to Be Added to Aqueous Monomer Solution

In the present invention, from the viewpoint of improving the physical properties of the water-absorbing agent composition or the water-absorbent resin, the following substances may be added to the aqueous monomer solution during the polymerization reaction and the crosslinking reaction or at any one or more points after the polymerization reaction and the crosslinking reaction at the time of producing the aqueous monomer solution. Specific examples of the substances include hydrophilic polymers such as starch, starch derivatives, cellulose, cellulose derivatives, polyvinyl alcohol (hereinafter, it may be referred to as "PVA"), polyacrylic acid (salt), and a crosslinked body of polyacrylic acid (salt); and compounds such as carbonates, azo compounds, foaming agents that generate various bubbles, surfactants, chelating agents, and chain transfer agents.

The amount of the hydrophilic polymer added is preferably 50 mass% or less, more preferably 20 mass% or less, still more preferably 10 mass% or less, particularly preferably 5 mass% or less, and preferably 0 mass% or more, more preferably more than 0 mass%, relative to the aqueous monomer solution. The amount of the compound added is preferably 5 mass% or less, more preferably 1 mass% or less, still more preferably 0.5 mass% or less, and preferably 0 mass% or more, more preferably more than 0 mass%, relative to the aqueous monomer solution.

When a water-soluble resin or a water-absorbent resin is used as the hydrophilic polymer, a graft polymer such as a starch-acrylic acid (salt) copolymer or a PVA-acrylic acid (salt) copolymer, or a water-absorbent resin composition is obtained. These graft polymers or water-absorbent resin compositions are also within the scope of the polyacrylic acid (salt)-based water-absorbent resin according to the present invention.

Concentration of Monomer Component

Various substances and components (hereinafter, referred to as "monomer component") described above are selected according to the purpose, and the amounts thereof are defined so as to satisfy the above-described ranges, and mixed with each other, whereby an aqueous monomer solution is produced. In the present invention, the monomer may be a mixed solution of water and a hydrophilic solvent in addition to an aqueous solution, and such a form is also referred to as an aqueous monomer solution.

The total concentration of the monomer components is preferably 10 mass% or more, more preferably 20 mass% or more, still more preferably 30 mass% or more, and preferably 80 mass% or less, more preferably 75 mass% or less, still more preferably 70 mass% or less, from the viewpoint of the physical properties of the water-absorbing agent composition or the water-absorbent resin. A preferred range of the total concentration of the monomer components can be a range defined by any combinations selected from the upper and lower limit values. The concentration of the monomer component is calculated from the following (Equation I).

$$\text{Concentration of monomer component (mass\%)} = \{(\text{mass of monomer component})/(\text{mass of aqueous monomer solution})\} \times 100 \cdots (\text{Equation I}) \quad (\text{Equation I})$$

In (Equation I), the "mass of aqueous monomer solution" does not include the mass of the graft component, the water-absorbent resin, or the hydrophobic organic solvent in the reverse phase suspension polymerization.

[2-2] Polymerization Step

This step is a step of polymerizing the aqueous monomer solution containing a monomer containing acrylic acid (salt) as a main component and one or more polymerizable internal crosslinking agents obtained in the step of preparing an aqueous monomer solution to obtain a hydrogel.

Polymerization Initiator

In the present invention, a polymerization initiator is used at the time of polymerization. Examples of the polymerization initiator include thermally decomposable polymerization initiators, photo-decomposable polymerization initiators, or redox-based polymerization initiators that use a reducing agent in combination to accelerate the decomposition of these polymerization initiators. Specific examples of the polymerization initiator include radical polymerization initiators such as sodium persulfate, potassium persulfate, ammonium persulfate, t-butyl hydroperoxide, hydrogen peroxide, and 2,2'-azobis(2-amidinopropane)dihydrochloride. Among these polymerization initiators, one or more polymerization initiators are selected in consideration of the type of polymerization and the like. From the viewpoint of the handleability of the polymerization initiator and the physical properties of the water-absorbing agent composition or the water-absorbent resin, a peroxide or an azo compound is preferably selected as the polymerization initiator, a peroxide is more preferably selected as the polymerization initiator, and a persulfate is still more preferably selected as the polymerization initiator. When an oxidative radical polymerization initiator is used, redox polymerization may be performed using a reducing agent such as sodium sulfite, sodium hydrogen sulfite, ferrous sulfate, or L-ascorbic acid in combination. When a peroxide is used as the polymerization initiator, the peroxide may be the same as or different from the peroxide used in the surface crosslinking step described later, but is preferably the same (preferably, both are persulfates, further, sodium persulfate).

The amount of the polymerization initiator used is preferably 0.001 mol% or more, more preferably 0.01 mol% or more, and preferably 1 mol% or less, more preferably 0.5 mol% or less, still more preferably 0.1 mol% or less, relative to the monomer excluding the internal crosslinking agent. The amount of the reducing agent used is preferably 0.0001 mol% or more, more preferably 0.0005 mol% or more, and preferably 0.02 mol% or less, more preferably 0.015 mol% or less, relative to the monomer excluding the internal crosslinking agent. Preferable ranges of the amount of the polymerization initiator used and the amount of the reducing agent used can be ranges defined by any combinations selected from the upper and lower limit values. By setting the amount used within these ranges, the water-absorbing agent composition or the water-absorbent resin having desired water absorption performance is more easily obtained.

In the present invention, the polymerization reaction may be started through irradiation with an active energy ray such as a radiation, an electron beam, or an ultraviolet ray. Irradiation with an active energy ray and the polymerization initiator may be used in combination.

Type of Polymerization

Examples of the type of polymerization applicable to the present invention include aqueous solution polymerization, reverse phase suspension polymerization, spray polymerization, droplet polymerization, bulk polymerization, and precipitation polymerization. Among these, from the viewpoint of ease of polymerization control and water absorption performance of the water-absorbing agent composition or the water-absorbent resin, aqueous solution polymerization or reverse phase suspension polymerization is preferably selected, aqueous solution polymerization is more preferably selected, and continuous aqueous solution polymerization is still more preferably selected. The reverse phase suspension polymerization is described in WO 2007/004529, WO 2012/023433, and the like. Examples of the continuous aqueous solution polymerization described above include continuous belt polymerization described in US 4893999, US 6906159, US 7091253, US 7741400, US 8519212, JP 2005-36100 A, and the like, and continuous kneader polymerization described in US 6987151, and the like.

Preferable forms of the continuous aqueous solution polymerization include high-temperature initiation polymerization, high-concentration polymerization, and foaming polymerization. The "high-temperature initiation polymerization" is a type of polymerization in which the temperature of the aqueous monomer solution when polymerization is initiated is preferably set to 30°C or higher, more preferably 35°C or higher, still more preferably 40°C or higher, particularly preferably 50°C or higher, with the upper limit being the boiling point of the aqueous monomer solution. The "high-concentration polymerization" is a type of polymerization in which the monomer concentration when polymerization is initiated is preferably 30 mass% or more, more preferably 35 mass% or more, still more preferably 40 mass% or more, and particularly preferably 42 mass% or more, with the upper limit being the saturation concentration of the aqueous monomer solution. The "foaming polymerization" refers to a type of polymerization in which the aqueous monomer solution containing a foaming agent or bubbles is polymerized. Each of these types of polymerization may be performed alone, or two or more thereof may be used in combination.

The foaming polymerization is one of methods for improving the specific surface area of the present invention, and is one of preferred embodiments. Examples of the method for dispersing bubbles in the foaming polymerization include:

(I) a method in which a gas dissolved in the aqueous monomer solution is dispersed as bubbles by decreasing the solubility;

(II) a method in which a gas is introduced from the outside and dispersed as bubbles; and
(III) a method in which a foaming agent is added to the aqueous monomer solution and foaming is performed. The dispersion methods may be used in combination depending on the water absorption performance of the water-absorbing agent composition or the water-absorbent resin. The bubbles dispersed in the aqueous monomer solution are confined in the gel when the aqueous monomer solution is gelled with the progress of the polymerization reaction, and the resulting gel has a foamed shape. Thus, the polymerization is referred to as foaming polymerization in the present invention.

Examples of the gas dissolved in the aqueous monomer solution in (I) include oxygen used for stabilizing the monomer, nitrogen as an inert gas, carbon dioxide, and ozone, and mixed gases thereof.

In the case of (II) a method in which a gas is introduced from the outside and dispersed as bubbles, specific examples of the gas include oxygen, air, nitrogen, carbon dioxide, and ozone, and mixed gases thereof. Among these, from the viewpoint of polymerizability and cost, an inert gas such as nitrogen or carbon dioxide is preferably used, and nitrogen is more preferably used.

In the case of (III) a method in which a foaming agent is added to the aqueous monomer solution and foaming is performed, specific examples of the foaming agent include azo compounds, organic or inorganic carbonate solutions, dispersions, and powders having a particle size of 0.1 μm or more and 1000 μm or less. Carbonates such as sodium carbonate, ammonium carbonate, and magnesium carbonate, and hydrogen carbonate are preferably used. In the aqueous monomer solution containing the foaming agent or bubbles, a surfactant may be used for stably holding the bubbles.

In the methods (I) to (III) listed as the method for dispersing the bubbles in the foaming polymerization, a surfactant may be used in combination. Examples of the surfactant include an anionic surfactant, a nonionic surfactant, a cationic surfactant, an amphoteric surfactant, a fluorine-based surfactant, and an organometallic surfactant. Specific examples thereof include surfactants described in WO 97/017397 and US 6107358.

Each of the types of polymerization described above can be performed in an air atmosphere, but from the viewpoint of the color tone of the water-absorbing agent composition or the water-absorbent resin, the polymerization is preferably performed in an inert gas atmosphere such as nitrogen or argon, and more preferably in an atmosphere having an oxygen concentration of 1 vol% or less. It is also preferred to sufficiently substitute dissolved oxygen in the aqueous monomer solution using an inert gas, and it is more preferred to set the amount of dissolved oxygen to less than 1 mg/L.

By forming a hydrogel, a water-absorbent resin, or a water-absorbing agent composition in a foamed shape through foaming polymerization, the water absorption speed of the water-absorbing agent composition or the water-absorbent resin is increased, and the water-absorbing agent composition is easily immobilized on an absorbent article, which is preferred. The foamed shape can be confirmed by observing pores on the particle surface with an electron microscope. As the size of the hole, a hole having a diameter of 1 μm or more and 100 μm or less is exemplified. The lower limit value of the number of pores is preferably 1 or more, more preferably 10 or more per particle of the water-absorbing agent composition or the water-absorbent resin. On the other hand, the upper limit value thereof is preferably 10000 or less, more preferably 1000 or less. The pores can be controlled through the foaming polymerization. The foaming polymerization is a preferred technique for increasing the specific surface area of the water-absorbing agent composition and the water-absorbent resin.

[2-3] Gel-Grinding Step

The gel-grinding step is a step of performing gel-grinding on the hydrogel obtained in the polymerization step to obtain a hydrogel having a particulate shape (hereinafter, also referred to as "particulate hydrogel"). To distinguish from "pulverizing" in the pulverizing step described later, the grinding in the present step is referred to as "gel-grinding". The "gel-grinding" means that the hydrogel is adjusted to a predetermined size using a gel-grinding apparatus such as a kneader, a meat chopper, or a cutter mill.

As to embodiments, operating conditions and the like of the gel-grinding, the contents described in JP 5989913 B or JP 6067126 B are also preferably applied to the present invention. When the type of polymerization is kneader polymerization, the polymerization step and the gel-grinding step are performed simultaneously. When the particulate hydrogel is obtained in the polymerization step through polymerization such as reverse phase suspension polymerization, spray polymerization, or droplet polymerization, the gel-grinding step is regarded to be performed simultaneously with the polymerization step. By undergoing the gel-grinding step in the present invention, a water-absorbing agent composition or a water-absorbent resin in an irregularly crushed shape can be obtained.

The particle diameter of the particulate hydrogel that has been finely granulated by the gel-grinding step is preferably 0.05 mm or more and 10 mm or less. When the particle diameter of the particulate hydrogel is too small, the physical properties of the resulting water-absorbing agent composition or water-absorbent resin may deteriorate. On the other hand, when the particle diameter of the particulate hydrogel is too large, drying of the particulate hydrogel may be

insufficient.

D50 (mass-average particle diameter) of the particulate hydrogel is preferably 50 μm or more, more preferably 100 μm or more, still more preferably 140 μm or more, and preferably 2000 μm or less, more preferably 1500 μm or less, still more preferably 1000 μm or less. A preferred range of the D50 (mass-average particle diameter) of the particulate hydrogel can be a range defined by any combinations selected from the upper and lower limit values. For example, controlling D50 (mass-average particle diameter) of the particulate hydrogel to fall within the above preferred ranges by the following method (B) is one of the methods for improving the specific surface area of the water-absorbing agent composition or the water-absorbent resin, and is one of preferred aspects.

As the PSD (particle size) of the particulate hydrogel, σζ (logarithmic standard deviation) indicating the narrowness of the particle size distribution is preferably 0.2 or more, and preferably 1.5 or less, more preferably 1.3 or less, still more preferably 1.2 or less. The smaller the value of σζ (logarithmic standard deviation of particle size distribution) is, the more uniform the particle size is, and there is an advantage that more uniform drying can be performed. However, to set σζ (logarithmic standard deviation of particle size distribution) to less than 0.2, it is necessary to perform special operations such as particle size control at the time of polymerization before gel-grinding and classification of a particulate hydrogel after gel-grinding, and thus, it is substantially difficult to perform it from the viewpoint of productivity and cost.

In the present invention, it is desirable to control any one or more methods of (A) foaming polymerization of the aqueous monomer solution, (B) pulverization and granulation of a particulate hydrogel or a dried polymer thereof, and (C) fine powder recycling so that the specific surface area of the water-absorbent resin is 25 $m^2$/kg or more.

The specific surface area of the water-absorbent resin can be increased to 25 $m^2$/kg or more by adopting, as (A) foaming polymerization of the aqueous monomer solution, for example, a foaming polymerization method in which a surfactant is made to coexist in the aqueous monomer solution, that is, a foaming polymerization method described in JP 5647625 B (specifically, for example, a method of generating bubbles in the aqueous monomer solution by reducing the solubility of a dissolved gas in the aqueous monomer solution in the presence of a surfactant), a foaming polymerization method in which a gas is externally introduced into the aqueous monomer solution and dispersed as bubbles to perform polymerization, a foaming polymerization method in which a foaming agent is added to the aqueous monomer solution and foaming is performed, or the like. Thus, it is also preferred that the water-absorbent resin is obtained by foaming polymerization of an aqueous unsaturated monomer solution.

The specific surface area of the water-absorbent resin can be increased to 25 $m^2$/kg or more by adopting, as (B) granulation of the particulate hydrogel or a dried polymer thereof, for example, a gel-grinding method described in JP 5989913 B, JP 6067126 B, or WO 2016/204302 in the gel-grinding step, and by further performing drying. A water-absorbent resin having a desired specific surface area can also be obtained by appropriately controlling the die hole diameter, the number of holes, the die thickness, the amount of hot water added, the rotational speed of the screw shaft, and the like of a gel-grinding apparatus such as a meat chopper. The granulation may be performed on the hydrogel at the time of polymerization, may be performed simultaneously with drying of the finely pulverized product of the hydrogel after polymerization, or may be performed using water and/or an organic or inorganic binder for the finely pulverized product after drying. Thus, it is also preferred to contain a granulated product of a hydrogel of the water-absorbent resin or a dried product thereof.

The specific surface area of the water-absorbent resin can be increased to 25 $m^2$/kg or more by adopting, as (C) fine powder recycling, for example, recovering fine powder of the water-absorbent resin that has passed through a sieve with a mesh size of 150 μm in the polymerization step, the gel-grinding step, or the drying step, or granulating and recovering the fine powder. Thus, it is also preferred that the water-absorbent resin contains a fine recycled product of the water-absorbent resin. The methods (A) to (C) may be performed alone or in combination.

As the method for increasing the specific surface area of the water-absorbent resin to 25 $m^2$/kg or more, there is also a method of containing a large amount of particles having a small particle diameter. However, in this method, a large amount of particles having a small particle diameter, in particular, fine powder passing through a sieve with a mesh diameter of 150 μm is contained. As a result, gel blocking of the resulting water-absorbing agent composition is likely to occur, and liquid absorbing performance and liquid permeability under pressure deteriorate, which is not preferred. Thus, when the specific surface area is adjusted using the fine powder, it is preferred to adopt the method of (B) and/or (C) described above. In the present invention, it is preferred to perform the adjustment method described later with sufficient attention to the adjustment of the particle size distribution.

D50 (mass-average particle diameter) and σζ (logarithmic standard deviation of particle size distribution) of the particulate hydrogel are measured by the method described in [0257] to [0270] of WO 2016/111223, which is incorporated herein by reference.

[2-4] Drying Step

This step is a step of producing a dried polymer by drying the hydrogel and/or the particulate hydrogel obtained

through the polymerization step and/or the gel-grinding step to have a desired resin solid content. The resin solid content of the dried polymer is determined from a mass change when 1 g of the water-absorbent resin is heated at 180°C for 3 hours, and is preferably 80 mass% or more, more preferably 85 mass% or more, still more preferably 90 mass% or more, particularly preferably 92 mass% or more, and is preferably 99 mass% or less, more preferably 98 mass% or less, still more preferably 97 mass% or less. A preferred range of the resin solid content of the dried polymer can be a range defined by any combinations selected from the upper and lower limit values.

Specific examples of the method for drying the hydrogel and/or the particulate hydrogel include thermal drying, hot air drying, drying under reduced pressure, fluidized bed drying, infrared drying, microwave drying, drum dryer drying, drying through azeotropic dehydration with a hydrophobic organic solvent, and high humidity drying with the use of high-temperature water vapor. Among these, from the viewpoint of drying efficiency, hot air drying is preferred, and band drying in which hot air drying is performed on a ventilation belt is more preferred.

The drying temperature in the hot air drying is preferably 100°C or higher, more preferably 150°C or higher, and preferably 300°C or lower, more preferably 200°C or lower, from the viewpoint of the color tone and drying efficiency of the water-absorbing agent composition or the water-absorbent resin. A preferred range of the drying temperature can be a range defined by any combinations selected from the upper and lower limit values. The drying temperature in hot air drying is defined by the temperature of hot air. Drying conditions other than the drying temperature, such as hot air speed and drying time, may be appropriately set according to the water content and total mass of the particulate hydrogel to be dried and the target resin solid content. When band drying is performed, various conditions described in WO 2006/100300, WO 2011/025012, WO 2011/025013, WO 2011/111657, and the like, are appropriately applied.

The drying time in the present invention is preferably 1 minute or more, more preferably 5 minutes or more, still more preferably 10 minutes or more, and preferably 10 hours or less, more preferably 3 hours or less, still more preferably 1 hour or less. A preferred range of the drying time can be a range defined by any combinations selected from the upper and lower limit values. By setting the drying temperature and the drying time within these ranges, the physical properties of the resulting water-absorbing agent composition can be set within desired ranges. In addition, the physical properties of the water-absorbent resin as an intermediate product can be set within a desired range. When drying is performed by hot air drying, the wind speed of the hot air is preferably 0.5 m/s or more, and preferably 3.0 m/s or less, more preferably 2.0 m/s or less. Other drying conditions may be appropriately set according to the water content and the total mass of the particulate hydrogel to be dried, the intended solid content, and the like.

[2-5] Pulverizing Step and Classification Step

This step is a step of pulverizing the dried polymer obtained through the drying step in the pulverizing step and then adjusting the particle size to a desired range in the classification step. In this step, a water-absorbent resin before surface crosslinking is obtained. Through the pulverizing step after drying, a water-absorbent resin in an irregularly crushed shape is obtained.

Specific examples of the pulverizing apparatus used in the pulverizing step include high-speed rotary pulverizing apparatuses such as a roll mill, a hammer mill, a screw mill, and a pin mill; vibration mills, knuckle type pulverizing apparatuses, and cylindrical mixers. Among these, a roll mill is preferably selected from the viewpoint of pulverizing efficiency. A plurality of these pulverizing apparatuses can be used in combination.

Examples of the method for adjusting the particle size in the classification step include air flow classification and sieve classification using JIS standard sieves (JIS Z 8801-1 (2000)). Among these, the sieve classification is preferably selected from the viewpoint of classification efficiency. The particle size adjustment of the water-absorbing agent composition or the water-absorbent resin is not limited to in the pulverizing step and/or the classification step, and it may be performed in the polymerization step, particularly, reverse phase suspension polymerization, droplet polymerization, or the like, or in other steps, for example, a granulation step or a fine powder recovery step.

The proportion of (i) particles having a particle diameter of less than 150 μm contained in the water-absorbent resin before surface crosslinking after classification is preferably 3 mass% or less, more preferably less than 3 mass%, still more preferably 2.5 mass% or less, yet still more preferably 2 mass% or less. In continuous commercial production, it may be very difficult to set the proportion of particles of less than 150 μm to 0 mass% from the viewpoint of production efficiency. Thus, the proportion of (i) particles having a particle diameter of less than 150 μm contained in the water-absorbent resin before surface crosslinking after classification is preferably more than 0 mass%, more preferably 0.1 mass% or more, still more preferably 0.2 mass% or more, yet still more preferably 0.3 mass% or more. A preferred range of the proportion of the particles having a particle diameter of less than 150 μm can be a range defined by any combinations selected from the upper and lower limit values. Thus, the proportion of (i) particles having a particle diameter of less than 150 μm contained in the water-absorbent resin before surface crosslinking after classification may be, for example, more than 0 mass% and 3 mass% or less, more than 0 mass% and less than 3 mass%, 0.1 mass% or more and 2.5 mass% or less, 0.2 mass% or more and 2 mass% or less, or 0.3 mass% or more and 2 mass% or less. When the proportion of (i) particles having a particle diameter of less than 150 μm contained in the water-absorbent resin before surface crosslinking after classification is

within the ranges described above, it becomes easier to control AAP (absorption capacity under pressure) and SFC (saline flow conductivity) in a more balanced manner, the surface crosslinking agent is easily dispersed uniformly, and the performance of the water-absorbing agent composition improves, which is preferred.

(ii) D50 (mass-average particle diameter) of the water-absorbent resin before surface crosslinking after classification is preferably 250 μm or more, more preferably 300 μm or more, still more preferably 330 μm or more, and preferably less than 550 μm, more preferably less than 500 μm, still more preferably less than 450 μm. A preferred range of (ii) D50 (mass-average particle diameter) of the water-absorbent resin before surface crosslinking after classification can be a range defined by any combination selected from the upper and lower limit values. (ii) D50 (mass-average particle diameter) of the water-absorbent resin before surface crosslinking after classification is, for example, preferably 250 μm or more and less than 550 μm, more preferably 300 μm or more and less than 500 μm, still more preferably 330 μm or more and less than 450 μm. When (ii) D50 (mass-average particle diameter) of the water-absorbent resin before surface crosslinking after classification is within the ranges described above, AAP (absorption capacity under pressure) and SFC (saline flow conductivity) can be more easily controlled in a well-balanced manner, which is preferred.

Further, in (iii) the particle size distribution of the water-absorbent resin before surface crosslinking, it is more preferred that D50 (mass-average particle diameter) falls within the range of (ii), and the proportion of particles having a particle diameter of less than 150 μm falls within the range of (i). In continuous commercial production, it may be very difficult to set the proportion of particles of less than 150 μm to 0 mass% from the viewpoint of production efficiency. Thus, the proportion is preferably more than 0 mass%, more preferably 0.1 mass% or more, still more preferably 0.2 mass% or more, yet still more preferably 0.3 mass% or more. Specifically, D50 (mass-average particle diameter) of the water-absorbent resin before surface crosslinking is 250 μm or more and less than 550 μm, 300 μm or more and less than 500 μm, or 330 μm or more and less than 450 μm, and the mass proportion of particles having a particle diameter of less than 150 μm contained in the water-absorbent resin is more than 0 mass% and 3 mass% or less, more than 0 mass% and less than 3 mass%, 0.1 mass% or more and 2.5 mass% or less, 0.2 mass% or more and 2 mass% or less, or 0.3 mass% or more and 2 mass% or less.

Further, (iv) $\sigma\zeta$ (logarithmic standard deviation of particle size distribution) is preferably 0.20 or more, more preferably 0.25 or more, still more preferably 0.27 or more, and preferably 0.50 or less, more preferably 0.40 or less, still more preferably 0.35 or less. The preferred range of $\sigma\zeta$ (logarithmic standard deviation of particle size distribution) can be a range defined by any combinations selected from the upper and lower limit values. $\sigma\zeta$ (Logarithmic standard deviation of particle size distribution) has an advantage that the smaller the value is, the more uniform the particle size is and the less segregation of particles is. However, to make the $\sigma\zeta$ (logarithmic standard deviation of particle size distribution) excessively small, it is necessary to remove coarse particles and fine particles by repeating pulverization and classification, which may bring a disadvantage from the viewpoint of productivity and cost.

The above-described particle sizes and the like, that is, the above-described (i) to (iv) are applied not only to the water-absorbent resin before surface crosslinking, but also to the water-absorbent resin after surface crosslinking and the water-absorbing agent composition. Thus, it is preferred to perform the surface crosslinking treatment, that is, the surface crosslinking step so as to maintain the particle size in the above-described range adjusted with the water-absorbent resin before surface crosslinking, and it is more preferred to perform the particle size adjustment by providing the particle size adjustment step after the surface crosslinking step. In the present invention, the above-described (i) and (iv), (ii) and (iv), (iii) and (iv), and the like can be freely selected and combined, and in such cases, the respective preferred ranges can be freely combined.

The specific surface area of the water-absorbent resin before surface crosslinking is 25 $m^2$/kg or more. Even when the specific surface area of the water-absorbent resin before surface crosslinking is 25 $m^2$/kg or more, a water-absorbing agent composition having a high water absorption speed, a high absorption capacity under pressure, and a high gap water ratio under pressure is obtained according to the production method and the water-absorbing agent composition of the present invention. The specific surface area of the water-absorbent resin before surface crosslinking is preferably as high as possible, and is preferably 26 $m^2$/kg or more, 27 $m^2$/kg or more, 28 $m^2$/kg or more, 29 $m^2$/kg or more, 30 $m^2$/kg or more in this order, and it may be 36 $m^2$/kg or more. The upper limit of the specific surface area of the water-absorbent resin before surface crosslinking is preferably 60 $m^2$/kg or less, and 55 $m^2$/kg or less, in this order. From the viewpoint of increasing the water absorption speed, it is desirable that the specific surface area is as high as possible. However, when the specific surface area is too high, excessive foaming polymerization in the polymerization step or too fine gel-grinding in the gel-grinding step is required, and as a result, there is a possibility that AAP (absorption capacity under pressure) and SFC (saline flow conductivity) decrease. On the other hand, when the specific surface area of the water-absorbent resin is too small, it is difficult to obtain a water-absorbent resin having a desired water absorption speed, which is not preferred. A preferred range of the specific surface area of the water-absorbent resin before surface crosslinking can be a range defined by any combinations selected from the upper and lower limit values. For example, the specific surface area may be 25 $m^2$/kg or more and 60 $m^2$/kg or less, 26 $m^2$/kg or more and 60 $m^2$/kg or less, 27 $m^2$/kg or more and 60 $m^2$/kg or less, 28 $m^2$/kg or more and 60 $m^2$/kg or less, 29 $m^2$/kg or more and 60 $m^2$/kg or less, 30 $m^2$/kg or more and 55 $m^2$/kg or less, or 36

$m^2/kg$ or more and 55 $m^2/kg$ or less. The specific surface area of the water-absorbent resin before surface crosslinking can be controlled by conditions for grinding the hydrogel, and the like.

The shape of the water-absorbent resin before surface crosslinking may be any of a spherical shape, a granulated product, an aggregate, an irregularly crushed shape, and the like, but in consideration of the water absorption speed of the water-absorbent resin, an irregularly crushed shape is preferred. Here, the irregularly crushed shape is a crushed particle having a nonuniform shape. The water-absorbent resin according to one embodiment of the present invention is preferably a pulverized product obtained through aqueous solution polymerization. On the other hand, when the pulverizing step is not performed, typically, a granulated product of spherical particles or spherical particles obtained by reverse phase suspension polymerization, spray droplet polymerization in which a polymerization monomer is sprayed or dropped and polymerized, or the like do not have an irregularly crushed shape. In one embodiment of the present invention, the average circularity of the water-absorbent resin before surface crosslinking is preferably 0.83 or less, more preferably 0.80 or less, still more preferably 0.75 or less.

[2-6] Surface Crosslinking Step

This step is a step of further providing a portion having a high crosslinking density on the surface layer of the water-absorbent resin before surface crosslinking obtained through the steps described above, and includes a mixing step and a heat treatment step. In the surface crosslinking step, radical crosslinking, surface polymerization, a crosslinking reaction with a surface crosslinking agent, and the like occur on the surface of the water-absorbent resin before the surface crosslinking step, and thus a surface crosslinked water-absorbent resin is obtained.

[2-6-1] Mixing Step

This step is a step of obtaining a humidified mixture by mixing a surface crosslinking agent, preferably a solution containing a surface crosslinking agent (hereinafter, referred to as "surface crosslinking agent solution") with the water-absorbent resin before surface crosslinking in a mixing apparatus.

(Organic Surface Crosslinking Agent)

In the present invention, an organic surface crosslinking agent capable of reacting with a carboxyl group at the time of surface crosslinking is used. Specific examples of the organic surface crosslinking agent include a polyhydric alcohol compound, an amino alcohol, an alkylene carbonate compound, an oxazolidinone compound, an oxetane compound, and an epoxy compound. It is preferred to use at least one or more organic surface crosslinking agents selected from these organic surface crosslinking agents. As the organic surface crosslinking agent, an organic surface crosslinking agent capable of forming an ester bond with a carboxyl group is preferred. Examples of the organic surface crosslinking agent that forms an ester bond, preferably, a dehydrated ester bond, with a functional group of the polyacrylic acid (salt)-based water-absorbent resin, for example, a carboxyl group, include organic surface crosslinking agents having a hydroxyl group in the molecule, such as a polyhydric alcohol compound or an amino alcohol compound, and organic surface crosslinking agents that generate a hydroxyl group via ring-opening, such as an alkylene carbonate compound, an oxazolidinone compound, an oxetane compound, and an epoxy compound.

More specific examples of the organic surface crosslinking agent include polyhydric alcohol compounds such as ethylene glycol, diethylene glycol, triethylene glycol, tetraethylene glycol, polyethylene glycol, propylene glycol, 1,3-propanediol, 1-methyl-1,3-propanediol, 2-methyl-1,3-propanediol, dipropylene glycol, 2,2,4-trimethyl-1,3-pentanediol, 2,3,4-trimethyl-1,3-pentanediol, polypropylene glycol, glycerin, polyglycerin, 2-butene-1,4-diol, 1,4-butanediol, 1,3-butanediol, 1,5-pentanediol, 1,6-hexanediol, 1,2-cyclohexanemethanol, 1,2-cyclohexanedimethanol, 1,2-cyclohexanediol, trimethylolpropane, diethanolamine, triethanolamine, polyoxypropylene, oxyethylene-oxypropylene block copolymers, pentaerythritol, meso erythritol, D-sorbitol, and sorbitol; epoxy compounds such as ethylene glycol diglycidyl ether, polyethylene glycol diglycidyl ether, glycerol polyglycidyl ether, diglycerol polyglycidyl ether, polyglycerol polyglycidyl ether, propylene glycol diglycidyl ether, polypropylene glycol diglycidyl ether, and glycidol; polyvalent amine compounds such as ethylenediamine, diethylenetriamine, triethylenetetramine, tetraethylenepentamine, pentaethylenehexamine, polyethyleneimine, and polyamidepolyamine, and inorganic salts or organic salts thereof, for example, aziridinium salts, and the like; polyvalent isocyanate compounds such as 2,4-tolylene diisocyanate and hexamethylene diisocyanate; haloepoxy compounds such as epichlorohydrin, epibromhydrin, and $\alpha$-methylepichlorohydrin; polyvalent oxazoline compounds such as 1,2-ethylene bisoxazoline; oxazolidinone compounds such as N-acyl oxazolidinone and 2-oxazolidinone; alkylene carbonate compounds such as 1,3-dioxolan-2-one(ethylene carbonate), 4-methyl-1,3-dioxolan-2-one, 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, 4,6-dimethyl-1,3-dioxan-2-one, 1,3-dioxopan-2-one; cyclic urea compounds; oxetane compounds such as oxetane, 2-methyloxetane, 3-methyl-3-hydroxymethyloxetane, and 3-

ethyl-3-hydroxymethyloxetane; and amino alcohol compounds such as ethanolamine. The organic surface crosslinking agent and an inorganic surface crosslinking agent such as a polyvalent metal compound of a hydroxide or a chloride of zinc, calcium, magnesium, aluminum, iron, zirconium, or the like may be used in combination. One of these organic surface crosslinking agents may be used alone, or two or more thereof may be used in combination.

Among the organic surface crosslinking agents, at least one organic surface crosslinking agent selected from the group consisting of a polyhydric alcohol compound, an epoxy compound, a polyvalent amine compound and salts thereof, an oxetane compound, and an alkylene carbonate compound is preferred, and at least one organic surface crosslinking agent selected from the group consisting of a polyhydric alcohol compound and an alkylene carbonate compound is more preferred. The organic surface crosslinking agent is preferably one or more selected from the group consisting of a polyhydric alcohol having 3 or more and 8 or less carbon atoms (preferably 3 or more and 6 or less carbon atoms) and having 2 or more and 3 or less hydroxyl groups contained in the molecule, an epoxy compound having 6 or more and 12 or less carbon atoms, an alkylene carbonate having 3 or more and 5 or less carbon atoms, and an oxetane compound having 3 or more and 10 or less carbon atoms, more preferably one or more selected from the group consisting of a polyhydric alcohol having 3 or more and 8 or less carbon atoms (preferably 3 or more and 6 or less carbon atoms) and having 2 or more and 3 or less hydroxyl groups contained in the molecule and an alkylene carbonate having 3 or more and 5 or less carbon atoms. As the organic surface crosslinking agent described above, one or two or more organic surface crosslinking agents are used (two of these may be used in combination) in consideration of the reactivity thereof and the heating temperature in the heat treatment step. The organic surface crosslinking step may be performed twice or more in consideration of the effect, and in this case, the second and subsequent steps may be performed using the same organic surface crosslinking agent as in the first step, or may be performed using different organic surface crosslinking agents.

The amount of the organic surface crosslinking agent used is preferably 0.01 parts by mass or more, and preferably 10 parts by mass or less, more preferably 5 parts by mass or less, still more preferably 2 parts by mass or less, relative to 100 parts by mass of the water-absorbent resin before surface crosslinking. The amount of the organic surface crosslinking agent used is preferably 0.01 parts by mass or more and 10 parts by mass or less, more preferably 0.01 parts by mass or more and 5 parts by mass or less, still more preferably 0.01 parts by mass or more and 2 parts by mass or less, relative to 100 parts by mass of the water-absorbent resin before surface crosslinking. By setting the amount of the organic surface crosslinking agent used within the above-described ranges, an optimum crosslinked structure can be formed on the surface layer of the water-absorbent resin before surface crosslinking, and a water-absorbent resin and a water-absorbing agent composition having higher physical properties are more easily obtained. The amount used when a plurality of organic surface crosslinking agents are used is the total amount thereof.

The organic surface crosslinking agent is preferably added to the water-absorbent resin in a solution state (as a surface crosslinking agent solution), more preferably added to the water-absorbent resin before surface crosslinking as an aqueous solution. In this case, the amount of water used is preferably 0.1 parts by mass or more, more preferably 0.3 parts by mass or more, still more preferably 0.5 parts by mass or more, and preferably 20 parts by mass or less, more preferably 15 parts by mass or less, still more preferably 10 parts by mass or less, relative to 100 parts by mass of the water-absorbent resin before surface crosslinking. A preferred range of the amount of water used can be a range defined by any combinations selected from the upper and lower limit values. By setting the amount of water used within the above range, the handleability of the surface crosslinking agent solution is further improved, and the surface crosslinking agent is easily mixed uniformly with the water-absorbent resin before surface crosslinking.

The concentration of the organic surface crosslinking agent in the surface crosslinking agent solution is preferably 0.1 mass% or more, more preferably 10 mass% or more, still more preferably 15 mass% or more, and preferably 60 mass% or less, more preferably 50 mass% or less, still more preferably 45 mass% or less, still more preferably 35 mass% or less. A preferred range of the concentration of the organic surface crosslinking agent can be a range defined by any combinations selected from the upper and lower limit values. The concentration of the organic surface crosslinking agent in the surface crosslinking agent solution is, for example, preferably 0.1 mass% or more and 60 mass% or less, more preferably 10 mass% or more and 50 mass% or less, still more preferably 15 mass% or more and 45 mass% or less, yet still more preferably 15 mass% or more and 35 mass% or less. By setting the concentration of the surface crosslinking agent within the above-described ranges, an optimum crosslinked structure can be formed on the surface layer of the water-absorbent resin before surface crosslinking, the water-absorbent resin having a high specific surface area, and physical properties such as water absorption performance can be improved. The amount used when a plurality of organic surface crosslinking agents are used is the total amount thereof.

The surface crosslinking agent solution can also be obtained by using a hydrophilic organic solvent in combination with the water as necessary. In this case, the amount of the hydrophilic organic solvent used is preferably 5 parts by mass or less (lower limit 0 parts by mass), more preferably 3 parts by mass or less, still more preferably 1 part by mass or less, relative to 100 parts by mass of the water-absorbent resin before surface crosslinking. Specific examples of the hydrophilic organic solvent include lower alcohols such as methyl alcohol; ketones such as acetone; ethers such as dioxane; amides such as N,N-dimethylformamide; sulfoxides such as dimethyl sulfoxide; and polyhydric alcohols such as ethylene glycol. These hydrophilic organic solvents contribute as a mixing aid for uniformly dispersing the surface

crosslinking agent on the surface of the water-absorbent resin, but this leads to an increase in cost from a commercial viewpoint, and thus, it is preferred that the amount of the hydrophilic organic solvent used is limited to as small as possible when used.

In addition, various additives to be added in the following "[2-8] Additive and Step of Adding Additive" may be added to the surface crosslinking agent solution within a range of 5 parts by mass or less relative to 100 parts by mass of the water-absorbent resin before surface crosslinking, or may be separately added in the mixing step.

(Combination of Peroxide and Organic Surface Crosslinking Agent)

In the present invention, a peroxide is mixed in a step after the polymerization step. The mixed peroxide performs a surface crosslinking reaction of the water-absorbent resin together with the organic surface crosslinking agent. The type of peroxide is not particularly limited, but hydrogen peroxide; persulfates such as sodium persulfate, potassium persulfate, and ammonium persulfate; permanganate; inorganic peroxides such as perchlorate, and organic peroxides such as cumene hydroperoxide, t-butyl hydroperoxide, di-t-butyl peroxide, benzoyl peroxide, and lauroyl peroxide can be suitably used. One of these may be used, or two or more of these may be used in combination. Among these, inorganic peroxides are preferred, and persulfates are particularly preferred from the viewpoint of high reactivity and being excellent in safety. For example, ammonium persulfate is used as a food additive in Japan.

The amount of the peroxide used (the abundance of the peroxide in the surface crosslinking step, preferably the amount of the peroxide added to the water-absorbent resin before surface crosslinking in the surface crosslinking step) is 0.001 parts by mass or more, preferably 0.01 parts by mass or more, more preferably 0.02 parts by mass or more, particularly preferably 0.05 parts by mass or more, and preferably 3 parts by mass or less, preferably 2 parts by mass or less, more preferably 1 part by mass or less, particularly preferably 0.5 parts by mass or less, relative to 100 parts by mass of the water-absorbent resin before surface crosslinking. A preferred range of the amount of the peroxide used can be a range defined by any combinations selected from the upper and lower limit values. The amount of the peroxide used is, for example, 0.001 parts by mass or more and 3 parts by mass or less, preferably 0.01 parts by mass or more and 2 parts by mass or less, more preferably 0.02 parts by mass or more and 1 part by mass or less, particularly preferably 0.05 parts by mass or more and 0.5 parts by mass or less, relative to 100 parts by mass of the water-absorbent resin before surface crosslinking. When the amount of the peroxide used is 0.001 parts by mass or more, desired effects (improvement in liquid permeability and affinity control of the surface of the water-absorbent resin with a liquid to be absorbed) are easily obtained, and when the amount of the peroxide used is 3 parts by mass or less, deterioration of the water-absorbent resin and deterioration of physical properties such as an increase in the water-soluble content can be suppressed.

As the mass ratio between the organic surface crosslinking agent and the peroxide to be used in the surface crosslinking step, the organic surface crosslinking agent: peroxide is preferably from 1 : 0.005 to 1 : 1, more preferably from 1 : 0.01 to 1 : 0.8, still more preferably from 1 : 0.03 to 1 : 0.5, yet still more preferably from 1 : 0.05 to 1 : 0.5, particularly preferably from 1 : 0.05 to 1 : 0.4. When the amount of the peroxide used relative to the organic surface crosslinking agent is within the ranges described above, deterioration of the water-absorbing agent composition and deterioration of physical properties such as an increase in water-soluble content can be suppressed. In addition, the possibility that the water-absorbing agent composition is colored brown or yellow is suppressed, and the water-absorbing agent composition is also suitable for a hygienic material having a high content of the water-absorbing agent composition.

Water may be used for mixing the peroxide and the water-absorbent resin. The amount of water used is preferably 0.5 parts by mass or more, more preferably 1.0 parts by mass or more, and preferably 10 parts by mass or less, more preferably 8 parts by mass or less, relative to 100 parts by mass of the water-absorbent resin. A preferred range of the amount of water used can be a range defined by any combinations selected from the upper and lower limit values. The amount of water used is, for example, preferably 0.5 parts by mass or more and 10 parts by mass or less, more preferably 1.0 parts by mass or more and 8 parts by mass or less, relative to 100 parts by mass of the water-absorbent resin. When the amount used is equal to or more than the lower limit, uniform mixing with the entire water-absorbent resin is facilitated, and when the amount used is equal to or less than the upper limit, deterioration in mixing properties such as formation of lumps can be suppressed. The peroxide may coexist in the surface crosslinking agent solution.

The time for adding the peroxide is not particularly limited, and the peroxide may be added in any step after the polymerization step, as long as a state in which the peroxide and the organic surface crosslinking agent coexist in the surface crosslinking step can be maintained. The phrase "after the polymerization step" includes the polymerization step. As described in the description of the polymerization step, some peroxides are used as a polymerization initiator, such as sodium persulfate or potassium persulfate. The peroxide may also function as a surface crosslinking agent.

Thus, when the peroxide added in the polymerization step and remaining in the reaction system without being used in the polymerization reaction is present in the vicinity of the surface of the water-absorbent resin, the peroxide undergoes a crosslinking reaction together with the organic surface crosslinking agent in the surface crosslinking step. In this manner, the peroxide may be added only in the polymerization step. When the peroxide is added only in the polymerization step, it is preferred that a suitable amount of the peroxide used remains in the surface crosslinking step.

In addition, the peroxide may be added at any time after the polymerization step, for example, before the start of the drying step after the completion of the polymerization step, during the drying step, after the completion of the drying step, during and before and after the pulverizing step, during and before and after the classification step, during and before and after the granulation step, as well as transportation step, intermediate storage step (hopper or the like), surface crosslinking step, and the like. In a preferred aspect, the peroxide is added in the surface crosslinking step. When the peroxide is added in a step other than the polymerization step, it is preferred that a suitable amount of the peroxide used remains in the surface crosslinking step. When the peroxide and the organic surface crosslinking agent can coexist in the vicinity of the surface of the water-absorbent resin in the surface crosslinking step, the peroxide and the organic surface crosslinking agent cause a crosslinking reaction on the particle surface, and thus, the gel strength improves. Thus, the liquid diffusibility and liquid permeability of the particulate water-absorbing agent composition can be improved, and the affinity of the surface of the water-absorbent resin with the liquid to be absorbed can be improved.

In the surface crosslinking step, when water is further present, mixing of the peroxide and the water-absorbent resin and mixing of the organic surface crosslinking agent and the water-absorbent resin are easily performed. As a result, the surface crosslinking reaction caused by the combined use of the peroxide and the organic surface crosslinking agent is reliably performed, which is preferred. The amount of water used is as described above.

In this manner, when the peroxide and the organic surface crosslinking agent can coexist in the surface crosslinking step, the effect of the present invention can be obtained, but in the production method according to the present invention, it is particularly preferred to add an aqueous solution containing the peroxide and the organic surface crosslinking agent to the water-absorbent resin before surface crosslinking (before the heat treatment step) in the surface crosslinking step.

In this case, the aqueous solution containing the peroxide and the organic surface crosslinking agent is added to the water-absorbent resin before surface crosslinking, and thus, the peroxide and the organic surface crosslinking agent simultaneously act as a surface crosslinking agent. The "surface crosslinking agent" means a substance capable of performing surface crosslinking. As described above, when the peroxide and the organic surface crosslinking agent coexist in the surface crosslinking step, the affinity of the surface of the water-absorbent resin with the liquid to be absorbed can be improved. Thus, the timing of adding the peroxide and the timing of adding the organic surface crosslinking agent are not necessarily the same. However, a particularly high effect can be obtained by adding an aqueous solution containing the peroxide and the organic surface crosslinking agent to the water-absorbent resin in the surface crosslinking step.

Usually, in the surface crosslinking step, the water-absorbent resin and the surface crosslinking agent are mixed, and then heat treatment is performed. However, when one of the peroxide and the organic surface crosslinking agent is added first, the component added first before the other is added permeates the inside of the water-absorbent resin, and there is a possibility that the proportion of crosslinking in the vicinity of the surface of the resulting water-absorbent resin decreases.

On the other hand, when an aqueous solution containing the peroxide and the organic surface crosslinking agent is added, a crosslinking reaction between both components occurs in the vicinity of the surface. Thus, a surface crosslinking layer stronger than before is formed, and the gel strength is considered to be improved.

In one embodiment, the production method according to the present invention is performed by adding an aqueous solution of a peroxide after the polymerization step, before performing the surface crosslinking step, preferably after the drying step, and adding an aqueous solution of an organic surface crosslinking agent in the surface crosslinking step. In this case, the crosslinking caused by the peroxide also occurs inside the particles, and thus, the absorption capacity under pressure (AAP) is slightly reduced as compared with the method of adding the aqueous solution containing the peroxide and the organic surface crosslinking agent to the water-absorbent resin in the surface crosslinking step.

From this point as well, it can be said that it is more effective that the crosslinking with the peroxide is performed in the vicinity of the surface of the water-absorbent resin. The phrase "after the polymerization step" means that the polymerization step is included, and the phrase "before performing the surface crosslinking step" means that the surface crosslinking step is not included.

The water content of the mixture obtained by adding an aqueous solution of a peroxide after the drying step is preferably 0.5 mass% or more and less than 10 mass%. When the water content is 0.5 mass% or more, uniform mixing is easily performed. When the water content is less than 10 mass%, formation of lumps is suppressed, and mixing properties and handleability are improved.

Here, a heating step may be included after the drying step and after the addition of an aqueous solution of a peroxide before the surface crosslinking step. The heating temperature is not particularly limited, but is preferably 40°C or higher, more preferably 50°C or higher, and preferably 200°C or lower, more preferably 150°C or lower. A preferred range of the heating temperature can be a range defined by any combinations selected from the upper and lower limit values. According to the configuration described above, undesirable aggregates in the mixture obtained by adding the aqueous solution of a peroxide described above can be easily crushed, and handleability can be improved.

The surface crosslinking reaction is preferably performed by mixing the water-absorbent resin before surface crosslinking with the organic surface crosslinking agent, the peroxide, and water as described above, and then performing

heat treatment. The heat treatment temperature may be preferably 130°C or higher, more preferably 150°C or higher, still more preferably 180°C or higher, and preferably 300°C or lower, more preferably 250°C or lower, still more preferably 230°C or lower depending on the surface crosslinking agent to be used. A preferred range of the heat treatment temperature can be a range defined by any combinations selected from the upper and lower limit values. The heat treatment temperature is preferably 130°C or higher and 300°C or lower, more preferably 150°C or higher and 250°C or lower, and still more preferably 180°C or higher and 230°C or lower. When the heat treatment temperature is lower than 130°C, absorption characteristics such as the absorption capacity and liquid permeability under pressure are not sufficiently improved in some cases. When the heat treatment temperature is higher than 300°C, the water-absorbent resin may deteriorate, and various types of performance may deteriorate, and thus, caution is required.

The heat treatment temperature is defined by the heat medium temperature, but when the heat medium temperature cannot be defined in the case of microwaves or the like, the heat treatment temperature is defined by the material temperature. The heat treatment time is preferably 1 minute or more, more preferably 5 minutes or more, and preferably 2 hours or less, more preferably 1 hour or less. A preferred range of the heat treatment time can be a range defined by any combinations selected from the upper and lower limit values.

In addition, to more uniformly mix the water-absorbent resin and the surface crosslinking agent, a non-crosslinkable water-soluble inorganic base (preferably, an alkali metal salt, an ammonium salt, an alkali metal hydroxide, and ammonia or a hydroxide thereof) other than the peroxide, a non-reducing alkali metal salt pH buffer (preferably, hydrogen carbonate, dihydrogen phosphate, hydrogen phosphate, and the like), a surfactant, and inorganic fine particles (fumed silica, colloidal silica, colloidal alumina, and the like) may coexist when mixing the water-absorbent resin and the surface crosslinking agent. The amount of these used depends on the type, particle diameter, and the like of the water-absorbent resin, but is preferably 0.005 parts by mass or more, more preferably 0.05 parts by mass or more, and is preferably 10 parts by mass or less, more preferably 5 parts by mass or less, relative to 100 parts by mass of the water-absorbent resin. A preferred range of the amount used can be a range defined by any combinations selected from the upper and lower limit values.

The mixing method for mixing the water-absorbent resin and the surface crosslinking agent is not particularly limited, and examples thereof include a method in which the water-absorbent resin is immersed in a hydrophilic organic solvent, and as necessary, the surface crosslinking agent dissolved in water and/or the hydrophilic organic solvent is mixed, and a method in which the surface crosslinking agent dissolved in water and/or the hydrophilic organic solvent is directly sprayed or dropped onto the water-absorbent resin and mixed.

Mixing Method and Mixing Conditions

As a method for mixing the water-absorbent resin before surface crosslinking with the surface crosslinking agent solution (optionally a surface crosslinking agent solution containing peroxide), a surface crosslinking agent solution is produced in advance, and the solution is preferably added to and mixed with the water-absorbent resin before surface crosslinking by spraying, dropping, or straight rod flow. More preferably, a method of mixing by spraying is exemplified.

In the addition by straight rod flow, it is preferred to design the inner diameter of the addition nozzle so that the flow rate of the surface crosslinking agent solution in the nozzle is preferably in the range of 1 m/s or more and 20 m/s or less, more preferably 1 m/s or more and 10 m/s or less, still more preferably 1 m/s or more and 5 m/s or less. When the flow rate in the nozzle is 1 m/s or more, the shape is less likely to be in the form of droplets, and intermittent addition can be suppressed, which is preferred from the viewpoint of mixing properties. On the other hand, when the flow rate in the nozzle is 20 m/s or less, it is possible to prevent a part of the surface crosslinking agent solution from being emitted in a liquid state without being mixed with the water-absorbent resin because of scattering of the surface crosslinking agent solution charged into the mixing apparatus, or clogging of the mixing apparatus because of an increase of adhesion in the mixing apparatus of a part of the surface crosslinking agent solution scattered in the mixing apparatus opposite to the traveling direction of the water-absorbent resin in the mixing apparatus.

To more uniformly mix the water-absorbent resin before surface crosslinking and the surface crosslinking agent solution, it is preferred to add the surface crosslinking agent solution from a plurality of places of the mixing apparatus. When addition is performed from a plurality of places, the number of addition nozzles is preferably 2 or more, more preferably 5 or more, still more preferably 10 or more, particularly preferably 20 or more. The upper limit of the number of addition nozzles is 100 or less. As the number of the addition nozzles increases, the mixing of the water-absorbent resin and the surface crosslinking agent solution becomes more uniform, but on the other hand, the probability that a defect such as clogging of the addition nozzle occurs during liquid feeding increases, and it may be difficult to specify the addition nozzle in which the defect has occurred.

As the mixing apparatus for the mixing, a mixing apparatus having a torque necessary for uniformly and reliably mixing the water-absorbent resin before surface crosslinking and the surface crosslinking agent solution is preferred. The mixing apparatus is preferably a high-speed stirring type mixer, and more preferably a high-speed stirring type continuous mixer. The rotational speed of the high-speed stirring type mixer is preferably 100 rpm or more, more preferably 300 rpm or

more, and preferably 10000 rpm or less, more preferably 2000 rpm or less. A preferred range of the rotational speed can be a range defined by any combinations selected from the upper and lower limit values.

The temperature of the water-absorbent resin before surface crosslinking to be supplied in this step is preferably 35°C or higher, and preferably 80°C or lower, more preferably 70°C or lower, still more preferably 60°C or lower, from the viewpoint of the mixing property with the surface crosslinking agent solution and the cohesiveness of the humidified mixture. The mixing time is preferably 1 second or more, more preferably 5 seconds or more, and preferably 1 hour or less, more preferably 10 minutes or less. A preferred range of the temperature and mixing time of the water-absorbent resin before surface crosslinking can be a range defined by any combinations selected from the upper and lower limit values.

[2-6-2] Heat Treatment Step

Heat Treatment Apparatus

This step is a step of applying heat to the humidified mixture obtained in the mixing step to cause a crosslinking reaction on the surface of the water-absorbent resin before surface crosslinking. The heat treatment is performed simultaneously with or after the addition of the organic surface crosslinking agent to the water-absorbent resin. Preferably, the heat treatment is performed after the addition of the organic surface crosslinking agent to the water-absorbent resin, more preferably after the addition of the organic surface crosslinking agent and the peroxide to the water-absorbent resin. In the heat treatment on the humidified mixture, the humidified mixture may be heated in a stationary state or may be heated in a flowing state using power such as stirring, but it is preferred to heat the humidified mixture under stirring in that the entire humidified mixture can be uniformly heated. Specific examples of the heat treatment apparatus that performs the heat treatment include a paddle dryer, a multi-fin processor, and a towered dryer.

In the case of using the heat treatment apparatus, in particular, a continuous conduction heat transfer type heat treatment apparatus, the heat transfer area (heating area) is not particularly limited as long as the powder temperature of the mixture can be controlled to be in the following temperature range, but is preferably 5 $m^2$/(t/hr) or more, more preferably 10 $m^2$/(t/hr) or more, and preferably 100 $m^2$/(t/hr) or less, more preferably 50 $m^2$/(t/hr) or less, relative to the supply amount of the mixture to be treated. A preferred range of the heat transfer area can be a range defined by any combinations selected from the upper and lower limit values. By setting the heat transfer area within the ranges described above, the powder temperature of the mixture and the inner wall surface temperature of the heat treatment apparatus can be easily controlled within the following temperature ranges, and stable operation can be performed, which is preferred.

As the heat treatment apparatus used in the present invention, it is preferred to use an apparatus including a gas supply mechanism and/or a gas emission mechanism (hereinafter, it may be referred to as "gas supply/emission mechanism") in a dryer or a heating furnace. The gas supply/emission mechanism controls the atmospheric water vapor density in the heat treatment apparatus. At this time, it is preferred not only to simply install the intake port/exhaust port but also to adjust the amount and pressure of the gas flowing through the heat treatment apparatus using a blower or the like. Each of the intake port and the exhaust port is not limited to one location, and a plurality of locations can be provided in consideration of the size of the heat treatment apparatus to be used and the atmospheric water vapor density.

A heating method, a stirring method, a gas supply method, and a gas emission method in the heat treatment apparatus can be used as a plurality of heat treatment apparatuses in which the same or different types are combined.

Water Vapor Density Inside Heat Treatment Apparatus

The lower limit value of the water vapor density inside the heat treatment apparatus used in the present heat treatment step is more than 0.005 g/L, preferably more than 0.05 g/L, more preferably 0.07 g/L or more, still more preferably 0.10 g/L or more, yet still more preferably 0.15 g/L or more, particularly preferably 0.20 g/L or more. When the water vapor density is 0.005 g/L or less, the amount of water evaporated from the water-absorbent resin is large, and the evaporation rate is high. Thus, a water-absorbent resin having excellent affinity with a desired liquid to be absorbed cannot be obtained. The upper limit value of the water vapor density inside the heat treatment apparatus used in the present heat treatment step is preferably 0.60 g/L or less, more preferably 0.55 g/L or less, still more preferably 0.50 g/L or less. A preferred range of the water vapor density can be a range defined by any combinations selected from the upper and lower limit values. The water vapor density inside the heat treatment apparatus used in the present heat treatment step is preferably more than 0.005 g/L and 0.60 g/L or less, more preferably more than 0.05 g/L and 0.60 g/L or less, still more preferably 0.07 g/L or more and 0.60 g/L or less, yet still more preferably 0.10 g/L or more and 0.60 g/L or less, yet still more preferably 0.15 g/L or more and 0.55 g/L or less, particularly preferably 0.20 g/L or more and 0.50 g/L or less. When the water vapor density is equal to or less than the upper limit, the water vapor density can be easily controlled without imposing an excessive burden on the production facility. Details such as measurement conditions of the water vapor density are described in Examples.

In the present invention, the water vapor density in this step means the average water vapor density of the gas

present in the upper space of the mixture in the heat treatment apparatus, and is preferably measured vertically above the mixture heated in the heating unit in the heat treatment apparatus.

The water vapor density in the present invention is a value obtained by dividing the weight of water contained in the gas by the amount of a non-condensable gas, and has a dimension of "weight/volume". Specifically, a condensable component (water) is collected from the sampled gas by cooling, solvent absorption, or the like and measured, and the amount of the remaining non-condensable gas is measured using a gas meter or the like. The water vapor density is determined by dividing the weight of the collected water by the volume of the amount of the non-condensable gas converted to the standard state of 0°C and 1 atm. In the present invention, "non-condensable gas" refers to a gas in a standard state of 0°C and 1 atm. As the non-condensable gas to be preferably used, air or nitrogen, or a mixed gas of air and nitrogen is suitable.

Operation Conditions

To achieve the effect of the present invention, the present invention is characterized in that the water vapor density inside the heat treatment apparatus used in the heat treatment step is controlled to more than 0.005 g/L. Hereinafter, operation conditions in the heat treatment step will be described in detail.

In the present invention, to control the water vapor density in the heat treatment apparatus to a desired range, a predetermined gas is introduced from an intake port provided in the heat treatment apparatus, or a gas present in an upper space of a mixture in the heat treatment apparatus is emitted from an exhaust port. The flow rate of the air flow of the intake and exhaust air is not particularly limited, but is preferably at least more than 0.1 ($Nm^3$/hr) and 10000 ($Nm^3$/hr) or less, more preferably 5000 ($Nm^3$/hr) or less, still more preferably 3000 ($Nm^3$/hr) or less. The proportion to the amount of the mixture to be treated is preferably 3000 ($Nm^3$/t) or less, more preferably 1000 ($Nm^3$/t) or less. "$Nm^3$" means a volume of a gas converted into a standard state (0°C, 1 atm).

The gas introduced into the heat treatment apparatus is not particularly limited as long as the water vapor density can be controlled within the range described above, and examples thereof include a method using steam, dry air, nitrogen, helium, argon, and dry air. In addition, to control the water vapor density, a method of utilizing water vapor generated from water contained in the water-absorbent resin by heating in this step is also exemplified.

The pressure in the heat treatment apparatus is preferably a slight reduced pressure. Specifically, the differential pressure (gauge pressure) relative to the atmospheric pressure is preferably -10 kPa or more and 0 kPa or less, more preferably -5 kPa or more and 0 kPa or less, still more preferably -2 kPa or more and 0 kPa or less. To control the water vapor density in the heat treatment apparatus to a desired range, adjustment of the differential pressure (gauge pressure) is one method, and when the pressure in the heat treatment apparatus is within the range described above, the water vapor density can be easily controlled to more than 0.005 g/L.

In the present heat treatment step, it is preferred to control the powder temperature to be in a desired temperature range by subjecting the mixture to a heat treatment and controlling the heat medium temperature in the heat treatment apparatus, the atmospheric temperature and the atmosphere dew point inside the heat treatment apparatus, the water vapor density, the inner wall surface temperature, the heat transfer area, the retention time, and the like. The powder temperature is preferably 150°C or higher, more preferably 160°C or higher, still more preferably 170°C or higher, and preferably 250°C or lower, more preferably 240°C or lower, still more preferably 230°C or lower. A preferred range of the powder temperature can be a range defined by any combinations selected from the upper and lower limit values. When the powder temperature is lower than 150°C, the covalent bond for forming the surface crosslinking layer may be insufficient. On the other hand, when the powder temperature exceeds 250°C, the water-absorbent resin is thermally deteriorated, and a desired water-absorbent resin cannot be obtained, which is not preferred.

The powder temperature of the mixture means the maximum temperature in the reaction step, but in the continuous method, the powder temperature is evaluated with the temperature of the water-absorbent resin immediately after being emitted from the heat treatment apparatus (the reaction product after the mixture of the water-absorbent resin and the surface crosslinking agent is subjected to the heat treatment).

The residence time (heat treatment time) in the heat treatment apparatus is not particularly limited, but it is preferably 5 minutes or more and 90 minutes or less, more preferably 10 minutes or more and 60 minutes or less.

The water vapor density may be controlled by appropriately controlling the supply amount of gas, the amount of gas emission, the temperature, and the like in consideration of the heat transfer from the inner wall surface of the heat treatment apparatus and the water-absorbent resin, the amount of water vapor generated from the water-absorbent resin, and the like. Specifically, a method of installing a measuring instrument in the heat treatment apparatus and introducing the gas as necessary to adjust the temperature, a method of adjusting the temperature by changing the emission amount or pressure of the gas, or the like may be used. In the present invention, a plurality of control methods may be combined as appropriate.

The water vapor density varies with the position of the heated portion and the lapse of the treatment time, but is desirably controlled to be within a certain range in the heat treatment apparatus. The phrase "within a certain range" means

that the water vapor density is within the range described above in preferably 50% or more, more preferably 70% or more, still more preferably 80% or more of the total time of the heat treatment, and the variation is preferably within 0.017 g/L, more preferably within 0.009 g/L, still more preferably within 0.007 g/L, particularly preferably within 0.006 g/L.

When the flow rate of the airflow is within the range described above, the water vapor density at an appropriate measurement point in the gas emission mechanism of the heat treatment apparatus may be set as the water vapor density inside the heat treatment apparatus defined in the present invention.

(Atmospheric Dew Point and Atmospheric Temperature in Heat Treatment Apparatus)

In the present invention, the atmospheric dew point and the atmospheric temperature in this step mean the average dew point and the average temperature of the gas present in the upper space of the mixture in the heat treatment apparatus, and is preferably measured vertically above the mixture heated in the heating unit in the heat treatment apparatus.

The lower limit value of the atmospheric dew point inside the heat treatment apparatus used in the present heat treatment step is preferably controlled to be more than 0°C, 10°C or more, 20°C or more, 30°C or more, 40°C or more, and 45°C or more, and the upper limit value is preferably controlled to be 100°C or less. When the atmospheric dew point is too low, the amount of water evaporated from the water-absorbent resin is large, and the evaporation rate is high. Thus, there is a possibility that a water-absorbent resin having excellent affinity with a desired liquid to be absorbed cannot be obtained.

The atmospheric temperature inside the heat treatment apparatus used in the present heat treatment step is controlled to preferably 100°C or more and 300°C or less, more preferably 100°C or more and 250°C or less, still more preferably 100°C or more and 230°C or less. When the atmospheric temperature is lower than 100°C, moisture evaporated from the water-absorbent resin condenses in the heating apparatus, and further, the water-absorbent resin adheres to the apparatus. Thus, stable continuous production cannot be performed, and there is a possibility that productivity and physical properties deteriorate.

[2-7] Cooling Step

This step is an optional step provided as necessary after the heat treatment step in the surface crosslinking step. This step is a step of forcibly cooling the water-absorbent resin after surface crosslinking after the heat treatment step to a predetermined temperature to quickly terminate the surface crosslinking reaction.

The water-absorbent resin after surface crosslinking may be cooled in a stationary state or may be cooled in a flowing state using power such as stirring, but it is preferred to cool the water-absorbent resin under stirring from the viewpoint of uniformly cooling the entire water-absorbent resin. From the above viewpoint, examples of the cooling apparatus that performs the cooling include a paddle dryer, a multi-fin processor, and a towered dryer. These cooling apparatuses may have the same specifications as those of the heat treatment apparatus used in the heat treatment step. This is because the heat treatment apparatus can be used as a cooling apparatus by changing the heat medium of the heat treatment apparatus to a refrigerant.

The cooling temperature in this step may be appropriately set according to the heating temperature in the heat treatment step, the water absorption performance of the water-absorbing agent composition or the water-absorbent resin after surface crosslinking, and the like. Specifically, the temperature of the water-absorbent resin after surface crosslinking is preferably 150°C or lower, more preferably 100°C or lower, still more preferably 90°C or lower, particularly preferably 80°C or lower, and preferably 20°C or higher, and more preferably 30°C or higher. A preferred range of the temperature of the water-absorbent resin after surface crosslinking can be a range defined by any combinations selected from the upper and lower limit values.

The shape of the water-absorbent resin after surface crosslinking may be any of a spherical shape, a granulated product, an aggregate, an irregularly crushed shape, and the like, but in consideration of the water absorption speed of the water-absorbent resin, an irregularly crushed shape is preferred. When the water-absorbent resin is crushed or the like after surface crosslinking, the surface crosslinking effect is reduced. Thus, the shape of the water-absorbent resin before and after surface crosslinking is preferably an irregularly crushed shape. The water-absorbent resin in an irregularly crushed shape can be obtained by pulverizing a hydrogel or a dried polymer. In one embodiment of the present invention, the average circularity of the particulate water-absorbing agent composition is preferably 0.83 or less, more preferably 0.80 or less, still more preferably 0.75 or less. In the case of an aggregate, the average circularity of the primary particles is in the ranges described above.

Suitable ranges of (i) mass proportion of particles of less than 150 $\mu$m, (ii) D50 (mass-average particle diameter), (iii) D50 (mass-average particle diameter) and particles of less than 150 $\mu$m, (iv) $\sigma\zeta$ (logarithmic standard deviation of particle size distribution), and combinations thereof in the water-absorbent resin after surface crosslinking are as described above.

[2-8] Additive and Step of Adding Additive

In the present invention, an additive may be added to any one or more of the water-absorbent resin before surface crosslinking, the water-absorbent resin during surface crosslinking, and the water-absorbent resin after surface crosslinking. In other words, the water-absorbing agent composition may contain an additive in addition to the water-absorbent resin. Examples of the additive include a liquid permeability improver or a similar component agent, and other additives. One of these may be used, or two or more thereof may be used in combination.

[2-8-1] Additive

Liquid Permeability Improver or Agent Having Similar Component

Examples of the liquid permeability improver used in the present invention include an additive having a function of improving the saline flow conductivity (hereinafter, referred to as "SFC") and the gel bed permeability (hereinafter, referred to as "GBP") under a load or no load of the water-absorbing agent composition or the water-absorbent resin. For example, at least one compound selected from a polyvalent metal salt, a cationic polymer, and inorganic fine particles can be used, and two or more compounds can be used in combination as necessary.

These additives may be used to exhibit other functions, such as functions of an anti-Caking agent under moisture absorption, a powder flow control agent, a deodorant, an aromatic agent, and a binder of a water-absorbent resin, without intending to improve liquid permeability. When an additive is added for the purpose of other functions, it is referred to as an agent having a similar component. The amount of the liquid permeability improver or the agent having a similar component is appropriately set according to the selected compound. In addition to the case of using one of these additives alone, the range of suitable amounts added in the case of using two or more additives in combination can be appropriately selected within the range described below.

"GBP" is an abbreviation for Gel Bed Permeability, is the liquid permeability of a 0.9 mass% aqueous sodium chloride solution through the water-absorbing agent composition or the water-absorbent resin under a load or under free swelling, and is a value measured in accordance with the GBP test method described in WO 2005/016393.

(Polyvalent Metal Salt)

When a polyvalent metal salt is used, the polyvalent metal cation of the polyvalent metal salt preferably has divalent or higher valence, more preferably has trivalent or higher valence, and preferably has tetravalent or lower valence. Examples of the polyvalent metal that can be used include aluminum and zirconium. Thus, examples of the polyvalent metal salt that can be used in this step include aluminum lactate, zirconium lactate, aluminum sulfate, and zirconium sulfate. Among these, from the viewpoint of the effect of improving SFC, aluminum lactate or aluminum sulfate is more preferred, and aluminum sulfate is still more preferred. The amount of the polyvalent metal salt added is preferably 0 mol or more and less than $3.6 \times 10^{-5}$ mol, more preferably 0 mol or more and less than $1.4 \times 10^{-5}$ mol, still more preferably 0 mol or more and less than $1.0 \times 10^{-5}$ mol, relative to 1 g of the water-absorbent resin.

Cationic Polymer

When a cationic polymer is used, examples of the cationic polymer include the substances described in US 7098284. In particular, a vinylamine polymer is more preferred from the viewpoint of the effect of improving SFC and GBP. The mass-average molecular weight of the cationic polymer is preferably 5000 or more and 1000000 or less.

The amount of the cationic polymer added is preferably 0 parts by mass or more, more preferably more than 0 parts by mass, and preferably less than 2.5 parts by mass, more preferably less than 2.0 parts by mass, still more preferably less than 1.0 parts by mass, relative to 100 parts by mass of the water-absorbent resin.

Inorganic Fine Particles

When an inorganic fine particle is used, examples of the inorganic fine particle include substances described in US 7638570. Among these, silicon dioxide (amorphous fumed silica, colloidal silica, and the like) is preferred from the viewpoint of the effect of improving SFC and GBP.

When the primary particle diameter is less than 20 nm, the inorganic fine particles may be added so as to be in an amount of preferably 0 parts by mass or more, more preferably more than 0 parts by mass, and preferably less than 1.2 parts by mass, more preferably less than 1.0 parts by mass, still more preferably less than 0.5 parts by mass, relative to 100 parts by mass of the water-absorbent resin. When the primary particle diameter is 20 nm or more, the inorganic fine particles may be added so as to be in an amount of preferably 0 parts by mass or more, more preferably more than 0 parts

by mass, and preferably less than 2.0 parts by mass, more preferably less than 1.5 parts by mass, still more preferably less than 1.0 parts by mass, relative to 100 parts by mass of the water-absorbent resin.

Other additives

Specific examples of the other additives include chelating agents, inorganic reducing agents, aromatic substances, organic reducing agents, hydroxycarboxylic acid compounds, surfactants, compounds having a phosphorus atom, oxidizing agents, organic powders such as metal soaps, deodorants, antibacterial agents, pulps, and thermoplastic fibers. One or more of these other additives can be used. Among these, a chelating agent is preferred, and an amino polycarboxylic acid or an amino polyphosphoric acid is more preferred. Specific examples of the chelating agent include chelating agents described in JP 11-060975 A, WO 2007/004529, WO 2011/126079, WO 2012/023433, JP 2009-509722 T, JP 2005-097519 A, JP 2011-074401 A, JP 2013-076073 A, JP 2013-213083 A, JP 59-105448 A, JP 60-158861 A, JP 11-241030 A, JP 2-41155 A, and the like.

Another additives, in particular, a chelating agent, is added or contained in an amount of preferably 0.001 mass% or more and 1 mass% or less relative to the monomer or the water-absorbent resin.

[2-8-2] Step of Adding Additive

The additive can be added before or after or in the middle of at least one step selected from the step of preparing an aqueous monomer solution, the polymerization step, the gel-grinding step, the drying step, the pulverizing step, the classification step, and the surface crosslinking step. Preferably, the additive is added before, after, or in the middle of any step after the polymerization step.

When the additive is added to the water-absorbent resin, and the additive is a liquid or a solution of an aqueous medium such as water, it is preferred to spray the liquid or solution onto the water-absorbent resin and apply sufficient torque to uniformly and reliably mix the water-absorbent resin and the additive. On the other hand, when the additive is in a solid form such as a powder form, the additive may be dry-blended with the water-absorbent resin, or an aqueous liquid such as water may be used as a binder.

Specific examples of the apparatus used for the mixing include a stirring type mixer, a cylindrical mixer, a double-wall conical mixer, a V-shaped mixer, a ribbon type mixer, a screw type mixer, a flow-type rotary disk-type mixer, a flow-type mixer, a double-arm kneader, an internal mixer, a pulverizing type kneader, a rotary mixer, and a screw type extruder. In the case of using a stirring type mixer, the rotational speed thereof is preferably 5 rpm or more, more preferably 10 rpm or more, and preferably 10000 rpm or less, more preferably 2000 rpm or less.

[2-9] Other Steps

In the present invention, in addition to the above-described steps, a granulation step, a particle size adjustment step, a fine powder removal step, a fine powder recovery step, a step of reusing fine powder, an iron removal step, and the like, can be performed as necessary. At least one step selected from among a transportation step, a storage step, a packing step, a warehousing step, and the like may be further included.

[3] Physical Property of Water-Absorbing Agent Composition

The water-absorbing agent composition obtained through the above steps is a final product as long as it is ready for shipment. The water-absorbing agent composition according to the present invention is a water-absorbing agent composition containing a water-absorbent resin as a main component and satisfies all of (1) to (2) described below:

(1) the water-absorbing agent composition has a specific surface area of 25 $m^2$/kg or more;
(2) the water-absorbing agent composition has a Washburn method contact angle θ2 of 72° or less as measured with a 20 mass% aqueous sodium chloride solution.

[3-1] Specific Surface Area

By setting the specific surface area of the water-absorbing agent composition according to the present invention to 25 $m^2$/kg or more, a further excellent water absorption speed and gap water retention force can be obtained. The specific surface area of the water-absorbing agent composition is preferably as high as possible, preferably 26 $m^2$/kg or more, 27 $m^2$/kg or more, 28 $m^2$/kg or more, 29 $m^2$/kg or more, and 30 $m^2$/kg or more in this order, and may be 36 $m^2$/kg or more. The specific surface area of the water-absorbing agent composition according to the present invention is preferably 60 $m^2$/kg or less and 55 $m^2$/kg or less in this order. A preferred range of the specific surface area of the water-absorbing agent

composition can be a range defined by any combinations selected from the upper and lower limit values. From the viewpoint of increasing the water absorption speed and increasing the gap water retention force, it is desirable that the specific surface area is as high as possible. However, when the specific surface area is too high, excessive foaming polymerization in the polymerization step or too fine gel-grinding in the gel-grinding step is required, and as a result, there is a possibility that AAP (absorption capacity under pressure) and SFC (saline flow conductivity) decrease. On the other hand, when the specific surface area of the water-absorbing agent composition is too small, it is difficult to obtain a water-absorbing agent composition having a desired water absorption speed and gap water retention force, which is not preferred. The specific surface area of the water-absorbing agent composition may be, for example, 25 $m^2$/kg or more and 60 $m^2$/kg or less, 26 $m^2$/kg or more and 60 $m^2$/kg or less, 27 $m^2$/kg or more and 60 $m^2$/kg or less, 28 $m^2$/kg or more and 60 $m^2$/kg or less, 29 $m^2$/kg or more and 60 $m^2$/kg or less, 30 $m^2$/kg or more and 55 $m^2$/kg or less, or 36 $m^2$/kg or more and 55 $m^2$/kg or less.

In the present description, the "specific surface area" means a surface area (unit: $m^2$/kg) per unit mass of the water-absorbing agent composition or the water-absorbent resin, and can be determined by analyzing three-dimensional image data of the water-absorbing agent composition or the water-absorbent resin acquired using a microfocus X-ray CT system (available from Shimadzu Corporation: inspeXio SMX-100CT) to be described later with high-speed three-dimensional analysis software (available from RATOC SYSTEM ENGINEERING Co., Ltd.: TRI/3D-VOL-FCS64).

[3-2] Washburn Method Contact Angles θ1 and θ2

The Washburn method contact angle θ1 (unit: °) of the water-absorbing agent composition according to the present invention is preferably 65° or less, 63° or less, 60° or less, 58° or less, 56° or less, 55° or less, 54° or less, 53° or less, 52° or less, and 51° or less in this order, more preferably 20° or more, 22° or more, 25° or more, 28° or more, and 30° or more in this order. A preferred range of the Washburn method contact angle θ1 can be a range defined by any combinations selected from the upper and lower limit values. The Washburn method contact angle θ1 (unit: °) of the water-absorbing agent composition according to the present invention may be, for example, 20° or more and 65° or less, 22° or more and 63° or less, 25° or more and 60° or less, 28° or more and 58° or less, 30° or more and 56° or less, 30° or more and 55° or less, 30° or more and 54° or less, 30° or more and 53° or less, 30° or more and 52° or less, or 30° or more and 51° or less.

The Washburn method contact angle θ2 (unit: °) of the water-absorbing agent composition according to the present invention is preferably 72° or less, 70° or less, 68° or less, 67° or less, 65° or less, 63° or less, 61° or less, or 60° or less in this order, more preferably 25° or more, 27° or more, 30° or more, 33° or more, 35° or more, 40° or more, 45° or more, or 50° or more in this order. A preferred range of the Washburn method contact angle θ2 can be a range defined by any combinations selected from the upper and lower limit values. When the Washburn method contact angle θ2 (unit: °) of the water-absorbing agent composition according to the present invention exceeds 72°, it is difficult to improve the water absorption properties (for example, AAP or gap water ratio under pressure) under pressure while maintaining the water absorption speed which is an advantage of a high specific surface area. The Washburn method contact angle θ2 (unit: °) of the water-absorbing agent composition according to the present invention is, for example, 25° or more and 72° or less, 27° or more and 70° or less, 30° or more and 68° or less, 33° or more and 67° or less, 35° or more and 65° or less, 40° or more and 63° or less, 45° or more and 61° or less, or 50° or more and 60° or less in this order.

By controlling the Washburn method contact angle θ2 to 72° or less, and preferably controlling the Washburn method contact angle θ1 to 65° or less, a water-absorbing agent composition excellent in affinity between the surface of the water-absorbent resin and the liquid to be absorbed and the gap water retention force can be obtained. In addition, the Washburn method contact angles θ1 and θ2 are preferably as small as possible from the viewpoint of excellent affinity between the surface of the water-absorbent resin and the liquid to be absorbed, but to achieve a Washburn method contact angle of less than 20° in the case of θ1 and less than 25° in the case of θ2, an excessive additional treatment or addition of an excessive hydrophilic surfactant is required, which is not preferred from the viewpoint of economic efficiency.

The Washburn method contact angle is calculated by measuring the time and the wet load when the liquid from the lower end of the tube filled with the powder wets between the powders and using a capillary constant calculated for a liquid (hexane, methanol, or the like) having a contact angle of 0°. The Washburn method contact angle can be determined by analyzing the permeation speed data obtained using a high-performance surface tensiometer (DyneMaster series DY 500, available from Kyowa Interface Science Co., Ltd.) described below with the attached analysis software DYNALYZER.

[3-3] Other Physical Properties of Water-Absorbing Agent Composition

The water-absorbing agent composition according to the present invention preferably has at least one of the following preferred ranges of physical properties (a) to (k).

(a) D50 (mass-average particle diameter), (b) mass proportion of particles having particle diameter of less than 150 μm, (c) σζ (logarithmic standard deviation of particle size distribution), (d) CRC (absorption capacity without pressure), (e) AAP (absorption capacity under pressure), (f) gap water ratio under pressure, (g) water content, (h) SFC (saline flow

conductivity), (i) Vortex (water absorption speed), (j) surface tension, (k) peroxide residual amount

The water-absorbing agent composition according to the present invention may have any two or more of the preferred ranges of the physical properties (a) to (k) in combination. Preferably, at least a combination of suitable ranges of (e) AAP (absorption capacity under pressure) and (g) water content may be provided, more preferably in addition to these, a combination with a suitable range of (f) gap water ratio under pressure may be provided, or preferably a combination of suitable ranges of (e) AAP (absorption capacity under pressure) and (f) gap water ratio under pressure may be provided, still more preferably in addition to these, a combination with a suitable range of (i) Vortex (water absorption speed) may be provided, yet still more preferably in addition to these, a combination with suitable ranges of (a) to (c) may be provided. Most preferably, all of the preferred ranges of (a) to (k) are provided. Specific preferred ranges of the physical properties (a) to (k) are the ranges described in the following items.

(a) D50 (mass-average particle diameter), (b) mass proportion of particles having particle diameter of less than 150 μm, (c) σζ (logarithmic standard deviation of particle size distribution)

D50 (mass-average particle diameter), the mass proportion of particles having a particle diameter of less than 150 μm, and σζ (logarithmic standard deviation of particle size distribution) of the water-absorbing agent composition according to the present invention may be set to be the same as the ranges in the water-absorbent resin after the above-described classification and before surface crosslinking.

By setting (a) D50 (mass-average particle diameter) of the water-absorbing agent composition within the ranges described above, AAP (absorption capacity under pressure) and SFC (saline flow conductivity), which are preferred absorption characteristics, can be further controlled in a well-balanced manner. When D50 (mass-average particle diameter) is too small, the gel bulk density may be too high, or AAP and SFC, which are preferred absorption characteristics, may be too low. On the other hand, when D50 (mass-average particle diameter) is too large, the roughness of the particles of the water-absorbing agent composition becomes conspicuous, and the texture and wearing feeling may deteriorate when used for an absorbent article such as a disposable diaper or a sanitary napkin.

By setting (b) mass proportion of particles having a particle diameter of less than 150 μm within the range described above, AAP (absorption capacity under pressure) and SFC (saline flow conductivity) can be controlled easily in a more balanced manner. When (b) mass proportion of the particles having a particle diameter of less than 150 μm is too large, not only AAP (absorption capacity under pressure), which is a preferred absorption characteristic, may become too low, but also the working environment may deteriorate due to scattering of dust at a place where the water-absorbing agent composition is handled, and handling properties may become difficult because of deposition of fine particles in the apparatus, which is not preferred.

Preferably, the water-absorbing agent composition has (a) D50 (mass-average particle diameter) of 250 μm or more and less than 550 μm, and (b) mass proportion of particles having a particle diameter of less than 150 μm is 3 mass% or less. More preferably, the water-absorbing agent composition satisfies D50 (mass-average particle diameter) in the range described above, and satisfies the mass proportion of particles having a particle diameter of less than 150 μm in the range described above. By satisfying both of them, the above-described effect is synergistically obtained. D50 (mass-average particle diameter) of the water-absorbing agent composition and the mass proportion of particles having a particle diameter of less than 150 μm are measured by the method described in Examples.

(d) CRC (Absorption Capacity Without Pressure)

CRC (absorption capacity without pressure) of the water-absorbing agent composition according to the present invention is preferably 25 g/g or more, preferably 40 g/g or less, more preferably 38 g/g or less, still more preferably 35 g/g or less, particularly preferably 33 g/g or less. When the CRC (absorption capacity without pressure) is too low, the absorption capacity of the water-absorbing agent composition decreases, and there is a possibility that the water-absorbing agent composition is not suitable for use as an absorbent body of an absorbent article such as a disposable diaper or a sanitary napkin. On the other hand, when the CRC (absorption capacity without pressure) is too high, the gel strength may be weakened.

(e) AAP (Absorption Capacity Under Pressure)

AAP (absorption capacity under pressure) of the water-absorbing agent composition according to the present invention is preferably 20.0 g/g or more, more preferably 23.0 g/g or more, still more preferably 25.5 g/g or more, more preferably 26.0 g/g or more, still more preferably 26.5 g/g or more, and preferably 32.0 g/g or less, more preferably 31.0 g/g or less, still more preferably 30.0 g/g or less, more preferably 29.0 g/g or less. A preferred range of AAP (absorption capacity under pressure) can be a range defined by any combinations selected from the upper and lower limit values. AAP (absorption capacity under pressure) of the water-absorbing agent composition according to the present invention is, for

example, preferably 20.0 g/g or more and 32.0 g/g or less, more preferably 23.0 g/g or more and 31.0 g/g or less, more preferably 25.5 g/g or more and 30.0 g/g or less, more preferably 26.0 g/g or more and 29.0 g/g or less, still more preferably 26.5 g/g or more and 29.0 g/g or less. By setting AAP (absorption capacity under pressure) within the ranges described above, the water-absorbing agent composition can absorb the liquid to be absorbed even when pressure is applied to the absorbent body, and thus, the water-absorbent resin or the water-absorbing agent composition suitable for use as an absorbent body of an absorbent articles such as a disposable diaper or a sanitary napkins is obtained.

(f) gap water ratio under pressure

The gap water ratio under pressure of the water-absorbing agent composition according to the present invention is preferably 9.0 g/g or more, 9.3 g/g or more, 9.5 g/g or more, 10.0 g/g or more, 11.0 g/g or more, or 12.0 g/g or more in this order. The upper limit value is not particularly limited, but is preferably 19.0 g/g or less, and it may be 18.0 g/g or less, or 17.0 g/g or less. A preferred range of the gap water ratio under pressure can be a range defined by any combinations selected from the upper and lower limit values. When the gap water ratio under pressure is less than 9.0 g/g, and the water-absorbing agent composition is used as an absorbent body of an absorbent article such as a disposable diaper, body fluids such as urine and blood are not sufficiently absorbed and liquid leakage may occur under pressure in some cases, and thus, the water-absorbing agent composition is not suitable as an absorbent body of an absorbent article such as a disposable diaper. The gap water ratio under pressure of the water-absorbing agent composition according to the present invention is, for example, 9.0 g/g or more and 19.0 g/g or less, 9.3 g/g or more and 19.0 g/g or less, 9.5 g/g or more and 19.0 g/g or less, 10.0 g/g or more and 19.0 g/g or less, 11.0 g/g or more and 18.0 g/g or less, or 12.0 g/g or more and 17.0 g/g or less.

(g) Water Content

The water content of the water-absorbing agent composition according to the present invention is preferably 5 mass% or less, and preferably 4 mass% or less, 3 mass% or less, or 2 mass% or less in this order. The content is preferably more than 0 mass%. When the water content is more than 5 mass%, the surface of the water-absorbing agent composition starts to be tacky, the fluidity as a powder decreases, and the handleability deteriorates, which is not preferred.

(h) SFC (Saline Flow Conductivity)

SFC (saline flow conductivity) of the water-absorbing agent composition according to the present invention is preferably $1 \times 10^{-7}$ $cm^3 \cdot sec/g$ or more, more than $3 \times 10^{-7}$ $cm^3 \cdot sec/g$, $5 \times 10^{-7}$ $cm^3 \cdot sec/g$ or more, $10 \times 10^{-7}$ $cm^3 \cdot sec/g$ or more, $20 \times 10^{-7}$ $cm^3 \cdot sec/g$ or more, $25 \times 10^{-7}$ $cm^3 \cdot sec/g$ or more, or $30 \times 10^{-7}$ $cm^3 \cdot sec/g$ or more in this order from the viewpoint of improving the gap water ratio under pressure. SFC (saline flow conductivity) of the water-absorbing agent composition according to the present invention is preferably $120 \times 10^{-7}$ $cm^3 \cdot sec/g$ or less, $110 \times 10^{-7}$ $cm^3 \cdot sec/g$ or less, $100 \times 10^{-7}$ $cm^3 \cdot sec/g$ or less, $90 \times 10^{-7}$ $cm^3 \cdot sec/g$ or less, $80 \times 10^{-7}$ $cm^3 \cdot sec/g$ or less, $70 \times 10^{-7}$ $cm^3 \cdot sec/g$ or less, $60 \times 10^{-7}$ $cm^3 \cdot sec/g$ or less, $50 \times 10^{-7}$ $cm^3 \cdot sec/g$ or less, or less than $40 \times 10^{-7}$ $cm^3 \cdot sec/g$ in this order. A preferred range of SFC (saline flow conductivity) can be a range defined by any combinations selected from the upper and lower limit values. SFC (saline flow conductivity) of the water-absorbing agent composition according to the present invention may be, for example, $1 \times 10^{-7}$ $cm^3 \cdot sec/g$ or more and $120 \times 10^{-7}$ $cm^3 \cdot sec/g$ or less, more than $3 \times 10^{-7}$ $cm^3 \cdot sec/g$ and $110 \times 10^{-7}$ $cm^3 \cdot sec/g$ or less, $5 \times 10^{-7}$ $cm^3 \cdot sec/g$ or more and $100 \times 10^{-7}$ $cm^3 \cdot sec/g$ or less, $10 \times 10^{-7}$ $cm^3 \cdot sec/g$ or more and $90 \times 10^{-7}$ $cm^3 \cdot sec/g$ or less, $20 \times 10^{-7}$ $cm^3 \cdot sec/g$ or more and $80 \times 10^{-7}$ $cm^3 \cdot sec/g$ or less, $25 \times 10^{-7}$ $cm^3 \cdot sec/g$ or more and $70 \times 10^{-7}$ $cm^3 \cdot sec/g$ or less, $25 \times 10^{-7}$ $cm^3 \cdot sec/g$ or more and $60 \times 10^{-7}$ $cm^3 \cdot sec/g$ or less, $25 \times 10^{-7}$ $cm^3 \cdot sec/g$ or more and $50 \times 10^{-7}$ $cm^3 \cdot sec/g$ or less, and $30 \times 10^{-7}$ $cm^3 \cdot sec/g$ or more and less than $40 \times 10^{-7}$ $cm^3 \cdot sec/g$. When SFC is equal to or more than the lower limit, gel blocking is suppressed, and deterioration of absorption characteristics of the hygienic material is suppressed even when the water-absorbing agent composition is used in a thin hygienic material at a high concentration, and when SFC is equal to or less than the upper limit, deterioration of CRC having a trade-off relationship is suppressed.

(i) Vortex (Water Absorption Speed)

The upper limit of Vortex (water absorption speed) of the water-absorbing agent composition according to the present invention is preferably 50 seconds or less, 48 seconds or less, 46 seconds or less, 44 seconds or less, 42 seconds or less, 40 seconds or less, 38 seconds or less, or 36 seconds or less in this order. The lower limit of Vortex (water absorption speed) of the water-absorbing agent composition according to the present invention is preferably more than 10 seconds, more preferably 15 seconds or more. A preferred range of the Vortex (water absorption speed) can be a range defined by any combinations selected from the upper and lower limit values. Vortex (water absorption speed) of the water-

absorbing agent composition according to the present invention is, for example, more than 10 seconds and 50 seconds or less, more than 10 seconds and 48 seconds or less, more than 10 seconds and 46 seconds or less, more than 10 seconds and 44 seconds or less, more than 10 seconds and 42 seconds or less, more than 10 seconds and 40 seconds or less, 15 seconds or more and 38 seconds or less, or 15 seconds or more and 36 seconds or less. When Vortex (water absorption speed) is low (for example, more than 50 seconds), the water absorption speed for body fluids such as urine and blood of the water-absorbing agent composition to be obtained is slow, and there is a risk that liquid leakage may occur. Thus, the water-absorbing agent composition is not suitable as an absorbent body of an absorbent article such as a disposable diaper. Vortex (water absorption speed) can be controlled by foaming polymerization, gel-grinding, the production method according to the present invention, or the like.

(j) Surface Tension

The surface tension of the water-absorbing agent composition according to the present invention is preferably 56 mN/m or more, more preferably 58 mN/m or more, still more preferably 60 mN/m or more, particularly preferably 65 mN/m or more. The upper limit value is not particularly limited, and is preferably 75 mN/m or less from the viewpoint of balance with other physical properties. When the surface tension is 56 mN/m or more, the return amount of the liquid when a pressure is applied to the absorbent body does not become excessively large, and thus, the water-absorbing agent composition is suitable as a water-absorbing agent composition to be used for an absorbent article such as a disposable diaper. A preferred range of the surface tension can be a range defined by any combinations selected from the upper and lower limit values.

(k) Peroxide Remaining Amount

In the water-absorbing agent composition according to the present invention, a peroxide decomposition product is preferably present on the surface, and the concentration on the surface of the water-absorbing agent composition is more preferably higher than the concentration inside the water-absorbing agent composition. According to the method for producing a water-absorbing agent composition of the present invention, the residual component of the peroxide (peroxide decomposition product) is more distributed on the particle surface as compared with the inside of the particles of the water-absorbing agent composition. More specifically, according to the production method as one aspect of the water-absorbing agent composition according to the present invention, the peroxide is allowed to coexist with the organic surface crosslinking agent in the surface crosslinking step, and thus, the concentration on the surface of the water-absorbing agent composition is higher than the concentration inside the water-absorbing agent composition in the peroxide decomposition product than when the peroxide is added as the polymerization initiator only at the time of polymerization. Such a configuration is preferred because the effect of the present invention is more likely to be obtained. The peroxide is decomposed by, for example, heat treatment at the time of surface crosslinking to become a decomposed product. When the peroxide is sodium persulfate, the peroxide decomposition product is sodium sulfate, and when the peroxide is potassium persulfate, the peroxide decomposition product is potassium sulfate.

The peroxide residual amount remaining on the particle surface of the water-absorbing agent composition according to the present invention after decomposition is preferably more than 0 mass%, 0.005 mass% or more, 0.010 mass% or more, 0.015 mass% or more, and 0.020 mass% or more in this order, and more preferably 1.0 mass% or less, 0.7 mass% or less, 0.35 mass% or less, and 0.1 mass% or less in this order. A preferred range of the peroxide residual amount can be a range defined by any combinations selected from the upper and lower limit values. The peroxide residual amount is, for example, more than 0 mass% and 1.0 mass% or less, 0.005 mass% or more and 0.7 mass% or less, 0.010 mass% or more and 0.35 mass% or less, 0.015 mass% or more and 0.1 mass% or less, or 0.020 mass% or more and 0.1 mass% or less. When the peroxide residual amount is 0 mass% or less, that is, when the peroxide residual amount remaining on the particle surface is equal to or less than the peroxide residual amount inside the particle, the peroxide does not effectively act on the particle surface of the water-absorbing agent composition, and thus, a desired effect (affinity control of the surface of the water-absorbent resin with the liquid to be absorbed) cannot be obtained. On the other hand, when the peroxide residual amount is more than 1.0 mass%, deterioration of the water-absorbing agent composition is caused, and deterioration of physical properties such as an increase in the water-soluble content is observed, which is not preferred.

The peroxide residual amount is determined by the following methods (A) to (E).

(A) performing a predetermined impact test on the water-absorbing agent composition according to the present invention;
(B) sieving the water-absorbing agent composition subjected to the impact test into a particle group a having a particle diameter of 300 μm or more and a particle group b having a particle diameter of less than 300 μm using a JIS standard sieve having a mesh size of 300 μm;
(C) defining the content of the peroxide decomposition product present in the particle group a as C1;

(D) defining the content of the peroxide decomposition product present in the particle group b as C2;
(E) calculating the peroxide residual amount from "C2 - C1".

As a result of the impact test, most of the scraped surface portion of the water-absorbing agent composition becomes the particle group b. Thus, it can be said that as C2 - C1 is larger, more peroxide is present on the surface of the water-absorbing agent composition.

The details of the impact test in (A) are described in Examples.

[3-4] Relationship Between Water-Absorbent Resin and Water-Absorbing Agent Composition

The amount of the water-absorbent resin contained in the water-absorbing agent composition according to the present invention is preferably 95 mass% or more, more preferably 98 mass% or more, still more preferably 99 mass% or more, relative to the total amount of the water-absorbing agent composition. When the above-described various additives are contained, the amount of the water-absorbent resin contained in the water-absorbing agent composition is not 100 mass%.

The shape of the water-absorbing agent composition according to the present invention may be any of a spherical shape, a granulated product, an aggregate, an irregularly crushed shape, and the like, but in consideration of the water absorption speed, it is preferred that the water-absorbing agent composition has an irregularly crushed shape.

[4] Application of Water-Absorbing Agent Composition

The water-absorbing agent composition according to the present invention is preferably used mainly as an absorbent body of an absorbent article such as a disposable diaper or a sanitary napkin, or as an absorbent layer (hereinafter, collectively referred to as an "absorbent body"), and more preferably used as an absorbent body of an absorbent article having a large amount used per absorbent article.

The absorbent body means an article obtained by molding the particulate water-absorbing agent composition into a sheet shape, a fibrous shape, a cylindrical shape, or the like, and is preferably molded into a sheet shape to form an absorbent layer. In addition to the water-absorbing agent composition according to the present invention, an absorbent material such as pulp fibers, or an adhesive, a nonwoven fabric, or the like can be molded and used in combination. In this case, the amount of the water-absorbing agent composition in the absorbent body (hereinafter, referred to as "core concentration") is preferably 50 mass% or more, more preferably 60 mass% or more, still more preferably 70 mass% or more, particularly preferably 80 mass% or more, and is preferably 100 mass% or less. By setting the core concentration within the above-described ranges, when the absorbent body is used for an absorbent article, an appropriate space can be formed between gel particles even though the water-absorbing agent composition is gelled by absorbing urine.

[5] Absorbent Article

The absorbent article according to the present invention contains the absorbent body and is usually provided with a liquid-permeable top sheet and a liquid-impermeable back sheet. Examples of the absorbent article include a disposable diaper and a sanitary napkin.

When the absorbent article is, for example, a disposable diaper, the disposable diaper is produced by sandwiching an absorbent body containing the water-absorbing agent composition of the present invention between a liquid-permeable topsheet positioned on the side that comes into contact with human skin when worn and a liquid-impermeable backsheet positioned outside when worn. The disposable diaper is further provided with a member known to those skilled in the art, such as an adhesive tape for fixing the disposable diaper while being worn.

In the absorbent article according to the present invention, when the absorbent body absorbs liquid and the water-absorbing agent composition swells and gelates, an appropriate space is formed between the gel particles. Thus, moisture is not retained, and the stuffy feeling in the disposable diaper is reduced. When the water-absorbing agent composition contains a fragrance or a deodorant, these components suitably volatilize through the space. Thus, it is possible to provide an absorbent article comfortable for the wearer and the caregiver by any one or both of the effects described above. In addition to disposable diapers and sanitary napkins, the water-absorbing agent composition according to the present invention can be suitably used for applications such as a pet urine absorbent and a urine gelling agent for portable toilets.

Examples

Hereinafter, the present invention will be described more specifically by way of Examples, but the present invention is not limited by the following Examples as a matter of course, and can be implemented with appropriate

modifications within a range that can be adapted to the gist described above and below, and all of them are included in the technical scope of the present invention. In addition, in the present invention, the measurement method of each physical property is based on the measurement method described in Examples unless otherwise specified.

The method for measuring each physical property is as follows. For example, when the object to be measured is a substance other than the water-absorbing agent composition, the "water-absorbing agent composition" in the following description is applied by replacing it with "particulate hydrogel", "water-absorbent resin before surface crosslinking", and "water-absorbent resin after surface crosslinking".

[D50 (mass-average particle diameter), mass proportion of particles having particle diameter of less than 150 μm, σζ (logarithmic standard deviation of particle size distribution)]

D50 (mass-average particle diameter), the mass proportion of particles having a particle diameter of less than 150 μm, and σζ (logarithmic standard deviation of particle size distribution) of the water-absorbing agent composition according to the present invention were measured in accordance with the measurement method described in US 7638570.

CRC (Absorption Capacity Without Pressure)

CRC (absorption capacity without pressure) of the water-absorbing agent composition according to the present invention was measured in accordance with the EDANA method (NWSP 241.0. R2 (19)). Specifically, 0.2 g of the water-absorbing agent composition was put in a bag made of a nonwoven fabric, and then immersed in a large excess of a 0.9 mass% aqueous sodium chloride solution for 30 minutes to freely swell. Thereafter, the water-absorbing agent composition was dehydrated using a centrifuge (250 G), and then absorption capacity without pressure (CRC) (unit: g/g) was measured.

AAP (Absorption Capacity Under Pressure)

AAP (absorption capacity under pressure) of the water-absorbing agent composition according to the present invention was measured in accordance with the EDANA method (NWSP 242.0. R2 (19)). Specifically, 0.9 g of the water-absorbing agent composition was swollen under a load of 4.83 kPa (49 g/cm$^2$, 0.7psi) for 1 hour relative to a large excess of a 0.9 mass% aqueous sodium chloride solution, and then the absorption capacity under pressure (AAP) (unit: g/g) was measured.

SFC (Saline Flow Conductivity)

SFC (saline flow conductivity) of the water-absorbing agent composition according to the present invention was measured in accordance with the measurement method described in US 5669894.

[Washburn Method Contact Angles θ1 and θ2]

The Washburn method contact angles θ1 and θ2 of the water-absorbing agent composition according to the present invention were determined by measuring the speed at which the water-absorbing agent composition sucks up a sample liquid by capillary force using a surface tensiometer (DY-500 available from Kyowa Interface Science Co., Ltd.) and measurement software DYNALYZER (Ver. 2.2.5.0), and calculating the contact angles based on the Lukas-Washburn equation ((Equation 1) described below). θ2 is obtained by changing a filter paper (θ1) to a stainless steel screen (θ2) having a mesh size of 400 mesh (36 μm) similar to the mesh used for AAP measurement to eliminate an adverse effect at the time of liquid suction of the water-absorbing agent composition, and the measurement principle, the test procedure, and the like are common.

(Measurement Principle)

The capillary phenomenon of the absorbent structure containing the water-absorbing agent composition in the present invention depends on the contact angle θ of the liquid that wets the particles or particulate matters. The contact angle θ is calculated from (Equation 1) and (Equation 2) described below. When the object to be measured is a powder, to obtain the contact angle θ, it is general to perform a permeation speed test using two types of liquids.

In the present description, first, a permeation speed test using n-hexane (contact angle θ is extremely close to zero) was performed, and "c" was determined according to (Equation 2) described below. Subsequently, a permeation speed test was performed using a 20 mass% aqueous sodium chloride solution adjusted to a liquid temperature of 15°C,

and "c" determined with n-hexane and the measurement result were substituted into (Equation 1) described below to determine the contact angle θ with the 20 mass% aqueous sodium chloride solution.

[Math. 1]

$$\cos\theta = \frac{m^2}{t}\frac{\eta}{c\rho^2\sigma} \quad \cdot\cdot\cdot \text{(Equation 1)}$$

where

m: sucked mass (g)
t: measurement time (sec)
as parameters in 20 mass% aqueous sodium chloride solution at 15°C,
η: absolute viscosity of liquid (mPa·s) = 1.768
ρ: density of liquid (g/cm$^3$) = 1.150
σ: surface tension of liquid (mN/m) =79.99.

"m$^2$/t" in (Equation 1) means the speed at which a 20 mass% aqueous sodium chloride solution is sucked up into the water-absorbing agent composition by capillary force, and the permeation speed was calculated from the difference in m$^2$ between 10 seconds and 20 seconds after the start of the measurement.

[Math. 2]

$$c = \frac{m^2}{t}\frac{\eta}{\rho^2\sigma\cos\theta} \quad \cdot\cdot\cdot \text{(Equation 2)}$$

where

m: sucked mass (g)
t: measurement time (sec)
as parameters in n-hexane at 20°C,
η: absolute viscosity of liquid (mPa·s) = 0.320
ρ: density of liquid (g/cm$^3$) = 0.66
σ: surface tension of liquid (mN/m) = 18.4
cosθ: contact angle of liquid = 1 (θ of n-hexane = 0 °).

"m$^2$/t" in (Equation 2) means the speed at which n-hexane is absorbed into the water-absorbing agent composition by capillary force, and the permeation speed was calculated from the difference in m$^2$ between 10 seconds and 20 seconds after the start of the measurement.

Test Procedure: θ1

Next, the test procedure: θ1 is shown. In each measurement, the following operations were performed. All the consumables with item numbers are described in the web catalog "Surface Tensiometer DyneMaster Series Option" on the homepage of Kyowa Interface Science Co., Ltd., and unless otherwise specified, the test procedure was in accordance with the moving image manual of the CD attached to DyneMaster DY-500.

First, to obtain the constant "c", the following operation was performed. Homogeneous n-hexane (FUJIFILM Wako Pure Chemical Corporation, special grade reagent) was prepared, and the temperature was adjusted to a constant temperature (20°C). Next, a measuring column was assembled using a round filter paper for powder measurement (product number 7221), a Teflon (trade name) column (product number 7279), and a Teflon (trade name) spinneret for column (product number 8077). At this time, in a state where the water-absorbing agent composition was not contained, the filter paper was compressed using an attached compressor, the gap between the spinneret and the filter paper was narrowed, and the heights of the spinneret surface and the bottom surface of the filter paper were aligned.

Then, 3.0 g of the water-absorbing agent composition was weighed and placed in the Teflon (trade name) column, and then the side surface of the column was patted with a finger 10 times to smooth the surface of the water-absorbing agent composition without compressing using the attached compressor. Thereafter, a column holder (product number 7175) was attached to the upper part of the column, and was suspended from the upper part of the measurement apparatus using a small hook (product number 6947).

Subsequently, a test liquid was prepared. That is, an attached petri dish (product number 1196) was rinsed well with pure water in advance to remove substances affecting the surface tension, such as sebum and a surfactant, and then washed three times with n-hexane for sufficient replacement. Thereafter, n-hexane adjusted to 20°C was poured to the line indicated in the petri dish, and the petri dish was placed on the stage of the apparatus.

Next, measurement conditions were set with measurement software in the following order.

1) Select "Powder contact angle measurement" from "Selection of measurement type"
2) Select "Powder permeation speed measurement" from "Measurement setting" > "Measurement" > "Measurement"
3) In "measurement setting" > "measurement" > "basic setting", enter the wet sensitivity of 0.03 g, the measurement end time of 60 seconds, and the sampling interval of 0.1 seconds. Select full auto in the operation method. Uncheck the valid range. Enter 10.0% for both the start and end ranges in the calculation range.
4) Input powder column radius of 5.00 mm in "Measurement setting" > "Measurement" > "Measurement element setting"
5) Input values of surface tension, density, and viscosity of liquid sample, and density of powder sample in "Measurement setting" > "Measurement" > "Sample setting"
6) In the lower part of "Measurement setting" > "Measurement" > "Sample setting", select "Weight" from among "Porosity", "Weight", and "Filling height", and input the mass of the filled water-absorbing agent composition.
7) In "Measurement setting" > "Measurement" > "Stage operation setting", input 0.200 mm/s for both the stage raising speed and the stage lowering speed, and input 2.0 mm as the lowering distance after measurement.
8) As necessary, from "Measurement setting" > "Display" > "Graph axis setting" and "Output setting", set a vertical axis and a horizontal axis of a graph desired to be viewed during measurement and immediately after measurement and parameters to be output.

After the measurement is completed, a CSV file in which all pieces of information necessary for the calculation using (Equation 1) and (Equation 2) are recorded can be obtained.

After setting of the measurement conditions was completed, the software was operated to raise the stage of the apparatus, and the apparatus was sufficiently brought close to the position of the spinneret of the column, and then measurement was started. When the set measurement end time elapses, the measurement automatically ends. A regression line was set assuming that a change in the square ($m^2$) of the mass of the liquid sucked up between 10 seconds and 20 seconds after the start of the measurement was a straight line. The slope of the regression line was determined as a value of "$m^2/t$". The operation described above was performed three times in total by replacing the sample every time. The average value of the obtained three measured values was substituted into (Equation 2) to calculate the constant "c".

Subsequently, a 20 mass% aqueous sodium chloride solution whose temperature was adjusted to 15°C was prepared as a test liquid, and the same operation as described above was performed except that n-hexane was changed to the test liquid, and the average value of "$m^2/t$" was determined. The reason of not employing a 0.9 mass% aqueous sodium chloride solution (physiological saline) as the test liquid and deviation of the temperature from the body temperature of 36°C is to suppress absorption of the water-absorbing agent composition.

From the constant "c" obtained through the operation described above and "$m^2/t$" of the 20 mass% aqueous sodium chloride solution, the Washburn contact angle $\theta$ was determined from (Equation 1). The contact angle $\theta$ obtained through the operation described above was defined as "$\theta 1$".

Test Procedure: $\theta 2$

The same operation as in the test procedure: $\theta 1$ was performed except that in the test procedure: $\theta 1$, the filter paper for powder measurement (product number 7221) was changed to the mesh used for the screen of the cylinder for AAP measurement, and "$m^2/t$" was the slope of the regression line for 0 seconds to 10 seconds after the start of measurement. In the measurement of the contact angle $\theta 2$, to eliminate the influence of the water-absorbing agent composition absorbing the test liquid, the following absorption mass correction was performed.

In the above-described absorption mass correction, the difference between the "measured value of the sucked mass" of the 20 mass% aqueous sodium chloride solution in a period from 0 seconds to 10 seconds after the start of measurement and the "absorption mass" of the 20 mass% aqueous sodium chloride solution in a period from 0 seconds to 10 seconds after the start of measurement was taken as the absorption correction sucked mass.

The "absorption mass" was measured by the following method.

First, in the method for measuring FSR (water absorption speed) described in [0196] to [0197] of WO 2009/016055, which are incorporated by reference, FSR (water absorption speed) of the water-absorbing agent composition was measured by changing the absorption liquid to 2 g of a 20 mass% aqueous sodium chloride solution at a temperature of 15°C. Hereinafter, FSR (water absorption speed) of the water-absorbing agent composition measured by changing the absorption liquid to 2 g of a 20 mass% aqueous sodium chloride solution at a temperature of 15°C is defined as FSR (20). Here, the temperature and concentration of the aqueous sodium chloride solution are adjusted to those of the aqueous sodium chloride solution used for measurement of the contact angle.

Next, the position of the mesh of the contact angle measurement column was set to a height of 0 mm, and scales in increments of 1 mm in the height direction were attached to the outer surface of the column. After 3.0 g of the water-absorbing agent composition was put into the column, the side surface was tapped 10 times. Thereafter, the height of the water-absorbing agent composition was read from the scale, and the mass of the water-absorbing agent composition was divided by the height of the water-absorbing agent composition to calculate the mass of the water-absorbing agent composition of 1 mm.

For each measurement, the sucking state of the 20 mass% aqueous sodium chloride solution was captured as a moving image, and the value obtained by multiplying the difference (mm) in liquid height between 10 seconds and 20 seconds after the start of measurement read from the scale by the mass of the water-absorbing agent composition of 1 mm was taken as a mass (a) of the water-absorbing agent composition involved in absorption.

Next, an absorption mass per 0.1 seconds was calculated from the mass (a) of the water-absorbing agent composition involved in absorption and FSR (20), and the mass obtained by accumulating 0 seconds to 10 seconds was defined as "absorption mass". Specifically,

the absorption mass (x) at 0.1 seconds is a × (1/100) × FSR (20) × 0.1 seconds,
the absorption mass (y) at 0.2 seconds is x + a × (2/100) × FSR (20) × 0.1 seconds, and
the absorption mass (z) at 0.3 seconds is y + a × (3/100) × FSR (20) × 0.1 seconds. This procedure was repeated to calculate the cumulative absorption mass from 0 seconds to 10 seconds.

From the constant "c" obtained through the operation described above and "$m^2/t$" of the 20 mass% aqueous sodium chloride solution on which absorption mass correction was performed, the Washburn contact angle θ was determined from (Equation 1). The contact angle θ obtained through the operation described above was defined as "θ2".

[Gap Water Ratio Under Pressure]

The gap water ratio under pressure of the water-absorbing agent composition according to the present invention was measured in accordance with the measurement method described in [0225] to [0251] of WO 2016/111223, which are incorporated by reference.

Specific Surface Area

The specific surface area of the water-absorbing agent composition according to the present invention was measured in accordance with the measurement method described in WO 2021/162072.

Specifically, the specific surface value was obtained by analyzing three-dimensional image data of the water-absorbing agent composition or the water-absorbent resin acquired using a microfocus X-ray CT system (available from Shimadzu Corporation: inspeXio SMX-100CT) with high-speed three-dimensional analysis software (available from RATOC SYSTEM ENGINEERING CO., LTD.: TRI/3D-VOL-FCS64). The measurement conditions of the X-ray CT system and calculation of the specific surface area (analysis by analysis software) are as described in [0182] to [0184] of WO 2021/162072 ([0301] to [0327] in US 2023/076935), which is incorporated by reference.

Water Content (WSP230.3 (10))

The "water content" means the amount of water in the water-absorbing agent composition or the water-absorbent resin as determined from the weight loss upon drying 1 g of the water-absorbing agent composition or the water-absorbent resin at 105°C for 3 hours. Although "Moisture Content" is described in WSP230.3 (10), the contents are substantially the same.

Vortex (Water Absorption Speed)

Vortex (water absorption speed) of the water-absorbing agent composition according to the present invention was measured by the following procedure in accordance with JIS K 7224 (1996). First, 0.02 parts by mass of Food Blue No.

1 (CAS No. 3844-45-9) as a food additive was added to 1000 parts by mass of physiological saline for coloring, and the liquid temperature was adjusted to 30°C. This was used as a test liquid. Next, 50 mL of the test liquid was weighed in a beaker having a volume of 100 mL, a cylindrical stirrer having a length of 40 mm and a diameter of 8 mm was put in the beaker, and stirring was started at 600 rpm. Subsequently, 2.0 g of the water-absorbent resin was placed in the test liquid during stirring, and the time until the stirrer (stirrer chip) was covered with the test liquid was measured and taken as the water absorption speed by the Vortex method.

Surface Tension

The surface tension of the water-absorbing agent composition according to the present invention was measured by the following method.

First, 40 ml of a 0.9 mass% aqueous sodium chloride solution adjusted from 23°C to 24°C was put in a sufficiently washed 50 ml beaker, and the surface tension of the 0.9 mass% aqueous sodium chloride solution was measured using a surface tensiometer (K11 automatic surface tensiometer available from KRUSS GmbH). In this measurement, the value of the surface tension needs to be within the range of 72 mN/m to 74 mN/m.

Next, after surface tension measurement adjusted from 23°C to 24°C was performed, a sufficiently washed cylindrical stirrer having a length of 25 mm and a section diameter of 7 mm and 0.5 g of the water-absorbing agent composition were put into the beaker containing 40 ml of the 0.9 mass% aqueous sodium chloride solution, and the content was stirred at 350 rpm for 3 minutes. After 3 minutes, the stirring was stopped, and the mixture was allowed to stand for 2 minutes. After the water-absorbing agent composition that had absorbed water was settled, the surface tension of the supernatant was measured again through the same operation. In the present measurement, a plate method using a platinum plate was adopted, and the plate was used after being sufficiently washed with deionized water and heated and washed with a gas burner before each measurement.

Peroxide Remaining Amount

The amount of peroxide decomposition product present on the particle surface of the water-absorbing agent composition can be measured by the following method.

Pretreatment 1: Impact Test

The water-absorbing agent composition in an amount of 30 g and 10 g of glass beads (soda lime glass beads for precision fractionation filling) having a diameter of 6 mm were placed in a glass container (available from Yamamura Glass Co., Ltd./mayonnaise 225) having a diameter of 6 cm and a height of 11 cm, and the container was sealed with a resin inner lid and an outer lid (available from TOP CORPORATION/product code: 604-003/product name: inner lid and outer lid attached to mayonnaise bottle PP cap set). Thereafter, a paint shaker (available from Toyo Seiki Seisaku-sho, Ltd./disperser for test: product No. 488) was shaken at 800 times/min to pulverize the water-absorbing agent composition. The shaking time was adjusted for each water-absorbing agent composition to be measured so that Relationship (3) described below was satisfied.

0.2 < (mass of particle group a)/(total mass of particle group a and particle group b) < 0.3 ••• Relationship (3) Relationship (3)

The details of the paint shaker are described in JP 9-235378 A. After the shaking, the glass beads were removed using JIS standard sieves having a mesh size of 2 mm.

(Pretreatment 2: Sieving)

The water-absorbing agent composition after pulverization obtained in the impact test of the pretreatment 1 was sieved into a particle group a having a particle size of 300 μm or more and a particle group b having a particle size of less than 300 μm using a JIS standard sieve having a mesh size of 300 μm. Most of the particle surface portions scraped off by the pulverization passed through the JIS standard sieve having a mesh size of 300 μm.

Quantification of Peroxide

The persulfate added as a peroxide in Examples of the present application is decomposed in the step of producing the water-absorbing agent composition (mainly drying step and heat treatment step), and the residual

component (peroxide decomposition product) is present in the water-absorbing agent composition as a sulfate. For example, in the case of sodium persulfate, the residual component is sodium sulfate. Hereinafter, the quantification of sodium sulfate will be described in detail, but the quantification of other substances is also measured according to the present method.

The following configuration is exemplified as the ion chromatography used for quantification of sodium sulfate.

Ion chromatography system: Dionex Integrion RFIC system

Column: Dionex IonPac AS-18, Dionex IonPac AG-18

Sodium sulfate to be measured was dissolved in a 0.1 mass% aqueous formaldehyde solution, and an aqueous sodium sulfate solution having any concentration was produced as a standard aqueous solution. The standard aqueous solution was analyzed using the ion chromatography having the configuration described above, and a calibration curve was produced from the relationship between the peak area of the obtained chromatogram and the concentration of sodium sulfate. Since the peak detected by ion chromatography is a peak derived from sulfate ions generated through ionization of sodium sulfate, mass conversion was performed assuming that the total amount of sulfate ions was sodium sulfate at the time of calculation.

Subsequently, 0.1 g of each of the water-absorbing agent compositions of the particle group a and the particle group b obtained in the pretreatment 2 was separately placed in a cylindrical polypropylene container having a volume of about 250 mL, 100 g of a 0.1 mass% aqueous formaldehyde solution was further added, and the resulting mixture was stirred at 500 rpm for 1 hour with a cylindrical stirrer chip having a length of 25 mm. After the stirring, a supernatant in which the swollen water-absorbing agent composition was not present was collected and filtered through a sample pretreatment filter (GL chromatographic disk, 25 A, 0.2 μm, Cat. No. 5040-28502) to obtain extraction liquid a and extraction liquid b to be measured.

The extraction liquid a and the extraction liquid b were subjected to quantitative analysis by the ion chromatography, and contents C1 and C2 of sodium sulfate remaining in the water-absorbing agent composition of the particle group a and the particle group b were determined from the calibration curve prepared in advance.

As the raw material compounds, reaction reagents, and solvents used in Examples and Comparative Examples, commercially available products (for example, commercially available products sold by NIPPON SHOKUBAI CO., LTD.) were used unless otherwise noted. In addition, the type of standard aqueous solution was changed in accordance with the residual components. For example, in the case of potassium persulfate, the residual component is potassium sulfate, and thus, an aqueous potassium sulfate solution was used as the standard aqueous solution.

The content of the peroxide remaining in the water-absorbing agent composition of the particle group a and the particle group b was a value obtained by correcting the solid content by (Equation 4) shown below.

Water content-corrected content (mass%) = content (mass%)/(charged amount of water-absorbing agent composition 0.1g × solid content (mass%)) ••• (Equation 4) (Equation 4)

The solid content in (Equation 4) was determined from (Equation 5).

$$\text{Solid content (mass\%)} = 100 - \text{water content (mass\%)} \quad \cdot \cdot \cdot \quad \text{(Equation 5)}$$

The water content of the water-absorbing agent composition was measured as described above.

Water Vapor Density in Heat Treatment Apparatus

In the production method according to the present invention, the water vapor density in the heat treatment apparatus was measured by the following method. That is, it is a value measured in an atmosphere vertically above the content (water-absorbent resin) heated by the heating unit of the heat treatment apparatus, and a gas present above the powder surface of the content within 5 cm, preferably within 3 cm, more preferably within 1 cm was collected and measured.

Examples of the method for collecting the gas include a method of storing the gas in a cylinder container having an appropriate capacity, and a method of sucking the gas using a pump and condensing or absorbing an organic surface crosslinking agent and water vapor (condensable component) in the middle. From the viewpoint of measurement accuracy, the latter method is preferred, and an apparatus having the following configuration is preferred.

As the gas collection apparatus, an apparatus including a sampling line that is a hard tube having an inner diameter of 1 mm or more and 10 mm or less and having both heat resistance and chemical resistance, preferably made of

SUS, a gas switching unit, preferably a 60000 psi heat resistance valve, a trap part that condenses or absorbs a condensable component, a flow rate measuring part that measures a flow rate of a non-condensable gas that has passed through the trap part, and a suction pump connected to a downstream side of the flow rate measuring part is preferred.

Further, it is preferred that the portion on the upstream side of the trap part has a specification capable of keeping the temperature to a temperature equal to or higher than the temperature of the collected gas at the inlet of the sampling line. The temperature at the time of heat retention is preferably 100°C or higher, more preferably 100°C or higher and 150°C or lower. When clogging or the like due to dust occurs, a filter, a cyclone dust collector, or the like is preferably installed in the sampling line.

The flow rate of the collected gas may be a flow rate at which a value obtained by dividing the capacity from the inlet of the sampling line to the inlet of the flow measurement part by the flow rate of the non-condensable gas is preferably 10 seconds or less, more preferably 5 seconds or less, still more preferably 3 seconds or less. The amount of water in the collected condensed liquid (solution of the condensed component) can be determined by a Karl Fischer titration method or the like.

Production of Water-Absorbent Resin

Production Example 1

(Step of Preparing Aqueous Monomer Solution)

Into a polypropylene container having a capacity of 2 L, 421.7 parts by mass of acrylic acid, 140.4 parts by mass of a 48 mass% aqueous sodium hydroxide solution, 2.3 parts by mass of polyethylene glycol diacrylate (PEGDA, n = 9), 1.3 parts by mass of a 2.0 mass% aqueous diethylenetriamine pentaacetic acid/trisodium solution, 4.4 parts by mass of a 1.0 mass% aqueous polyoxyethylene (20) sorbitan monostearate (available from Kao Corporation) solution, and 390.3 parts by mass of deionized water were charged and mixed, whereby an aqueous monomer solution The deionized water was heated to 40°C in advance.

Polymerization Step

Subsequently, the aqueous monomer solution (S1') was cooled while being stirred, and when the liquid temperature reached 39°C, 211.9 parts by mass of a 48 mass% aqueous sodium hydroxide solution was charged into the aqueous monomer solution (S1') in about 20 seconds in an atmosphere open state and mixed, whereby an aqueous monomer solution (S1) was prepared. At this time, the temperature of the aqueous monomer solution (S1) was increased to about 81°C with the heat of neutralization and the heat of dissolution generated in the mixing process.

Next, using a wooden glass ball filter (filter particle No. 4, available from Kinoshita Rika Kogyo Co., Ltd.), nitrogen gas was introduced into the aqueous monomer solution (S1) under the conditions of a pressure of 0.1 MPa and a flow rate of 0.1 L/min for 10 seconds into the stirred aqueous monomer solution (S1), 17.6 parts by mass of a 4.0 mass% aqueous sodium persulfate solution was then added, and the mixture was further stirred for about 5 seconds, and poured into a stainless butt-shaped container (bottom surface 340 × 340 mm, height 25 mm, inner surface: Teflon (trade name) coating) in an atmosphere open state. The time from the start of the second stage neutralization to the pouring of the aqueous monomer solution (S1) into the butt-shaped container was set to 55 seconds, and the butt-shaped container was heated using a hot plate (available from Iuchi Seieido Co., Ltd.: NEO HOTPLATE HI-1000) until the surface temperature reached 40°C. The aqueous monomer solution (S1) was poured into the butt-shaped container, and after 59 seconds, a polymerization reaction started. The polymerization reaction proceeded with expansion and foaming in all the directions upward while generating water vapor, and then the material shrank to a size slightly larger than the bottom surface of the butt-shaped container. The polymerization reaction (expansion, contraction) was completed within about 1 minute. A hydrogel (S1) was taken out 3 minutes after the start of the polymerization reaction.

Gel-Grinding Step

Next, the hydrogel (S1) was cut into an appropriate size, and then supplied to a meat chopper having a porous plate having a diameter of 100 mm, a hole diameter of 6.4 mm, the number of holes of 83, an opening ratio of 34%, and a thickness of 10 mm at the tip portion, a screw shaft outer diameter of 86 mm, a screw shaft rotational speed of 130 rpm, and a casing inner diameter of 88 mm. The hydrogel was gel-ground, whereby a particulate hydrogel (S1) was obtained. The obtained particulate hydrogel (S1) had a mass-average particle diameter of 360 μm.

Drying Step

Next, the particulate hydrogel (S1) was spread and placed on a wire mesh with a mesh size of 300 μm, and placed in a hot-air dryer. Thereafter, the particulate hydrogel (S1) was dried by passing hot air at 190°C for 30 minutes to obtain a dried polymer (S1).

Pulverizing Step/Classification Step

Subsequently, the dried polymer (S1) was charged into a roll mill (available from Inokuchi Giken Co., Ltd.: WML type roll pulverizing apparatus) and pulverized, and then classified using two types of JIS standard sieves having mesh sizes of 710 μm and 150 μm, whereby a water-absorbent resin (S1) before surface crosslinking in an irregularly crushed shape was obtained. In the obtained water-absorbent resin (S1) before surface crosslinking, CRC was 33.0 g/g, the specific surface area was 41 $m^2$/kg, D50 was 390 μm, and the mass proportion of particles having a particle diameter of less than 150 μm was 2.6 mass%. The physical properties of the water-absorbent resin (S1) before surface crosslinking are also described in Tables 1-1 and 2-1.

Surface Crosslinking Step

Next, a surface crosslinking agent solution composed of 0.18 parts by mass of 1,6-hexanediol, 0.4 parts by mass of triethylene glycol, 0.01 parts by mass of a 10.0 mass% aqueous polyoxyethylene (20) sorbitan monostearate solution, and 3.0 parts by mass of deionized water, relative to 100 parts by mass of the water-absorbent resin (S1) before surface crosslinking, was added from two places using a high-speed stirring continuous mixer and mixed uniformly, whereby a mixture (S1) was obtained.

Subsequently, the mixture (S1) was subjected to a heat treatment while being stirred for 30 minutes in a heat treatment apparatus (heat medium temperature: 205°C) in which the atmospheric temperature was adjusted to 180°C, and the gauge pressure in the heat treatment apparatus was adjusted to adjust the water vapor density to 0.42 g/L. Thereafter, the obtained material was crushed until it passed through a JIS standard sieve with a mesh size of 850 μm, whereby a water-absorbent resin (S1) after surface crosslinking was obtained.

Comparative Example 1

The water-absorbent resin (S1) before surface crosslinking in Production Example 1 was used as a water-absorbing agent composition (C1) obtained in Comparative Example 1. Table 1-2 shows various conditions in Comparative Example 1, and Table 1-3 shows various physical properties of the water-absorbing agent composition (C1).

Comparative Example 2

The water-absorbent resin (S1) after surface crosslinking in Production Example 1 was used as a water-absorbing agent composition (C2) after surface crosslinking obtained in Comparative Example 2. Table 1-2 shows various conditions in Comparative Example 2, and Table 1-3 shows various physical properties of the water-absorbing agent composition (C2).

Example 1

A water-absorbent resin (1) after surface crosslinking was obtained by performing the same operations as those of Comparative Example 2 except that the surface crosslinking agent solution was changed to a mixed solution composed of 0.18 parts by mass of 1,6-hexanediol, 0.4 parts by mass of triethylene glycol, 0.01 parts by mass of a 10.0 mass% aqueous polyoxyethylene (20) sorbitan monostearate solution, 0.2 parts by mass of sodium persulfate, and 3.0 parts by mass of deionized water, relative to 100 parts by mass of the water-absorbent resin (S1) before surface crosslinking, in Comparative Example 2. The water-absorbent resin (1) after surface crosslinking was used as a water-absorbing agent composition (1). Table 1-2 shows various conditions in Example 1, and Table 1-3 shows various physical properties of the water-absorbing agent composition (1).

Example 2

A water-absorbent resin (2) after surface crosslinking was obtained by performing the same operations as those of Example 1 except that the gauge pressure in the heat treatment apparatus was adjusted to adjust the water vapor density of the heat treatment apparatus to 0.13 g/L in Example 1. The water-absorbent resin (2) after surface crosslinking

was used as a water-absorbing agent composition (2). Table 1-2 shows various conditions in Example 2, and Table 1-3 shows various physical properties of the water-absorbing agent composition (2).

Comparative Example 3

A water-absorbent resin (C3) after surface crosslinking was obtained by performing the same operations as those of Comparative Example 2 except that the gauge pressure in the heat treatment apparatus was adjusted to adjust the water vapor density of the heat treatment apparatus to 0.13 g/L in Comparative Example 2. The water-absorbent resin (C3) after surface crosslinking was used as a water-absorbing agent composition (C3). Table 1-2 shows various conditions in Comparative Example 3, and Table 1-3 shows various physical properties of the water-absorbing agent composition (C3).

Table 1-1

Table 1-1

| | Name | CRC [g/g] | D50 [μm] | 150↓[1)] [mass%] | Specific surface area [$m^2$/kg] |
|---|---|---|---|---|---|
| Production Example 1 | Water-absorbent resin before surface crosslinking (S1) | 33.0 | 390 | 2.6 | 41 |
| 1) 150 ↓: Proportion of particles of less than 150 μm | | | | | |

Table 1-2

Table 1-2

| | Name | Water-absorbent resin before surface crosslinking | Organic surface crosslinking agent*) | Peroxide | Heat treatment step water vapor density [g/L] |
|---|---|---|---|---|---|
| Comparative Example 1 | Water-absorbing agent composition (C1) | (S1) | None | None | - |
| Example 1 | Water-absorbing agent composition (1) | (S1) | HD, TEG | Sodium persulfate | 0.42 |
| Comparative Example 2 | Water-absorbing agent composition (C2) | (S1) | HD, TEG | None | 0.42 |
| Example 2 | Water-absorbing agent composition (2) | (S1) | HD, TEG | Sodium persulfate | 0.13 |
| Comparative Example 3 | Water-absorbing agent composition (C3) | (S1) | HD, TEG | None | 0.13 |
| *) Abbreviation for organic surface crosslinking agent<br>HD: 1,6-hexanediol<br>TEG: triethylene glycol | | | | | |

Table 1-3

Table 1-3

| | Name | Specific surface area | Washburn method contact angle θ2 | Washburn method contact angle θ1 | Peroxide residue on surface of water-absorbing agent composition | CRC | SFC | D50 | 150↓ 2) | Water content | Surface tension | Vortex | AAP | gap water ratio under pressure |
|---|---|---|---|---|---|---|---|---|---|---|---|---|---|---|
| | | [$m^2$/kg] | [°] | [°] | [mass%] | [g/g] | [1)] | [μm] | [mass%] | [mass%] | [mN/m] | [Seconds] | [g/g] | [g/g] |
| Comparative Example 1 | Water-absorbing agent composition (C1) | 41 | 70 | 60 | | 33.0 | - | 390 | 2.6 | 3.5 | 72 | | - | - |
| Example 1 | Water-absorbing agent composition (1) | 41 | 60 | 50 | 0.09 | 29.5 | 32 | 390 | 2.6 | 1.4 | 70 | 23 | 27.1 | 12.4 |
| Comparative Example 2 | Water-absorbing agent composition (C2) | 41 | 76 | 69 | 0.00 | 29.3 | 31 | 390 | 2.8 | 1.3 | 71 | 27 | 25.4 | 11.8 |
| Example 2 | Water-absorbing agent composition (2) | 41 | 69 | | 0.06 | 29.6 | 30 | 390 | 2.5 | 1.5 | 71 | 25 | 25.8 | 12.4 |
| Comparative Example 3 | Water-absorbing agent composition (C3) | 41 | 77 | | 0.00 | 31.0 | 30 | 390 | 2.7 | 1.4 | 72 | 29 | 24.5 | 11.7 |

1) Unit of SFC: $\times 10^{-7}$ $cm^3 \cdot sec/g$
2) 150 ↓: Proportion of particles of less than 150 μm

Summary

From the results of the water-absorbing agent compositions of Example 1 and Comparative Example 2 under the same conditions except for the addition of the peroxide in the surface crosslinking step, the water-absorbing agent composition of Example 1 having the contact angle θ2 of 72° or less had a lower Vortex and a higher AAP and gap water ratio under pressure than the water-absorbing agent composition of Comparative Example 2. Similarly, from the results of the water-absorbing agent compositions of Example 2 and Comparative Example 3 under the same conditions except for the addition of the peroxide in the surface crosslinking step, the water-absorbing agent composition of Example 2 having the contact angle θ2 of 72° or less had a lower Vortex and a higher AAP and gap water ratio under pressure than the water-absorbing agent composition of Comparative Example 3. In addition, from the value of the peroxide residue on the surface of the water-absorbing agent composition, it is found that the peroxide added in the surface treatment step remains on the particle surface and acts on the surface. In Comparative Example 1, the surface treatment was not performed, and the values of the AAP and the gap water ratio under pressure are very small. Thus, the measurement was not performed.

Comparative Example 4

A water-absorbing agent composition (C4) after surface crosslinking was obtained by performing the same operation as in Production Example 1 except that in the surface crosslinking step in Production Example 1, the surface crosslinking agent solution was changed to a surface crosslinking agent solution composed of 0.4 parts by mass of 1,4-butanediol, 0.6 parts by mass of propylene glycol, 0.01 parts by mass of a 10.0 mass% aqueous polyoxyethylene (20) sorbitan monostearate solution, 0.1 parts by mass of sodium persulfate, and 2.7 parts by mass of deionized water, relative to 100 parts by mass of the water-absorbent resin (S1) before surface crosslinking, and further the mixture (S1) was subjected to a heat treatment while being stirred for 30 minutes in a heat treatment apparatus (heat medium temperature: 200°C) in which the atmospheric temperature was adjusted to 180°C and the gauge pressure in the heat treatment apparatus was adjusted to adjust the water vapor density to 0.05 g/L. Table 2-2 shows various conditions in Comparative Example 4, and Table 2-3 shows various physical properties of the water-absorbing agent composition (C4).

Comparative Example 5

A water-absorbent resin (C5) after surface crosslinking was obtained by performing the same operations as those of Example 1 except that the gauge pressure was adjusted to adjust the water vapor density of the heat treatment apparatus to 0.05 g/L in Example 1. The water-absorbent resin (C5) after surface crosslinking was used as a water-absorbing agent composition (C5). Table 2-2 shows various conditions in Comparative Example 5, and Table 2-3 shows various physical properties of the water-absorbing agent composition (C5).

Example 3

A water-absorbent resin (3) after surface crosslinking was obtained by performing the same operations as those of Comparative Example 4 except that the gauge pressure in the heat treatment apparatus was adjusted to adjust the water vapor density of the heat treatment apparatus to 0.42 g/L in Comparative Example 4. The water-absorbent resin (3) after surface crosslinking was used as a water-absorbing agent composition (3). Table 2-2 shows various conditions in Example 3, and Table 2-3 shows various physical properties of the water-absorbing agent composition (3).

Example 4

A water-absorbent resin (4) after surface crosslinking was obtained by performing the same operations as those of Example 1 except that the gauge pressure in the heat treatment apparatus was adjusted to adjust the water vapor density of the heat treatment apparatus to 0.51 g/L in Example 1. The water-absorbent resin (4) after surface crosslinking was used as a water-absorbing agent composition (4). Table 2-2 shows various conditions in Example 4, and Table 2-3 shows various physical properties of the water-absorbing agent composition (4).

Table 2-1

Table 2-1

| | Name | CRC [g/g] | D50 [μm] | 150↓1) [mass%] | Specific surface area [$m^2$/kg] |
|---|---|---|---|---|---|
| Production Example 1 | Water-absorbent resin before surface crosslinking (S1) | 33.0 | 390 | 2.6 | 41 |

Table 2-2

Table 2-2

| | Name | Water-absorbent resin before surface crosslinking | Organic surface crosslinking agent*) | Peroxide | Heat treatment step water vapor density [g/L] |
|---|---|---|---|---|---|
| Comparative Example 4 | Water-absorbing agent composition (C4) | (S1) | BD, PG | Sodium persulfate | 0.05 |
| Comparative Example 5 | Water-absorbing agent composition (C5) | (S1) | HD, TEG | Sodium persulfate | 0.05 |
| Example 2 | Water-absorbing agent composition (2) | (S1) | HD, TEG | Sodium persulfate | 0.13 |
| Example 1 | Water-absorbing agent composition (1) | (S1) | HD, TEG | Sodium persulfate | 0.42 |
| Example 3 | Water-absorbing agent composition (3) | (S1) | BD, PG | Sodium persulfate | 0.42 |
| Example 4 | Water-absorbing agent composition (4) | (S1) | HD, TEG | Sodium persulfate | 0.51 |
| *) Abbreviation for organic surface crosslinking agent<br>HD: 1,6-hexanediol<br>TEG: triethylene glycol<br>BD: 1,4-butanediol<br>PG: Propylene glycol | | | | | |

Table 2-3

Table 2-3

| | Name | Specific surface area | Washburn method contact angle θ2 | Washburn method contact angle θ1 | Peroxide residue on surface of water-absorbing agent composition | CRC | SFC | D50 | 150↓ 2) | Water content | Surface tension | Vortex | AAP | gap water ratio under pressure |
|---|---|---|---|---|---|---|---|---|---|---|---|---|---|---|
| | | [m²/kg] | [°] | [°] | [mass%] | [g/g] | [ 1) ] | [μm] | [mass%] | [mass%] | [mN/m] | [Seconds] | [g/g] | [g/g] |
| Comparative Example 4 | Water-absorbing agent composition (C4) | 41 | 81 | 74 | 0.01 | 30.0 | 30 | 390 | 2.3 | 1.5 | 69 | 31 | 24.3 | 11.6 |
| Comparative Example 5 | Water-absorbing agent composition (C5) | 41 | 78 | | 0.01 | 29.7 | 29 | 390 | 2.7 | 1.2 | 72 | 28 | 25.0 | 12.3 |
| Example 2 | Water-absorbing agent composition (2) | 41 | 69 | | 0.06 | 29.6 | 30 | 390 | 2.5 | 1.5 | 71 | 25 | 25.8 | 12.4 |
| Example 1 | Water-absorbing agent composition (1) | 41 | 60 | 50 | 0.09 | 29.5 | 32 | 390 | 2.6 | 1.4 | 70 | 23 | 27.1 | 12.4 |
| Example 3 | Water-absorbing agent composition (3) | 41 | 63 | | 0.07 | 29.8 | 33 | 390 | 2.3 | 1.5 | 68 | 27 | 26.4 | 12.3 |

(continued)

| | Name | Specific surface area | Washburn method contact angle θ2 | Washburn method contact angle θ1 | Peroxide residue on surface of water-absorbing agent composition | CRC | SFC | D50 | 150↓ 2) | Water content | Surface tension | Vortex | AAP | gap water ratio under pressure |
|---|---|---|---|---|---|---|---|---|---|---|---|---|---|---|
| | | [$m^2$/kg] | [°] | [°] | [mass%] | [g/g] | [ 1) ] | [μm] | [mass%] | [mass%] | [mN/m] | [Seconds] | [g/g] | [g/g] |
| Example 4 | Water-absorbing agent composition (4) | 41 | 65 | | 0.06 | 29.7 | 29 | 390 | 2.7 | 1.6 | 70 | 28 | 27.5 | 12.5 |

1) Unit of SFC: $\times 10^{-7}$ $cm^3 \cdot sec/g$
2) 150 ↓: Proportion of particles of less than 150 μm

Summary

From the water-absorbing agent compositions of Examples 1, 2, 4, and Comparative Example 5, and the results of the water-absorbing agent compositions of Example 3 and Comparative Example 4 under the same conditions except for the water vapor density in the surface crosslinking step, the water-absorbing agent compositions of Examples having the contact angle θ2 of 72° or less had a lower Vortex and a higher AAP and gap water ratio under pressure.

Production Example 2

According to Reference Example 1 described in JP 4926474 B, a water-absorbent resin (S2) before surface crosslinking and a water-absorbent resin (S2) after surface crosslinking step were obtained.

Step of Preparing Aqueous Monomer Solution

To 5500 parts by mass of an aqueous sodium acrylate solution having a neutralization percentage of 75 mol% (monomer concentration: 38 mass%), 15.0 parts by mass of polyethylene glycol diacrylate (average molecular weight: 523) was added and mixed, whereby an aqueous monomer solution (S2) was prepared.

Polymerization Step, Gel-Grinding Step

The aqueous monomer solution (S2) was degassed under a nitrogen gas atmosphere for 30 minutes, and then the aqueous monomer solution (S2) was charged into a reactor formed by installing a lid on a jacketed stainless-steel double-arm kneader having an inner volume of 10 L and two sigma-type blades. The inside of the reactor was purged with nitrogen gas while maintaining the temperature of the aqueous monomer solution (S2) at 30°C.

Subsequently, while stirring the aqueous monomer solution (S2) in the reactor, 2.46 parts by mass of sodium persulfate and 0.10 parts by mass of L-ascorbic acid were added. Polymerization reaction started after about 1 minute. The polymerization reaction was continued as it was at 30°C to 90°C, and 60 minutes after the start of the polymerization reaction, a particulate hydrogel (S2) in the reactor content was taken out. The particulate hydrogel (S2) was subdivided into a size of about 5 mm.

Drying Step

The particulate hydrogel (S2) was placed on a wire mesh with a mesh size of 300 μm, and put in a hot-air dryer. Thereafter, the particulate hydrogel (S2) was dried by passing hot air at 150°C for 90 minutes to obtain a dried polymer (S2). There was no undried product in the dried polymer (S2).

Pulverizing Step, Classification Step)

Next, the dried polymer (S2) was charged into a roll mill (WML type roll pulverizing apparatus, available from Inokuchi Giken Co., Ltd.) and pulverized. Thereafter, the pulverized product was classified with JIS standard sieves having mesh sizes of 850 μm and 150 μm. Through the classification operation, a water-absorbent resin (S2) before surface crosslinking in an irregularly crushed shape having a particle diameter of 150 μm or more and less than 850 μm was obtained. The water-absorbent resin (S2) had CRC of 33 g/g, a specific surface area of 22 $m^2$/kg, D50 (mass-average particle diameter) of 410 μm, and a mass proportion of particles of less than 150 μm of 2.2 mass%. The physical properties of the water-absorbent resin (S2) before surface crosslinking are also described in Table 3-1.

Surface Crosslinking Step

Subsequently, a surface crosslinking agent solution composed of 0.18 parts by mass of 1,6-hexanediol, 0.4 parts by mass of triethylene glycol, 0.2 parts by mass of sodium persulfate, and 3.0 parts by mass of deionized water, relative to 100 parts by mass of the water-absorbent resin (S2) before surface crosslinking, was added from two places using a high-speed stirring continuous mixer and mixed uniformly, whereby a mixture (S2) was obtained.

Subsequently, the mixture (S2) was subjected to a heat treatment while being stirred for 30 minutes in a heat treatment apparatus (heat medium temperature: 205°C) in which the atmospheric temperature was adjusted to 180°C, and the gauge pressure in the heat treatment apparatus was adjusted to adjust the water vapor density to 0.42 g/L. Thereafter, the obtained material was crushed until it passed through a JIS standard sieve with a mesh size of 850 μm, whereby a water-absorbent resin (S2) after surface crosslinking was obtained.

Comparative Example 6

The water-absorbent resin (S2) after surface crosslinking in Production Example 2 was used as a water-absorbing agent composition (C6) obtained in Comparative Example 6. Table 3-2 shows various conditions in Comparative Example 6, and Table 3-3 shows various physical properties of the water-absorbing agent composition (C6).

Production Example 3

A water-absorbent resin (S3) before surface crosslinking in an irregularly crushed shape was obtained by performing the same operations as those of Production Example 1 except that the amount of polyethylene glycol diacrylate was changed to 2.5 parts by mass, and the pore size of the porous plate of the meat chopper was changed to 9.5 mm in Production Example 1. The particulate hydrogel (S3) obtained in the middle of production had a mass-average particle diameter of 700 μm, and the water-absorbent resin (S3) before surface crosslinking had an irregularly crushed shape, and had CRC of 33 g/g, a specific surface area of 27 $m^2$/kg, a D50 (mass-average particle diameter) of 390 μm, and a mass proportion of particles having a particle diameter of less than 150 μm of 2.5 mass%.

Subsequently, a surface crosslinking agent solution composed of 0.18 parts by mass of 1,6-hexanediol, 0.4 parts by mass of triethylene glycol, 0.2 parts by mass of sodium persulfate, and 3.0 parts by mass of deionized water, relative to 100 parts by mass of the water-absorbent resin (S3) before surface crosslinking, was added from two places using a high-speed stirring continuous mixer and mixed uniformly, whereby a mixture (S3) was obtained.

Subsequently, the mixture (S3) was subjected to a heat treatment while being stirred for 30 minutes in a heat treatment apparatus (heat medium temperature: 205°C) in which the atmospheric temperature was adjusted to 180°C, and the gauge pressure in the heat treatment apparatus was adjusted to adjust the water vapor density to 0.42 g/L. Thereafter, the obtained material was crushed until it passed through a JIS standard sieve with a mesh size of 850 μm, whereby a water-absorbent resin (S3) after surface crosslinking was obtained.

Example 5

The water-absorbent resin (S3) after surface crosslinking in Production Example 3 was used as a water-absorbing agent composition (5) obtained in Example 5. Table 3-2 shows various conditions in Example 5, and Table 3-3 shows various physical properties of the water-absorbing agent composition (5).

Example 6

A water-absorbent resin (6) after surface crosslinking was obtained by performing the same operations as those of Production Example 3 except that the surface crosslinking agent solution was changed to a mixed solution composed of 0.7 parts by mass of propylene glycol, 0.4 parts by mass of ethylene carbonate, 0.2 parts by mass of sodium persulfate, and 2.9 parts by mass of deionized water, relative to 100 parts by mass of the water-absorbent resin (S3) before surface crosslinking, in Production Example 3. The water-absorbent resin (6) after surface crosslinking was used as a water-absorbing agent composition (6). Table 3-2 shows various conditions in Example 6, and Table 3-3 shows various physical properties of the water-absorbing agent composition (6).

Comparative Example 7

A water-absorbent resin (C7) after surface crosslinking was obtained by performing the same operations as those of Example 6 except that the surface crosslinking agent solution was changed to a mixed solution composed of 0.7 parts by mass of propylene glycol, 0.4 parts by mass of ethylene carbonate, and 2.9 parts by mass of deionized water, relative to 100 parts by mass of the water-absorbent resin (S3) before surface crosslinking, in Example 6. The water-absorbent resin (C7) after surface crosslinking was used as a water-absorbing agent composition (C7). Table 3-2 shows various conditions in Comparative Example 7, and Table 3-3 shows various physical properties of the water-absorbing agent composition (C7).

Example 7

A water-absorbent resin (7) after surface crosslinking was obtained by performing the same operations as those of Production Example 3 except that the surface crosslinking agent solution was changed to a mixed solution composed of 1.1 parts by mass of ethylene carbonate, 0.2 parts by mass of sodium persulfate, and 2.9 parts by mass of deionized water, relative to 100 parts by mass of the water-absorbent resin (S3) before surface crosslinking, in Production Example 3. The water-absorbent resin (7) after surface crosslinking was used as a water-absorbing agent composition (7). Table 3-2

shows various conditions in Example 7, and Table 3-3 shows various physical properties of the water-absorbing agent composition (7).

Example 8

A water-absorbent resin (8) after surface crosslinking was obtained by performing the same operations as those of Production Example 3 except that the surface crosslinking agent solution was changed to a mixed solution composed of 0.8 parts by mass of glycerin, 0.2 parts by mass of sodium persulfate, and 3.0 parts by mass of deionized water, relative to 100 parts by mass of the water-absorbent resin (S3) before surface crosslinking, in Production Example 3. The water-absorbent resin (8) after surface crosslinking was used as a water-absorbing agent composition (8). Table 3-2 shows various conditions in Example 8, and Table 3-3 shows various physical properties of the water-absorbing agent composition (8).

Example 9

A water-absorbent resin (9) after surface crosslinking was obtained by performing the same operations as those of Example 8 except that the heat medium temperature of the heat treatment apparatus was changed to 200°C, in Example 8. The water-absorbent resin (9) after surface crosslinking was used as a water-absorbing agent composition (9). Table 3-2 shows various conditions in Example 9, and Table 3-3 shows various physical properties of the water-absorbing agent composition (6).

Comparative Example 8

A water-absorbent resin (C8) after surface crosslinking was obtained by performing the same operations as those of Example 8 except that the surface crosslinking agent solution was changed to a mixed solution composed of 0.8 parts by mass of glycerin and 3.0 parts by mass of deionized water, relative to 100 parts by mass of the water-absorbent resin (S3) before surface crosslinking, and the heat medium temperature of the heat treatment apparatus was changed to 200°C in Example 8. The water-absorbent resin (C8) after surface crosslinking was used as a water-absorbing agent composition (C8). Table 3-2 shows various conditions in Comparative Example 8, and Table 3-3 shows various physical properties of the water-absorbing agent composition (C8).

Table 3-1

| | Name | CRC [g/g] | D50 [μm] | 150↓1) [mass%] | Specific surface area [$m^2$/kg] |
|---|---|---|---|---|---|
| Production Example 2 | Water-absorbent resin before surface crosslinking (S2) | 33.0 | 410 | 2.2 | 22 |
| Production Example 3 | Water-absorbent resin before surface crosslinking (S3) | 33.0 | 390 | 2.5 | 27 |
| 1) 150 ↓: Proportion of particles of less than 150 μm | | | | | |

Table 3-2

| | Name | Water-absorbent resin before surface crosslinking | Organic surface crosslinking agent*) | Peroxide | Heat treatment step water vapor density [g/L] |
|---|---|---|---|---|---|
| Comparative Example 6 | Water-absorbing agent composition (C6) | (S2) | HD, TEG | Sodium persulfate | 0.42 |
| Example 5 | Water-absorbing agent composition (5) | (S3) | HD, TEG | Sodium persulfate | 0.42 |

(continued)

| | Name | Water-absorbent resin before surface crosslinking | Organic surface crosslinking agent*) | Peroxide | Heat treatment step water vapor density [g/L] |
|---|---|---|---|---|---|
| Example 6 | Water-absorbing agent composition (6) | (S3) | PG, EC | Sodium persulfate | 0.42 |
| Comparative Example 7 | Water-absorbing agent composition (C7) | (S3) | PG, EC | None | 0.42 |
| Example 7 | Water-absorbing agent composition (7) | (S3) | EC | Sodium persulfate | 0.42 |
| Example 8 | Water-absorbing agent composition (8) | (S3) | Gly | Sodium persulfate | 0.42 |
| Example 9 | Water-absorbing agent composition (9) | (S3) | Gly | Sodium persulfate | 0.42 |
| Comparative Example 8 | Water-absorbing agent composition (C8) | (S3) | Gly | None | 0.42 |

*) Abbreviation for organic surface crosslinking agent
PG: Propylene glycol
HD: 1,6-hexanediol
TEG: triethylene glycol
EC: ethylene carbonate
Gly: glycerin

Table 3-3

|  | Name | Specific surface area | Washburn method contact angle θ2 | Peroxide residue on surface of water-absorbing agent composition | CRC | SFC | D50 | 150↓ 2) | Water content | Surface tension | Vortex | AAP | gap water ratio under pressure |
|---|---|---|---|---|---|---|---|---|---|---|---|---|---|
|  |  | [$m^2$/kg] | [°] | [mass%] | [g/g] | [ (1) ] | [μm] | [mass%] | [mass%] | [mN/m] | [Seconds] | [g/g] | [g/g] |
| Comparative Example 6 | Water-absorbing agent composition (C6) | 22 | 75 | 0.09 | 29.5 | 30 | 410 | 2.6 | 1.4 | 71 | 62 | 25.3 | 3.0 |
| Example 5 | Water-absorbing agent composition (5) | 27 | 71 | 0.07 | 29.7 | 29 | 390 | 2.6 | 1.4 | 72 | 38 | 26.5 | 9.7 |
| Example 6 | Water-absorbing agent composition (6) | 27 | 68 | 0.07 | 30.3 | 33 | 390 | 2.4 | 1.6 | 71 | 39 | 26.0 | 9.6 |
| Comparative Example 7 | Water-absorbing agent composition (C7) | 27 | 74 | -0.01 | 29.9 | 32 | 390 | 2.5 | 1.6 | 68 | 41 | 25.3 | 9.5 |
| Example 7 | Water-absorbing agent composition (7) | 27 | 67 | 0.07 | 30.0 | 34 | 390 | 2.5 | 1.4 | 72 | 35 | 26.2 | 10.4 |
| Example 8 | Water-absorbing agent composition (8) | 27 | 67 | 0.07 | 29.3 | 30 | 390 | 2.5 | 1.5 | 72 | 37 | 25.8 | 9.7 |

(continued)

| | Name | Specific surface area | Washburn method contact angle θ2 | Peroxide residue on surface of water-absorbing agent composition | CRC | SFC | D50 | 150↓ 2) | Water content | Surface tension | Vortex | AAP | gap water ratio under pressure |
|---|---|---|---|---|---|---|---|---|---|---|---|---|---|
| | | [$m^2$/kg] | [°] | [mass%] | [g/g] | [ (1) ] | [μm] | [mass%] | [mass%] | [mN/m] | [Seconds] | [g/g] | [g/g] |
| Example 9 | Water-absorbing agent composition (9) | 27 | 66 | 0.07 | 30.1 | 7 | 390 | 2.5 | 1.6 | 72 | 36 | 25.5 | 9.3 |
| Comparative Example 8 | Water-absorbing agent composition (C8) | 27 | 75 | 0.00 | 30.5 | 3 | 390 | 2.7 | 1.5 | 72 | 39 | 24.1 | 8.1 |

1) Unit of SFC: $\times 10^{-7}$ $cm^3 \cdot sec/g$
2) 150 ↓: Proportion of particles of less than 150 μm

Summary

From the results of the water-absorbing agent composition of Comparative Example 6 and the water-absorbing agent composition of Example 5 under the same conditions except that the specific surface area of the water-absorbent resin was different, the water-absorbing agent composition of Example 5 having a contact angle θ2 of 72° or less had a lower Vortex and a higher AAP and gap water ratio under pressure than the water-absorbing agent composition of Comparative Example 6.

From the results of the water-absorbing agent compositions of Example 6 and Comparative Example 7, and Example 9 and Comparative Example 8, under the same conditions except for the addition of the peroxide in the surface crosslinking step, the water-absorbing agent compositions of Examples having a contact angle θ2 of 72° or less had a lower Vortex and a higher AAP and gap water ratio under pressure than the water-absorbing agent compositions of Comparative Examples. In addition, from the value of the peroxide residual amount on the surface of the water-absorbing agent composition, it is found that the peroxide added in the surface treatment step remains on the particle surface and acts on the surface. Further, also in the water-absorbing agent compositions of Example 7 and Example 8 using different types of surface crosslinking agents, Vortex was low, and AAP and the gap water ratio under pressure were high.

The water-absorbing agent compositions (1) to (9) had a Washburn method contact angle θ2 of 72° or less. It is considered that the affinity between the liquid to be absorbed and the surface of the water-absorbing agent composition was able to be improved by the production method of the present application, and it is considered that the liquid easily penetrated into the cavity formed on the surface of the water-absorbent resin, and thus, the gap water ratio under pressure was improved. This makes it possible to reduce the liquid return amount in a water-absorbent article such as a disposable diaper.

In addition, from the value of the peroxide residual amount on the surface of the water-absorbing agent composition, it is found that the peroxide added in the surface treatment step remains on the particle surface and acts on the surface.

Industrial Applicability

The water-absorbing agent composition according to the present invention can be suitably used, for example, in disposable diapers. In addition, the water-absorbing agent composition according to the present invention can be suitably used for various applications such as absorbent articles other than disposable diapers (sanitary napkins, incontinence pads, etc.), soil humectants for agriculture and horticulture, and water-blocking agents for industry.

This application is based on Japanese Patent Application No. 2023-193270 filed on November 13, 2023, the disclosure of which is incorporated herein by reference in its entirety.

## Claims

1. A method for producing a water-absorbing agent composition including a surface-crosslinked water-absorbent resin as a main component, the method comprising a surface crosslinking step for a water-absorbent resin,
the method satisfying all of (1) to (3) described below:

   (1) the water-absorbent resin before surface crosslinking has a specific surface area of 25 $m^2$/kg or more;
   (2) the surface crosslinking step is performed in the presence of an organic surface crosslinking agent capable of reacting with a peroxide and a carboxyl group;
   (3) the surface crosslinking step includes a heat treatment step of performing a heat treatment in an atmosphere having a water vapor density of more than 0.005 g/L simultaneously with or after adding the organic surface crosslinking agent to the water-absorbent resin.

2. The production method according to claim 1, wherein the water vapor density is 0.60 g/L or less.

3. The production method according to claim 1, wherein the water-absorbent resin before surface crosslinking has an irregularly crushed shape.

4. The production method according to claim 1, wherein the water-absorbent resin before surface crosslinking has a D50 (mass-average particle diameter) of 250 μm or more and less than 550 μm, and a mass proportion of particles contained in the water-absorbent resin before surface crosslinking and having a particle diameter of less than 150 μm is 3 mass% or less.

**5.** The production method according to claim 1, the method further comprising a polymerization step for an aqueous monomer solution,
wherein the water-absorbent resin is obtained by foaming polymerization of the aqueous monomer solution.

**6.** The production method according to claim 1, wherein the organic surface crosslinking agent is added to the water-absorbent resin in a solution state, and a concentration of the organic surface crosslinking agent in the surface crosslinking agent solution is 0.1 mass% or more and 60 mass% or less.

**7.** The production method according to claim 1, wherein the organic surface crosslinking agent is added to the water-absorbent resin from two or more addition nozzles installed in a mixing apparatus.

**8.** The production method according to claim 1, wherein the surface crosslinking step is performed under a slight reduced pressure of -10 kPa or more and 0 kPa or less as a differential pressure relative to atmospheric pressure.

**9.** The production method according to claim 1, wherein the peroxide is a persulfate.

**10.** A water-absorbing agent composition including a surface-crosslinked water-absorbent resin as a main component, the water-absorbing agent composition satisfying all of (1) to (2) described below:

(1) the water-absorbing agent composition has a specific surface area of 25 $m^2$/kg or more;
(2) the water-absorbing agent composition has a Washburn method contact angle $\theta 2$ of 72° or less as measured with a 20 mass% aqueous sodium chloride solution.

**11.** The water-absorbing agent composition according to claim 10, wherein the surface-crosslinked water-absorbent resin has an irregularly crushed shape and has a D50 (mass-average particle diameter) of 250 μm or more and less than 550 μm, and a mass proportion of particles contained in the water-absorbent resin and having a particle diameter of less than 150 μm is 3 mass% or less.

**12.** The water-absorbing agent composition according to claim 10, wherein the water-absorbing agent composition has an absorption capacity under pressure (AAP) of 20.0 g/g or more at a load of 4.83 kPa.

**13.** The water-absorbing agent composition according to claim 10, wherein the water-absorbing agent composition has a gap water ratio under pressure of 9.0 g/g or more.

**14.** The water-absorbing agent composition according to claim 10, wherein the water-absorbing agent composition has a surface tension of 56 mN/m or more.

**15.** The water-absorbing agent composition according to claim 10, wherein the water-absorbing agent composition has a Vortex of 50 seconds or less.

**16.** The water-absorbing agent composition according to claim 10, further comprising a peroxide decomposition product, wherein the peroxide decomposition product has a higher concentration on a surface of the water-absorbing agent composition than a concentration inside the water-absorbing agent composition.

**17.** The water-absorbing agent composition according to claim 10, wherein when particles are sieved using a JIS standard sieve having a mesh size of 300 μm, and thereby particles having a particle diameter of 300 μm or more are defined as a particle group a and particles having a particle diameter of less than 300 μm are defined as a particle group b, and an impact test using a paint shaker is performed on the water-absorbing agent composition, and thereby an amount of a peroxide decomposition product present in the particle group a is defined as C1, and an amount of a peroxide decomposition product present in the particle group bis defined as C2, C2 - C1 is more than 0 mass% and 1 mass% or less.

**18.** The water-absorbing agent composition according to claim 10, wherein the water-absorbing agent composition has an SFC (saline flow conductivity) of $1 \times 10^{-7}$ $cm^3 \cdot sec/g$ or more.

**19.** An absorbent article comprising the water-absorbing agent composition according to any one of claims 10 to 18.

## INTERNATIONAL SEARCH REPORT

International application No. **PCT/JP2024/040355**

**A. CLASSIFICATION OF SUBJECT MATTER**

***C08J 3/24***(2006.01)i; ***A61F 13/53***(2006.01)i; ***B01J 20/26***(2006.01)i; ***B01J 20/30***(2006.01)i; ***C08J 3/12***(2006.01)i
FI: C08J3/24; C08J3/12 A CEY; B01J20/30; B01J20/26 D; A61F13/53 300

According to International Patent Classification (IPC) or to both national classification and IPC

**B. FIELDS SEARCHED**

Minimum documentation searched (classification system followed by classification symbols)

C08J3/24; A61F13/53; B01J20/26; B01J20/30; C08J3/12

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2020/145384 A1 (NIPPON SHOKUBAI CO., LTD.) 16 July 2020 (2020-07-16) whole document, particularly, claims, examples | 1-19 |
| A | WO 2015/093594 A1 (NIPPON SHOKUBAI CO., LTD.) 25 June 2015 (2015-06-25) whole document, particularly, claims, examples | 1-19 |
| A | JP 2009-531158 A (NIPPON SHOKUBAI CO., LTD.) 03 September 2009 (2009-09-03) whole document, particularly, claims, example 2 | 1-19 |
| A | JP 2000-504769 A (THE DOW CHEMICAL COMPANY) 18 April 2000 (2000-04-18) whole document | 1-19 |
| A | JP 2006-527641 A (THE PROCTER & GAMBLE COMPANY) 07 December 2006 (2006-12-07) whole document | 1-19 |
| A | JP 2012-236955 A (TEIJIN CHEM LTD.) 06 December 2012 (2012-12-06) whole document | 1-19 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 January 2025** | **04 February 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/040355**

**C. DOCUMENTS CONSIDERED TO BE RELEVANT**

| Category* | Citation of document, with indication, where appropriate, of the relevant passages | Relevant to claim No. |
|---|---|---|
| A | WO 2022/181771 A1 (NIPPON SHOKUBAI CO., LTD.) 01 September 2022 (2022-09-01) whole document | 1-19 |

Form PCT/ISA/210 (second sheet) (July 2022)

**INTERNATIONAL SEARCH REPORT**

International application No.
**PCT/JP2024/040355**

**Box No. III Observations where unity of invention is lacking (Continuation of item 3 of first sheet)**

This International Searching Authority found multiple inventions in this international application, as follows:

(i) The number of inventions in the international application set forth in the following claims is 2.
(Invention 1) Claims 1-9
(Invention 2) Claims 10-18 and 19

(ii) For the reasons indicated below, it is considered that the international application does not comply with the requirement of unity of invention (Regulations under the PCT, Rule 13 (PCT Rules 13.1, 13.2, and 13.3)).

(Invention 1) Claims 1-9

Document 1 (international publication number 2020/145384) discloses "a production method for a water absorbent having a water-absorbing resin as a main component, the method comprising a water-absorbing resin surface crosslinking step and a step for mixing in an aqueous sulfur-containing reductant solution, and satisfying (1)-(4) below

(1) The specific surface area of the water-absorbing resin is 25 $m^2$/kg or more

(2) The average droplet diameter of the aqueous sulfur-containing reductant solution is 2.5 mm or less and the temperature of the aqueous solution is 80°C or less

(3) In the mixing step, stirred mixing that satisfies expression (a) below is performed

the mixing power coefficient ≧ 0. 16 × (average droplet diameter (mm)) + 0.05 ・・・(a)

the mixing power coefficient being stipulated by (Froude number Fr) × (gravitational acceleration g)

(4) After the mixing step, a heated drying step of 40°C to 150°C is performed" (claim 1) and "for the surface crosslinking agent, an organic surface crosslinking agent that can form ester bonds between carboxyl groups is preferred" (paragraph [0103]). However,
the document does not indicate that "(2) the surface crosslinking step is performed in the presence of an organic surface crosslinking agent that can react with peroxides and carboxyl groups, and

(3) the surface crosslinking step has a heat treatment step for performing heat treatment in an atmosphere in which the water vapor density is greater than 0.005 g/L simultaneously with or after the addition of the organic surface crosslinking agent to the water-absorbing resin."

Accordingly, the invention in claim 1 is novel and involves an inventive step in light of document 1. Thus, the invention in claim 1 and the invention in claims 2-9, which depend therefrom, are classified as invention 1.

(Invention 2) Claims 10-18 and 19

The invention in claim 1 and the invention in claim 10 share the common technical feature of "the surface area of the water-absorbing resin being 25 $m^2$/kg or more."

However, said technical feature does not define a contribution over the prior art in the light of the content disclosed in document 1, and thus cannot be said to be a special technical feature. Moreover, there is no other same or corresponding special technical feature among these inventions.

Accordingly, the invention in claims 10-18 and 19 is classified as invention 2.

Form PCT/ISA/210 (continuation of first sheet) (July 2022)

**INTERNATIONAL SEARCH REPORT**

International application No.
**PCT/JP2024/040355**

**Box No. III Observations where unity of invention is lacking (Continuation of item 3 of first sheet)**

1. ☑ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☑ No protest accompanied the payment of additional search fees.

Form PCT/ISA/210 (continuation of first sheet) (July 2022)

**INTERNATIONAL SEARCH REPORT**
**Information on patent family members**

International application No.
**PCT/JP2024/040355**

| Patent document cited in search report | Publication date (day/month/year) | Patent family member(s) | Publication date (day/month/year) |
|---|---|---|---|
| WO 2020/145384 A1 | 16 July 2020 | US 2022/0111352 A1 whole document, particularly, claims, examples | |
| | | EP 3910004 A1 | |
| | | CN 113272365 A | |
| | | KR 10-2021-0110350 A | |
| WO 2015/093594 A1 | 25 June 2015 | US 2016/0375171 A1 whole document, particularly, claims, examples | |
| | | EP 3085439 A1 | |
| | | KR 10-2016-0102217 A | |
| | | CN 105848776 A | |
| JP 2009-531158 A | 03 September 2009 | JP 2009-531467 A whole document, particularly, claims, examples 2-4 | |
| | | US 2009/0208748 A1 whole document, particularly, claims, example 2 | |
| | | US 2009/0298685 A1 | |
| | | US 2012/0305842 A1 | |
| | | WO 2007/116777 A1 | |
| | | WO 2007/116778 A1 | |
| | | EP 2010316 A1 | |
| | | EP 2013251 A1 | |
| | | EP 3932541 A1 | |
| | | CN 101410177 A | |
| | | CN 101410424 A | |
| | | AT 524501 T | |
| | | CN 102698719 A | |
| JP 2000-504769 A | 18 April 2000 | US 6090875 A whole document | |
| | | WO 1997/030109 A1 | |
| | | EP 880554 A1 | |
| | | BR 9707511 A | |
| | | KR 10-1999-0082574 A | |
| | | MX 9806585 A | |
| | | ID 17901 A | |
| | | CN 1211265 A | |
| JP 2006-527641 A | 07 December 2006 | US 2005/0003191 A1 whole document | |
| | | WO 2005/004939 A1 | |
| | | EP 1493453 A1 | |
| | | CN 1816358 A | |
| | | MX PA05014158 A | |
| | | AT 492301 T | |
| JP 2012-236955 A | 06 December 2012 | (Family: none) | |
| WO 2022/181771 A1 | 01 September 2022 | US 2024/0299907 A1 whole document | |
| | | EP 4299651 A1 | |
| | | CN 116887915 A | |
| | | KR 10-2023-0138488 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9703114 A
- JP 10057805 A
- EP 0872491 A
- EP 0937739 A
- WO 9903577 A
- WO 2013018571 A
- WO 2016111223 A
- WO 02085959 A
- JP 5128098 B
- WO 2013072268 A
- US 20080161512 A
- WO 2007004529 A
- WO 2012023433 A
- US 4893999 A
- US 6906159 B
- US 7091253 B
- US 7741400 B
- US 8519212 B
- JP 2005036100 A
- US 6987151 B
- WO 97017397 A
- US 6107358 A
- JP 5989913 B
- JP 6067126 B
- JP 5647625 B
- WO 2016204302 A
- WO 2006100300 A
- WO 2011025012 A
- WO 2011025013 A
- WO 2011111657 A
- WO 2005016393 A
- US 7098284 B
- US 7638570 B
- JP 11060975 A
- WO 2011126079 A
- JP 2009509722 T
- JP 2005097519 A
- JP 2011074401 A
- JP 2013076073 A
- JP 2013213083 A
- JP 59105448 A
- JP 60158861 A
- JP 11241030 A
- JP 2041155 A
- US 5669894 A
- WO 2009016055 A
- WO 2021162072 A
- US 2023076935 A
- JP 9235378 A
- JP 4926474 B
- JP 2023193270 A


### Non-patent literature cited in the description


- *CHEMICAL ABSTRACTS*, 3844-45-9